# EUROPEAN PATENT APPLICATION

(11) **EP 2 275 093 A2**
(43) Date of publication of application: **19.01.2011**
(21) Application number: 09729736.0
(22) Date of filing: 08.04.2009
(51) Int. Cl.: A61K 9/22, A61K 31/165, A61K 9/48, A61K 31/41, A61K 31/4196, A61K 31/4178, A61P 9/10

(54) **PHARMACEUTICAL FORMULATION**

(30) Priority: 10.04.2008 KR 20080033362
(71) Applicant: HanAll Biopharma Co., Ltd., Daejeon 306-120 (KR)
(72) Inventor: KIM, Sung Wuk, Seongnam-si Gyeonggi-do 463-802 (KR); JUN, Sung Soo, Seongnam-si Gyeonggi-do 463-777 (KR); KOO, Ja Seong, Daejeon 305-756 (KR); KIM, Jin Wook, Daejeon 302-120 (KR); SON, Jae Woon, Suwon-si Gyeonggi-do 442-753 (KR); JO, Young Gwan, Daejeon 305-811 (KR)
(74) Representative: Pohlman, Sandra M.
(86) International application number: PCT/KR2009/001822
(87) International publication number: WO 2009/125981

(57) **Abstract**

The present invention provides a pharmaceutical formulation comprising a compartment containing a rennin inhibitor as a pharmacologically active ingredient, and a compartment having an angiotensin-II-receptor blocker as a pharmacologically active ingredient. One of the compartments is an immediate-release compartment and the other one is an extended-release compartment. Since the disclosed formulation delivers the rennin inhibitor and angiotensin-II-receptor blocker at a specific delivery rate at a different time. It has an advantage in reducing the concern about side effects, improving drug effects, and simplifying the instructions for use of the drug. In addition, the formulation can pharmacologically, clinically, scientifically, and economically achieve more useful effects than the complex prescription case of taking the ingredients separately or each at once, in preventing and treating metabolic syndrome, cardiovascular disease and renal disease.

## Description

### TECHNICAL FIELD

The present invention relates to a pharmaceutical formulation containing a renin inhibitor and an angiotensin-II-receptor blocker.

### BACKGROUND ART

A renin inhibitor is a drug which was developed to have the mechanism of inhibiting the conversion of angiotensinogen into angiotensin-I by inhibiting the cleavage of angiotensinogen through the binding with renin, and examples thereof include aliskiren, remikiren, enalkiren, zankiren, and the like. Among them, aliskiren is chemically defined as 2(S),4(S),5(S),7(S)-N-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide and is specifically disclosed in EP 678503 A. A hemi-fumarate salt thereof is known to be preferable [Recent patents on Cardiovascular Drug Discovery 2006; Nov 1(3): 233-40].

Renin, which is synthesized in the kidney, migrates to blood streams and cleaves angiotensinogen to promote the conversion of a decapeptide angiotensin-I into an active octapeptide angiotensin-II by the action of an angiotensin converting enzyme (ACE). The converted angiotensin-II is a factor responsible for blood pressure elevation, left ventricular hypertrophy, blood vessel hypertrophy, atherosclerosis, renal failure, cerebral stroke and the like. The renin inhibitor binds to renin to thereby inhibit the cleavage of angiotensinogen, which consequently inhibits the occurrence of blood pressure elevation, left ventricular hypertrophy, blood vessel hypertrophy, atherosclerosis, renal failure, cerebral stroke and the like.

The angiotensin-II-receptor blocker has been elucidated up to now as a drug exerting hypotensive effects on both systolic and diastolic phases of the myocardium, by blocking the binding of angiotensin-II, one of fundamental substances responsible for vasoconstriction, to an angiotensin receptor. As the angiotensin-II-receptor blocker, there are about 10 kinds of compound groups that are frequently clinically applied, including pharmaceutically acceptable salts. Further, these compounds are used alone or in combination with ACE inhibitor(s)exhibiting hypotensive effects through the similar mechanism, on mild to severe patients suffering from various symptoms associated with hypertension [Angiotensin 2 Receptor Antagonist: An Overview, Am J Health-Syst Pharm 57(13): 1231-1238, 2000].

Among these angiotensin-II-receptor blockers, valsartan, losartan, candesartan, telmisartan, eprosartan, olmesartan, and the like have been commercialized and have rapidly grown as anti-hypertension drugs over the past several years. In addition, effects of these drugs are also verified through various clinical trials [Pharmacologic, Pharmacokinetic, and Therapeutic Difference Among angiotensin II receptor Antagonist: Pharmacotherapy 20(2): 130-139, 2000].

As demonstrated through such various clinical trials, these angiotensin-II-receptor blockers are equivalent in hypotensive effects on myocardial systolic and diastolic phases at an optimum dose, even though they exhibit pharmacokinetic differences in *in vivo* metabolic pathways and half lives. Further, it is also known that these angiotensin-II-receptor blockers are similar in effects obtained by the same receptor blocker, such as prevention and treatment of secondary heart failure associated with various symptoms of hypertension, prevention and treatment of arrhythmia and heart failure occurring after myocardial infarction, prevention and treatment of diabetic complications, prevention and treatment of renal failure, prevention and treatment of cerebral stroke, anti-platelet action, arteriosclerosis-preventive action, suppression of harmful effects of aldosterone, alleviation of metabolic syndromes, and preventive effects against interactive aggravation of circulatory diseases [J. Wagner et al.: Effects of AT1 receptor blockade on blood pressure and the renin angiotensin system in spontaneously hypertensive rats of the stroke prone strain, Clin, Exp. Hypertens., vol. 20(1998), p. 205-221; M. Bohm et al.: angiotensin II receptor blockade in TGR(mREN2)27: Effects of renin-angiotensin-system gene expression and cardiovascular functions, J. Hypertens., vol. 13(8) (1995), p.891-899].

Valsartan, which is one of angiotensin-II-receptor blockers, is known to exert potent hypotensive effects from the middle of the night to daybreak during which a renin and angiotensin system (RAAS) actively works [J. Hypertens, 2005; 23; 1913-1922, Hypertension, 2003; 42; 283-290, Chronobiol. Int. 2005; 22; 755-776].

Meanwhile, combined use and combined administration of a renin inhibitor with an angiotensin-II-receptor blocker are expected to have hypotensive action and additional effects. As a combination treatment, related art regarding the combination therapy of a renin inhibitor and an angiotensin-II-receptor blocker has been proposed as follows.

International Patent Publication No. WO 2002/40007 discloses a synergistic combination for treating cardiovascular diseases, using an angiotensin-II-receptor blocker and a renin inhibitor.

Korean Patent Application Laid-Open Publication No. 1993-0702895 discloses a method for treating hypertension by combination of an angiotensin-II-receptor antagonist with a diuretic, a calcium channel blocker, a beta adrenalin blocker, a renin inhibitor, or an ACE inhibitor, but merely describes combination feasibility of formulations commonly used in the treatment of cardiovascular diseases, without taking into consideration characteristics of individual drug ingredients of the combination formulation.

However, the above-stated inventions relating to a combination therapy are merely simple combination formulations which do not take into consideration characteristics and absorption principles of individual drugs.

Aliskiren, which is a renin inhibitor, is a low-bioavailability drug that is easily soluble in water, but is not readily absorbed, so a care should be taken when aliskiren is administered in combination with other drugs which may have effects on competitive absorption of drug ingredients. It is also known that aliskiren is metabolized by hepatic cytochrome P450 3A4 in *in vitro* experiments.

It is known that concurrent administration of the renin inhibitor, aliskiren and the angiotensin-II-receptor blocker, irbesartan leads to a 50% decrease in blood aliskiren level, which consequently may result in decreased effects of aliskiren and may not provide additive effects of the combination formulation.

As a result of a variety of extensive and intensive studies and experiments to develop a formulation which is capable of preventing the drug interaction between a renin inhibitor and an angiotensin-II-receptor blocker and is expected to exhibit optimum therapeutic effects of individual drug ingredients without decreasing therapeutic effects upon combined administration thereof, the inventors have completed the present invention as follows.

### DISCLOSURE OF THE INVENTION

### TECHNICAL PROBLEM

Therefore, the present invention is intended to provide a formulation which is capable of minimizing side effects occurring upon combined administration of a renin inhibitor and an angiotensin-II-receptor blocker, inducing optimum pharmacological effects, obtaining clinical synergistic effects through the administration of drugs at a time point where pharmacological effects of individual drugs are expressed, and enhancing the compliance of patients.

### TECHNICAL SOLUTION

The present invention provides a pharmaceutical formulation including a compartment containing a renin inhibitor as a pharmacologically active ingredient, and a compartment containing an angiotensin-II-receptor blocker as a pharmacologically active ingredient, wherein one compartment is a prior-release compartment and the other compartment is a delayed-release compartment.

Unless otherwise specified, the term "renin inhibitor" as used herein is intended to include an isomer thereof or a pharmaceutically acceptable salt thereof, and the term "angiotensin-II-receptor blocker" is intended to include an isomer thereof, a pharmaceutically acceptable salt thereof, or a prodrug thereof.

For example, the renin inhibitor may be at least one selected from aliskiren, remikiren, enalkiren, zankiren, detikiren, terlakiren, isomers thereof and pharmaceutically acceptable salts thereof. A pharmaceutically acceptable salt of aliskiren may be aliskiren hemi-fumarate.

For example, the angiotensin-II-receptor blocker may be at least one selected from irbesartan, valsartan, losartan, telmisartan, candesartan, olmesartan, isomers thereof, pharmaceutically acceptable salts thereof, and prodrugs thereof.

Preferably, the renin inhibitor in the formulation of the present invention is aliskiren, an isomer thereof or a pharmaceutically acceptable salt thereof (unless otherwise specified, hereinafter referred to as "aliskiren") and the angiotensin-II-receptor blocker is irbesartan, an isomer thereof or a pharmaceutically acceptable salt thereof (unless otherwise specified, hereinafter referred to as "irbesartan"); or the renin inhibitor is aliskiren, an isomer thereof or a pharmaceutically acceptable salt thereof (unless otherwise specified, hereinafter referred to as "aliskiren") and the angiotensin-II-receptor blocker is valsartan, an isomer thereof or a pharmaceutically acceptable salt thereof (unless otherwise specified, hereinafter referred to as "valsartan"); or the renin inhibitor is aliskiren, an isomer thereof or a pharmaceutically acceptable salt thereof (unless otherwise specified, hereinafter referred to as "aliskiren") and the angiotensin-II-receptor blocker is losartan, an isomer thereof or a pharmaceutically acceptable salt thereof (unless otherwise specified, hereinafter referred to as "losartan"); or the renin inhibitor is aliskiren, an isomer thereof or a pharmaceutically acceptable salt thereof (unless otherwise specified, hereinafter referred to as "aliskiren") and the angiotensin-II-receptor blocker is telmisartan, an isomer thereof or a pharmaceutically acceptable salt thereof (unless otherwise specified, hereinafter referred to as "telmisartan"); or the renin inhibitor is aliskiren, an isomer thereof or a pharmaceutically acceptable salt thereof (unless otherwise specified, hereinafter referred to as "aliskiren") and the angiotensin-II-receptor blocker is candesartan, an isomer thereof, a pharmaceutically acceptable salt thereof, or a prodrug thereof (unless otherwise specified, hereinafter referred to as "candesartan"); or the renin inhibitor is aliskiren, an isomer thereof or a pharmaceutically acceptable salt thereof (unless otherwise specified, hereinafter referred to as "aliskiren") and the angiotensin-II-receptor blocker is olmesartan, an isomer thereof, a pharmaceutically acceptable salt thereof, or a prodrug thereof (unless otherwise specified, hereinafter referred to as "olmesartan").

The prodrug of candesartan is decomposed *in vivo* into an active ingredient candesartan and is preferably candesartan cilexetil. The prodrug of olmesartan is decomposed *in vivo* into an active ingredient olmesartan and is preferably olmesartan medoxomil.

The formulation in accordance with the present invention provides more useful therapeutic effects by the provision of a physical compartment for controlling release properties between two pharmacologically active ingredients, thereby improving problems of conventional combined administration or concurrent administration of single drugs.

A pharmacologically active ingredient of the prior-release compartment in the formulation of the present invention is released at a level of more than 80% by weight of a total amount of the pharmacologically active ingredient of the prior-release compartment in the formulation within one hour after the release of the pharmacologically active ingredient is initiated. Preferably, more than 90% by weight of a total amount of the pharmacologically active ingredient in the formulation is released within one hour.

Further, a pharmacologically active ingredient of the delayed-release compartment in the formulation of the present invention is released at a level of less than 40% by weight of a total amount of the pharmacologically active ingredient of the delayed-release compartment in the formulation by 2 hours after the release of the pharmacologically active ingredient of the prior-release compartment is initiated. Preferably, the pharmacologically active ingredient of the delayed-release compartment is released at a level of less than 20% by weight of a total amount of the pharmacologically active ingredient of the delayed-release compartment by 2 hours after the release of the pharmacologically active ingredient of the prior-release compartment is initiated.

The present invention provides a pharmaceutical formulation wherein the pharmacologically active ingredient of the delayed-release compartment is metabolized in the liver at a time-lag interval of 2 to 4 hours after the pharmacologically active ingredient of the prior-release compartment is metabolized.

A content of the renin inhibitor such as aliskiren in the formulation of the present invention may be in the range of 10 to 1000 mg, preferably 35 to 600 mg, and more preferably 75 to 300 mg, in the pharmaceutical formulation.

A content of the angiotensin-II-receptor blocker such as valsartan in the formulation of the present invention may be in the range of 10 to 1000 mg, preferably 20 to 600 mg, and more preferably 40 to 320 mg, in the pharmaceutical formulation.

Hereinafter, individual compartments of the pharmaceutical formulation in accordance with the present invention will be described in more detail.

### 1. Prior-release compartment

The "prior-release compartment" refers to a compartment which is released ahead of the "delayed-release compartment" in the pharmaceutical formulation of the present invention. The prior-release compartment may further contain "pharmaceutically acceptable additives", if necessary, in addition to "pharmacologically active ingredients".

### (1) Pharmacologically active ingredients

The pharmacologically active ingredient of the prior-release compartment is 1) a renin inhibitor, or 2) an angiotensin-II-receptor blocker.

### (2) Pharmaceutically acceptable additives

The formulation of the present invention may further contain commonly used additives such as pharmaceutically acceptable diluent, binder, disintegrant, lubricant, pH-adjusting agent, and solubilizer, within the range where effects of the present invention are not impaired and the release of pharmacologically active ingredients is not impaired.

In the prior-release compartment of the present invention, a content of the additive is in the range of 0.1 to 300 parts by weight, relative to 1 part by weight of the active ingredient.

Examples of the diluent that can be used in the prior-release compartment of the present invention may include starch, microcrystalline cellulose, lactose, glucose, mannitol, alginate, an alkaline earth metal salt, clay, polyethylene glycol, dicalcium phosphate, and a mixture thereof.

Examples of the binder that can be used in the prior-release compartment of the present invention may include starch, microcrystalline cellulose, highly dispersive silica, mannitol, sucrose, lactose, polyethylene glycol, polyvinylpyrrolidone, hydroxypropylmethylcellulose, hydroxypropylcellulose, natural gum, synthetic gum, copovidone, povidone, gelatin, and a mixture thereof.

Examples of the disintegrant that can be used in the prior-release compartment of the present invention may include starches or modified starches such as sodium starch glycolate, corn starch, potato starch, and pregelatinized starch, clays such as bentonite, montmorillonite, and veegum, celluloses such as microcrystalline cellulose, hydroxypropylcellulose, and carboxymethylcellulose, algins such as sodium alginate, and alginic acid, crosslinked celluloses such as croscarmellose sodium, gums such as guar gum, and xanthan gum, crosslinked polymers such as crosslinked polyvinylpyrrolidone (crospovidone), effervescent agents such as sodium bicarbonate and citric acid, and mixtures thereof.

Examples of the lubricant that can be used in the prior-release compartment of the present invention may include talc, stearic acid, magnesium stearate, calcium stearate, sodium lauryl sulfate, hydrogenated vegetable oil, sodium benzoate, sodium stearyl fumarate, glyceryl behenate, glyceryl monolaurate, glyceryl monostearate, glyceryl palmitostearate, polyethylene glycol, and mixtures thereof.

Examples of the stabilizer that can be used in the prior-release compartment of the present invention may include ascorbic acid, citric acid, butylated hydroxyanisole, butylated hydroxy toluene, and tocopherol derivatives. Further examples of the stabilizer may include alkalizers such as alkali metal salts, alkaline earth metal salts, or mixtures thereof Preferably, there may be used calcium carbonate, sodium carbonate, sodium hydrogen carbonate, magnesium oxide, magnesium carbonate, and sodium citrate. Examples of the pH-adjusting agent that can be used in the prior-release compartment of the present invention may include acidifying agents such as acetic acid, adipic acid, ascorbic acid, malic acid, succinic acid, tartaric acid, fumaric acid, and citric acid, and alkalizing agent such as precipitated calcium carbonate, aqueous ammonia, and meglumine.

Examples of the solubilizer that can be used in the prior-release compartment of the present invention may include sodium lauryl sulfate, polyoxyethylene sorbitan fatty acid ester such as polysorbate, and docusate sodium.

In addition, the formulation of the present invention may optionally contain pharmaceutically acceptable additives such as various additives selected from a colorant and a fragrance.

The range of the additive that can be used in the present invention is not limited to the above-mentioned additives, and the additive may be used in a conventional dose which can be suitably selected by those skilled in the art.

### 2. Delayed-release compartment

In the present invention, the "delayed-release compartment" refers to a compartment whose active ingredient is released at a certain time interval after the release of the active ingredient of the "prior-release compartment". The delayed-release compartment may contain (1) a "pharmacologically active ingredient" and (2-1) a "release-controlling material" or (2-2) an osmo-regulator and a semi-permeable membrane coating base, and (3), if necessary, "pharmaceutically acceptable additives".

### (1) Pharmacologically active ingredients

The pharmacologically active ingredient of the delayed-release compartment is 1) a renin inhibitor, or 2) an angiotensin-II-receptor blocker. Here, the pharmacologically active ingredient of the delayed-release compartment is an ingredient which is not identical with the pharmacologically active ingredient of the prior-release compartment. For instance, when the pharmacologically active ingredient of the prior-release compartment is 1) aliskiren, an isomer thereof or a pharmaceutically acceptable salt thereof, the pharmacologically active ingredient of the delayed-release compartment is 2) irbesartan, an isomer thereof or a pharmaceutically acceptable salt thereof, or vice versa.

The active ingredient of the delayed-release compartment in the formulation of the present invention is released at a level of less than 40% by weight of a total amount of the active ingredient of the delayed-release compartment in the formulation by 2 hours after the release of the active ingredient of the prior-release compartment is initiated. Preferably, the active ingredient of the delayed-release compartment is released at a level of less than 20% by weight of a total amount of the active ingredient of the delayed-release compartment by 2 hours after the release of the active ingredient of the prior-release compartment is initiated.

### (2-1) Release-controlling materials

The delayed-release compartment in the pharmaceutical formulation of the present invention contains at least one release-controlling material selected from an enteric polymer, a water-insoluble polymer, a hydrophobic compound, a hydrophilic polymer and a mixture thereof. The release-controlling material is preferably at least one selected from an enteric polymer, a hydrophilic polymer and a water-insoluble polymer, specifically at least one selected from a water-insoluble polymer, hydroxypropylcellulose, hydroxypropylmethylcellulose, hydroxypropylmethylcellulose phthalate, a methacrylic acid/ethyl acrylate copolymer, and a carboxyvinyl polymer.

In the delayed-release compartment of the present invention, a content of the release-controlling material is in the range of 0.01 to 100 parts by weight relative to 1 part by weight of the active ingredient. If a content of the release-controlling material is lower than 0.01 parts by weight, it may be difficult to achieve a sufficient time-lag. On the other hand, if a content of the release-controlling material is higher than 100 parts by weight, it may be difficult to achieve significant clinical effects due to delayed release of the drug.

In the delayed-release compartment of the present invention, the enteric polymer refers to a polymer which is insoluble or stable under the acidic conditions of less than pH 5, and is dissolved or degraded under the specific pH conditions of pH 5 or higher. The enteric polymer that can be used in the present invention is at least one selected from the group consisting of an enteric cellulose derivative, an enteric acrylic acid copolymer, an enteric maleic acid copolymer, an enteric polyvinyl derivative, and a mixture thereof.

Preferably, the enteric cellulose derivative is at least one selected from hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose, ethylhydroxyethylcellulose phthalate, methylhydroxyethylcellulose and a mixture thereof; the enteric acrylic acid copolymer is at least one selected from a styrene/acrylic acid copolymer, a methyl acrylate/acrylic acid copolymer, a methyl acrylate/methacrylic acid copolymer, a butyl acrylate/styrene/acrylic acid copolymer, a methacrylic acid/methyl methacrylate copolymer (e.g., Eudragit L 100 or Eudragit S, Degussa, Germany), a methacrylic acid/ethyl acrylate copolymer (e.g., Eudragit L 100-55, Degussa, Germany), a methyl acrylate/methacrylic acid/octyl acrylate copolymer and a mixture thereof; the enteric maleic acid copolymer is at least one selected from a vinylacetate/maleic anhydride copolymer, a styrene/maleic anhydride copolymer, a styrene/maleic monoester copolymer, a vinyl methyl ether/maleic anhydride copolymer, an ethylene/maleic anhydride copolymer, a vinyl butyl ether/maleic anhydride copolymer, an acrylonitrile/methyl acrylate/maleic anhydride copolymer, a butyl acrylate/styrene/maleic anhydride copolymer and a mixture thereof; and the enteric polyvinyl derivative is at least one selected from polyvinylalcohol phthalate, polyvinylacetal phthalate, polyvinylbutyrate phthalate, polyvinylacetacetal phthalate and a mixture thereof. The enteric polymer in the formulation of the present invention is preferably at least one selected from hydroxypropylmethylcellulose phthalate and a methacrylic acid/ethyl acrylate copolymer.

A content of the enteric polymer may be in the range of 0.1 parts by weight to 20 parts by weight, preferably 0.5 parts by weight to 10 parts by weight relative to 1 part by weight of the active ingredient. If a content of the enteric polymer is lower than 0.1 parts by weight, the enteric polymer may be easily dissolved at a pH of less than 5. On the other hand, if a content of the enteric polymer is higher than 20 parts by weight, this may lead to an unnecessary increase in a total weight of the formulation or excessively delayed release thereof.

In the delayed-release compartment of the present invention, the water-insoluble polymer refers to a pharmaceutically acceptable water-insoluble polymer which controls the release of a drug. The water-insoluble polymer that can be used in the present invention is preferably at least one selected from the group consisting of polyvinyl acetate, a water-insoluble polymethacrylate copolymer {e.g. poly(ethyl acrylate, methyl methacrylate) copolymer (such as Eudragit NE30D), a poly(ethyl acrylate, methyl methacrylate, trimethylaminoethyl methacrylate) copolymer (e.g. Eudragit RS PO), etc.}, ethylcellulose, cellulose ester, cellulose ether, cellulose acylate, cellulose diacylate, cellulose triacylate, cellulose acetate, cellulose diacetate, cellulose triacetate and a mixture thereof. A content of the water-insoluble polymer may be in the range of 0.1 parts by weight to 30 parts by weight, preferably 0.5 parts by weight to 20 parts by weight relative to 1 part by weight of the active ingredient. If a content of the water-insoluble polymer is lower than 0.1 parts by weight, release of the drug may be not controlled. On the other hand, if a content of the water-insoluble polymer is higher than 30 parts by weight, release of the drug may be excessively delayed.

Further, in the delayed-release compartment of the present invention formulation, a preferred example of the water-insoluble polymer which is a release-controlling material is at least one selected from the group consisting of polyvinylacetate, a poly(ethyl acrylate, methyl methacrylate, trimethylaminoethyl methacrylate) copolymer and a mixture thereof.

In the delayed-release compartment of the present invention, the hydrophobic compound refers to a pharmaceutically acceptable water-insoluble material which controls the release of a drug. The hydrophobic compound that can be used in the present invention may be at least one selected from the group consisting of fatty acid and fatty acid ester, fatty acid alcohol, wax, inorganic material, and a mixture thereof. Preferably, the fatty acid or fatty acid ester is at least one selected from glyceryl palmitostearate, glyceryl stearate, glyceryl distearate, glyceryl behenate, cetyl palmitate, glyceryl monooleate, stearic acid and a mixture thereof; the fatty acid alcohol is at least one selected from cetostearyl alcohol, cetyl alcohol, stearyl alcohol and a mixture thereof; the wax is at least one selected from carnauba wax, beeswax, microcrystalline wax and a mixture thereof; and the inorganic material is at least one selected from talc, precipitated calcium carbonate, calcium hydrogen phosphate, zinc oxide, titanium oxide, kaolin, bentonite, montmorillonite, veegum and a mixture thereof.

A content of the hydrophobic compound may be in the range of 0.1 parts by weight to 20 parts by weight, preferably 0.5 parts by weight to 10 parts by weight relative to 1 part by weight of the active ingredient. If a content of the hydrophobic compound is lower than 0.1 parts by weight, release of the drug may be not controlled. On the other hand, if a content of the hydrophobic compound is higher than 20 parts by weight, release of the drug may be excessively delayed.

In the delayed-release compartment of the present invention, the hydrophilic polymer refers to a pharmaceutically acceptable water-soluble polymer which controls the release of a drug. The hydrophilic polymer that can be used in the present invention may be at least one selected from the group consisting of saccharide, a cellulose derivative, gum, a protein, a polyvinyl derivative, a hydrophilic polymethacrylate copolymer, a polyethylene derivative, a carboxyvinyl copolymer and a mixture thereof. Preferably, the saccharide is at least one selected from dextrin, polydextrin, dextran, pectin and a pectin derivative, alginate, polygalacturonic acid, xylan, arabinoxylan, arabinogalactan, starch, hydroxypropyl starch, amylose, amylopectin and a mixture thereof; the cellulose derivative is at least one selected from hydroxypropylmethylcellulose, hydroxypropylcellulose, hydroxymethylcellulose, hydroxyethylcellulose, methylcellulose, sodium carboxymethylcellulose, hydroxypropylmethylcellulose acetate succinate, hydroxyethylmethylcellulose and a mixture thereof; the gum is at least one selected from guar gum, locust bean gum, tragacanth, carrageenan, gum acacia, gum arabic, gellan gum, xanthan gum and a mixture thereof; the protein is at least one selected from gelatin, casein, zein and a mixture thereof; the polyvinyl derivative is at least one selected from polyvinyl alcohol, polyvinyl pyrrolidone, polyvinylacetal diethylaminoacetate and a mixture thereof; the hydrophilic polymethacrylate copolymer is at least one selected from a poly(butyl methacrylate, (2-dimethylaminoethyl)methacrylate, methyl methacrylate) copolymer (e.g. Eudragit E100, Evonik, Germany), a poly(methacrylic acid, methyl methacrylate) copolymer (e.g. Eudragit L100), a poly(methacrylic acid, ethyl acrylate) copolymer (e.g. Eudragit L100-55) and a mixture thereof; the polyethylene derivative is at least one selected from polyethylene glycol, polyethylene oxide and a mixture thereof; and the carboxyvinyl polymer is carbomer. A preferred example of the hydrophilic polymer is a carboxyvinyl polymer.

A content of the hydrophilic polymer may be in the range of 0.05 parts by weight to 30 parts by weight, preferably 0.5 to 20 parts by weight relative to 1 part by weight of the active ingredient. If a content of the hydrophilic polymer is lower than 0.05 parts by weight, release of the drug may be not controlled. On the other hand, if a content of the hydrophilic polymer is higher than 30 parts by weight, release of the drug may be excessively delayed.

### (2-2) Osmo-regulator and semi-permeable membrane coating base

The delayed-release compartment of the present invention may be a compartment which contains an osmo-regulator and is coated by a semi-permeable membrane coating base.

In the delayed-release compartment of the present invention, the osmo-regulator is preferably at least one selected from the group consisting of magnesium sulfate, magnesium chloride, sodium chloride, lithium chloride, potassium sulfate, sodium sulfate, lithium sulfate and a mixture thereof.

A content of the osmo-regulator may be in the range of 0.01 parts by weight to 10 parts by weight, preferably 0.05 parts by weight to 0.5 parts by weight relative to 1 part by weight of the active ingredient. If a content of the osmo-regulator is lower than 0.01 parts by weight, it may be difficult to achieve a sufficient chronotherapeutic release. On the other hand, if a content of the osmo-regulator is higher than 10 parts by weight, it may be difficult to achieve significant clinical effects due to delayed release of the drug.

In the present invention, the semi-permeable membrane coating base is a material which is blended in a coating layer of the pharmaceutical formulation and refers to a material used to form a membrane through which some ingredients can pass but other ingredients cannot pass. The semi-permeable coating base in the present invention may employ the above-mentioned water-insoluble polymers.

For example, the semi-permeable membrane coating base that can be used in the present invention is at least one selected from the group consisting of polyvinyl acetate, a polymethacrylate copolymer, poly(ethyl acrylate, methyl methacrylate) copolymer, a poly(ethyl acrylate, methyl methacrylate, trimethylaminoethyl methacrylate) copolymer, ethylcellulose, cellulose ester, cellulose ether, cellulose acylate, cellulose diacylate, cellulose triacylate, cellulose acetate, cellulose diacetate, cellulose triacetate and a mixture thereof.

A content of the semi-permeable membrane coating base may be in the range of 0.01 parts by weight to 10 parts by weight, preferably 0.05 parts by weight to 1.25 parts by weight relative to 1 part by weight of the active ingredient. If a content of the semi-permeable membrane coating base is lower than 0.01 parts by weight, it may be difficult to achieve a sufficient time-lag. On the other hand, if a content of the semi-permeable membrane coating base is higher than 10 parts by weight, there is a problem associated with no release of the drug or an excessively long time-lag.

### (3) Pharmaceutically acceptable additives

In addition to (2-1) a release-controlling material and (2-2) an osmo-regulator and a semi-permeable membrane coating base, the formulation of the present invention may further contain commonly used additives such as pharmaceutically acceptable diluent, binder, disintegrant, lubricant, pH-adjusting agent, anti-foaming agent, and solubilizer, within the range where the effects of the present invention are not impaired and within the range where delayed-release properties are not compromised.

Examples of the diluent that can be used in the present invention may include starch, microcrystalline cellulose, lactose, glucose, mannitol, alginate, an alkaline earth metal salt, clay, polyethylene glycol, dicalcium phosphate, and a mixture thereof. Examples of the binder that can be used in the present invention include starch, microcrystalline cellulose, highly dispersive silica, mannitol, sucrose, lactose, polyethylene glycol, polyvinylpyrrolidone, hydroxypropylmethylcellulose, hydroxypropylcellulose, natural gum, synthetic gum, copovidone, povidone, gelatin, and a mixture thereof.

Examples of the disintegrant that can be used in the present invention may include starches or modified starches such as sodium starch glycolate, corn starch, potato starch, and pregelatinized starch, clays such as bentonite, montmorillonite, and veegum, celluloses such as microcrystalline cellulose, hydroxypropylcellulose, and carboxymethylcellulose, algins such as sodium alginate, and alginic acid, crosslinked celluloses such as croscarmellose sodium, gums such as guar gum, and xanthan gum, crosslinked polymers such as crosslinked polyvinylpyrrolidone (crospovidone), effervescent agents such as sodium bicarbonate and citric acid, and mixtures thereof.

Examples of the lubricant that can be used in the present invention may include talc, stearic acid, magnesium stearate, calcium stearate, sodium lauryl sulfate, hydrogenated vegetable oil, sodium benzoate, colloidal silicon dioxide, sodium stearyl fumarate, glyceryl behenate, glyceryl monolaurate, glyceryl monostearate, glyceryl palmitostearate and polyethylene glycol.

As the pharmaceutically acceptable additive, examples of the pH-control agent that can be used in the present invention may include acidifying agents such as acetic acid, adipic acid, ascorbic acid, malic acid, succinic acid, tartaric acid, fumaric acid, and citric acid, and alkalizing agents such as precipitated calcium carbonate, aqueous ammonia, and meglumine.

As the pharmaceutically acceptable additive of the present invention, examples of the anti-foaming agent may include dimethicone, oleyl alcohol, propylene glycol alginate, and simethicone such as simethicone emulsion.

As the pharmaceutically acceptable additive of the present invention, examples of the solubilizer may include sodium lauryl sulfate, polyoxyethylene sorbitan fatty acid ester such as polysorbate, and docusate sodium.

In addition, the formulation of the present invention may contain pharmaceutically acceptable additives such as various additives selected from a colorant and a fragrance. The range of the additives that can be used in the present invention is not limited to the above-mentioned additives, and the additive may be used in a conventional dose which can be suitably selected by those skilled in the art.

Further, in the delayed-release formulation of the present invention, as a binding solvent and a solvent for the delayed-release additive, there may be employed purified water, ethanol, methylene chloride, and the like. More preferred are purified water and ethanol.

In the pharmaceutically acceptable additive of the present invention, the range of the usable additive is not limited to the above-mentioned additive, and the additive may be used in a conventional dose which can be suitably selected by those skilled in the art.

The pharmaceutical formulation of the present invention can be prepared into various formulations, for example, tablets (such as uncoated tablets, coated tablets, multi-layered tablets, or press-coated tablets), powders, granules, or capsules.

The pharmaceutical formulation of the present invention may be in the form of an uncoated tablet which is obtained by optionally subjecting prior-release compartment-forming granules or the like and delayed-release compartment-forming granules or the like to post-mixing with additive(s), followed by compression, such that the prior-release compartment and the delayed-release compartment are present within a single tablet whereby active ingredients of individual compartments are separately released to exhibit the efficacy of each drug.

The pharmaceutical formulation of the present invention may be in the form of a two-phase matrix tablet which is obtained by uniformly mixing a delayed-release compartment and a prior-release compartment, followed by compression.

Further, the pharmaceutical formulation of the present invention may be in the form of a film-coated tablet including a tablet of a delayed-release compartment and a film-coated layer of a prior-release compartment enclosing the exterior of the tablet, whereby an active ingredient of the film-coated layer is first released as the film-coated layer is dissolved.

Further, the pharmaceutical formulation of the present invention may be in the form of a multi-layered tablet having a multi-layered structure of a delayed-release compartment and a prior-release compartment, each compartment of which is obtained by mixing the granules constituting the delayed-release compartment with pharmaceutical additives or mixing the granules constituting the prior-release compartment with pharmaceutical additives, and compressing the mixture into a double-layered or triple-layered tablet, using a multiple tablet press. The resulting formulation is a tablet for oral administration which was formulated to achieve the prior-release and delayed-release of drugs according to individual layers.

Further, the pharmaceutical formulation of the present invention may be in the form of a press-coated tablet including an inner core of a delayed-release compartment and an outer layer of a prior-release compartment enclosing the outer surface of the inner core. The press-coated tablet may be an osmotic press-coated tablet. The osmotic press-coated tablet is a formulation wherein the tablet mix is compressed into a tablet in a manner that an osmo-regulator is incorporated for the delayed-release of a drug, the tablet surface is coated with a semi-permeable membrane coating base to prepare an inner core, a granule constituting the prior-release compartment is mixed with pharmaceutical additives to prepare an outer layer, followed by compression to form a formulation having a delayed-release inner core and a prior-release layer enclosing the surface of the inner core.

The pharmaceutical formulation of the present invention may be in the form of a capsule containing a particle, granule, pellet, or tablet formed of a delayed-release compartment and a particle, granule, pellet, or tablet formed of a prior-release compartment.

The tablet formed of the delayed-release compartment of the capsule may be an osmotic coated tablet which contains an osmo-regulator inside the tablet and has a semi-permeable membrane coating base on the surface of the tablet.

The base material of the capsule may be one selected from gelatin, succinate gelatin, hydroxypropylmethylcellulose, and a mixture thereof.

The formulation of the present invention may further include a coating layer on the outside of the delayed-release compartment and/or the prior-release compartment. That is, the surface of particles, granules, pellets, or tablets formed of the delayed-release compartment and/or the prior-release compartment may be coated for the purpose of controlled release of drugs or stability of the formulation.

Further, the pharmaceutical formulation of the present invention may be in the form of a kit including a delayed-release compartment and a prior-release compartment. Specifically, the kit includes (a) prior-release compartment, (b) a delayed-release compartment, and (c) a container for filling the prior-release compartment and the delayed-release compartment. The kit can be prepared in the form of a kit wherein a particle, granule, pellet, or tablet constituting the prior-release compartment is prepared, a granule, pellet, or tablet constituting the delayed-release compartment is separately prepared, and the thus prepared two compartment materials are filled in a foil, blister, or bottle to prepare a dosage form for concurrent administration of different drugs.

The formulation of the present invention may be a formulation in the form of an uncoated tablet without further coating, or otherwise, if necessary, may be provided in the form of a coated tablet further including a coating layer formed on the outside of the formulation. The formation of a coating layer can provide a formulation which is capable of further securing stability of pharmacologically active ingredients.

A method for forming the coating layer may be suitably selected by a skilled person in the art, from among methods capable of forming a film-like coating layer on the surface of the tablet layer, such as a fluidized-bed coating method and a pan coating method. Preferably, a pan coating method may be used.

The coating layer may be formed by using a film-forming agent, a film-forming aid or a mixture thereof. For example, the film-forming agent may be cellulose derivatives such as hydroxypropylmethylcellulose and hydroxypropylcellulose, saccharide derivatives, polyvinyl derivatives, waxes, fats, gelatin and mixtures thereof, and the film-forming aid may be polyethylene glycol, ethylcellulose, glyceride, titanium oxide, talc, diethyl phthalate and mixtures thereof.

A content of the coating layer may be in the range of 0.5 to 15% by weight (% w/w) based on the total weight of the tablet.

If a content of the coating layer is lower than 0.5% by weight, it may be difficult to achieve the protection of products, and the stability thereof depending on formulations. On the other hand, if a content of the coating layer is higher than 15% by weight, release profiles of pharmacologically active ingredients may be affected.

Further, the delayed-release compartment in accordance with the present invention may also be administered simultaneously with a commercially available pharmacologically active ingredient of the prior-release compartment.

The present invention provides a method for preventing and treating at least one disease selected from a metabolic syndrome, a cardiovascular disease and a renal disease, including administering the pharmaceutical formulation of the present invention to a mammal including a human.

The cardiovascular disease is a very broad disease generically covering all of cardiovascular diseases and other blood vessel diseases including cerebrovascular diseases. The heart disease is represented by ischemic heart diseases (myocardial infarction, angina pectoris, etc.) due to the progression of arteriosclerosis and includes hypertension, heart failure, arrhythmia, myocardiopathy, endocarditis, etc. Examples of the blood vessel disease include cerebral stroke (palsy), peripheral vascular diseases, etc. Further, the cardiovascular disease includes hypertension and complications thereof of people suffering from a metabolic syndrome with combined manifestation of hypertension, diabetes, obesity, hyperlipidemia, and coronary artery diseases.

The pharmaceutical formulation of the present invention can be preferably formulated into a desired dosage form depending on individual diseases or ingredients, by an appropriate method known in the art, for example, using the principle of the chronotherapy as disclosed in Chronotherapeutics (2003, Peter Redfern, PhP), specifically by a method including the following steps.

Step 1 is a step of obtaining a delayed-release granule or tablet by subjecting a pharmacologically active ingredient of the delayed-release compartment and one or two release-controlling materials selected from the group consisting of an enteric polymer, a water-insoluble polymer, a hydrophobic compound, and a hydrophilic polymer together with a pharmaceutically acceptable conventional additive to mixing, kneading, drying, granulation or coating, and compression, or of obtaining a delayed-release granule or tablet by subjecting the pharmacologically active ingredient and an osmo-regulator together with a conventional pharmaceutically acceptable additive to mixing, kneading, drying, granulation or compression, followed by coating with a semi-permeable membrane coating base.

Step 2 is a step of obtaining a prior-release granule or tablet by subjecting a pharmacologically active ingredient of the prior-release compartment together with a conventional pharmaceutically acceptable additive to conventional processes for producing oral solid formulations, for example, mixing, kneading, drying, granulation or coating, and compression.

Step 3 is a step of obtaining a formulation for oral administration by mixing the granule or tablet obtained in Step 1 with a pharmaceutically acceptable excipient and/or the granule or tablet obtained in Step 2 with a pharmaceutically acceptable excipient and either compressing the mixture into a tablet or filling the mixture in a capsule for oral administration.

Step 1 may be carried out after Step 2, or Step 1 may be carried out simultaneously with Step 2.

The pharmaceutical formulation of the present invention can be prepared according to the above procedure, and a formulation method of Step 3 will be described in more detail hereinafter, but the present invention is not limited thereto.

### 1. Preparation of two-phase matrix tablets

The particles or granules prepared in Step 1 are optionally coated with a release-controlling material and then mixed with the granules prepared in Step 2, followed by compression into uniform weight, thereby preparing tablets. The resulting tablets may be film-coated for the purpose of improving the stability or shape, if necessary.

### 2. Preparation of film-coated tablets containing pharmacologically active ingredients

The coated tablets or granules prepared in Step 1 are optionally coated with a release-controlling material and dried, followed by compression into uniform weight and optionally further coating to prepare tablets. In addition, a pharmacologically active ingredient of the prior-release compartment is dissolved and dispersed in a water-soluble film coating solution and is coated on the outer layer of the tablets obtained in Step 1 to thereby prepare oral film-coated tablets containing pharmacologically active ingredients in the film coating.

### 3. Preparation of multi-layered tablets

The granules as it is prepared in Step 1 or optionally coated granules prepared in Step 1 with a release-controlling material and followed by drying are compressed with the granules prepared in Step 2 by using a multi-layered tablet press, thereby obtaining a double-layered tablet. According to the formulation design or if necessary, a triple or more multi-layered tablet may also be prepared by further adding a release adjuvant layer on the double-layered tablet. A coated multi-layered tablet may be prepared by coating the multi-layered tablet.

### 4. Preparation of press-coated tablets

The coated tablets or granules as it is prepared in Step 1 or optionally coated tablets or granules prepared in Step 1 with a release-controlling material and followed by drying are compressed into uniform weight. The resulting granules are used as an inner core optionally after performing further coating, and compressed with the granules prepared in Step 2 by using a press-coated tablet press, thereby providing press-coated tablets in where the surface of the tablet of Step 1 is enclosed by the prior-release layer. Coated press-coated tablets may be prepared by coating the press-coated tablets.

### 5. Preparation of capsules (containing granules or tablets)

The granules as it is prepared in Step 1 or the granules or the tablets prepared in Step 1 optionally coated with a release-controlling material, and followed by drying may be placed in a capsule filling machine together with the granules or the tablets prepared in Step 2, and filled in a capsule having a given size at an effective amount of each active ingredient, thereby preparing a capsule.

### 6. Preparation of capsules (pellets)

(1) A pharmacologically active ingredient of the delayed-release compartment, a release-controlling material, and if necessary, pharmaceutically acceptable additives are dissolved or suspended in water, an organic solvent, or a mixed solvent. This solution or suspension is coated on sugar spheres and dried, and if necessary, coated with one or more release-controlling materials dissolved in water, an organic solvent, or a mixed solvent, followed by drying. The mixture may be mixed with the granules prepared in Step 2 or the tablets obtained in Step 3 and then filled in capsules using a capsule filling machine, thereby preparing capsules.
(2) A pharmacologically active ingredient of the prior-release compartment and pharmaceutically acceptable additives may be dissolved or suspended in water, an organic solvent or a mixed solvent, coated on sugar spheres, followed by drying, and mixed with the controlled-release pellets of Section (1) containing a pharmacologically active ingredient of the delayed-release compartment and filled in capsules using a capsule filling machine to prepare capsules.

### 7. Preparation of kit

The formulation of Step 1 containing a pharmacologically active ingredient of the delayed-release compartment and the formulation of Step 2 containing a pharmacologically active ingredient of the prior-release compartment may be filled in a foil, blister, or bottle to prepare a kit for concurrent administration of different drugs.

### ADVANTAGEOUS EFFECTS

The formulation of the present invention can deliver a renin inhibitor and an angiotensin-II-receptor blocker with a time interval at a specific speed, thus reducing undesirable side-effects, improving the drug efficacy and promoting the patient compliance. Further, the pharmaceutical formulation of the present invention has pharmacological, clinical, scientific and economical advantages in the prevention or treatment of metabolic syndromes, cardiovascular diseases, renal diseases and the like, as compared with the complex drug regimens in which medicament ingredients are taken individually or simultaneously.

### DESCRIPTION OF THE DRAWINGS

FIG. 1 is a graph showing the comparative dissolution profiles of an aliskiren-irbesartan two-phase matrix tablet prepared in Example I-1, and the aliskiren and irbesartan ingredients of single drugs, Tektuma and Aprovel, as control drugs.
FIG. 2 is a graph showing the comparative dissolution profiles of an aliskiren-irbesartan two-phase matrix tablet prepared in Example I-4, and the aliskiren and irbesartan ingredients of single drugs, Tektuma and Aprovel, as control drugs.
FIG. 3 is a graph showing the comparative dissolution profiles of an aliskiren-valsartan multi-layered tablet prepared in Example I-10, and the aliskiren and valsartan ingredients of single drugs, Tektuma and Diovan, as control drugs.
FIG. 4 is a graph showing the comparative dissolution profiles of an aliskiren-valsartan multi-layered tablet prepared in Example I-9, and the aliskiren and valsartan ingredients of single drugs, Tektuma and Diovan, as control drugs.
FIG. 5 is a graph showing the dissolution profiles of aliskiren ingredients of formulations prepared in Examples I-12 to I-15.
FIG. 6 is a graph showing the dissolution profiles of aliskiren ingredients of formulations prepared in Examples I-14, and I-16 to I-18.
FIG. 7 is a graph showing the comparative dissolution profiles of an aliskirenolmesartan medoxomil capsule (pellet-tablet) prepared in Example I-23, and the aliskiren and olmesartan ingredients of single drugs, Tektuma and Olmetec, as control drugs.
FIG. 8 is a graph showing the comparative dissolution profiles of an aliskirencandesartan cilexetil capsule (granule-granule) prepared in Example I-25, and the aliskiren and candesartan ingredients of single drugs, Tektuma and Atacand, as control drugs.
FIG. 9 is a graph showing the comparative dissolution profiles of an aliskiren-irbesartan-containing formulation prepared in Example II-1, and the aliskiren single drug Tektuma and the irbesartan single drug Aprovel, as control drugs.
FIG. 10 is a graph showing the comparative dissolution profiles of an aliskiren-irbesartan-containing formulation prepared in Example II-8, and the aliskiren single drug Tektuma and the irbesartan single drug Aprovel, as control drugs.
FIG. 11 is a graph showing the comparative dissolution profiles of aliskiren-irbesartan-containing formulations prepared in Examples II-2, II-3, II-6 and II-7, and the irbesartan single drug Aprovel as a control drug.
FIG. 12 is a graph showing the comparative dissolution profiles of irbesartan-aliskiren-containing formulations prepared in Examples II-9, II-10, II-13 and II-14, and the aliskiren single drug Tektuma as a control drug.
FIG. 13 is a graph showing the comparative dissolution profiles of a formulation prepared in Example II-1, and the aliskiren single drug Tektuma and the valsartan single drug Diovan, as control drugs.
FIG. 14 is a graph showing the comparative dissolution profiles of a formulation prepared in Example III-8, and the valsartan single drug drug Diovan and the aliskiren single drug Tektuma, as control drugs.
FIG. 15 is a graph showing the comparative dissolution profiles of formulations prepared in Examples III-2, III-3, III-6 and III-7, and the valsartan single drug Diovan as a control drug.
FIG. 16 is a graph showing the comparative dissolution profiles of formulations prepared in Examples III-9, III-10, III-13 and III-14, and the aliskiren single drug Tektuma as a control drug.

### MODE FOR INVENTION

Now, the present invention will be described in more detail with reference to the following Examples. These examples are provided only for illustrating the present invention and should not be construed as limiting the scope and spirit of the present invention.

### <Example I, Experimental Example I>

### Example I-1: Preparation of two-phase matrix tablets

According to the ingredient compositions and contents shown in Table 1 below, the preparation was carried out by the following procedure.

### 1) Preparation of aliskiren-containing delayed-release compartment

Aliskiren hemi-fumarate, microcrystalline cellulose, crosslinked polyvinylpyrrolidone, and sodium chloride were sieved through a No. 35 sieve and mixed in a high-speed mixer for 5 minutes to prepare a mixture. Meanwhile, polyvinylpyrrolidone was dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. Meanwhile, cellulose acetate (acetyl group 32%), cellulose acetate (acetyl group 39.8%), and hydroxypropylmethylcellulose were dissolved and dispersed in 220 mg of ethanol and 980 mg of methylene chloride to prepare a coating solution which was then coated on the granules in a fluidized bed coater (GPCG-1: Glatt, Germany) to prepare aliskiren hemi-fumarate delayed-release granules.

### 2) Preparation of irbesartan-containing prior-release compartment

Irbesartan (Ranbaxy, India), microcrystalline cellulose, lactose, and croscarmellose sodium were sieved through a No. 35 sieve and mixed in a high-speed mixer. Meanwhile, pregelatinized starch and Poloxamer 188 were dissolved in water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. After completion of the drying process, the granules were sieved again through a No. 20 sieve.

### 3) Compression and coating

The final products of Processes 1) and 2) were placed and mixed in a double cone mixer. To the mixture was added colloidal silicon dioxide, followed by mixing, and magnesium stearate was added thereto, followed by final mixing. The final mixture was compressed into tablets using a rotary tablet press (MRC-33: Sejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example I-2: Preparation of two-phase matrix tablets

According to the ingredient compositions and contents shown in Table 1 below, the preparation was carried out by the following procedure.

### 1) Preparation of aliskiren-containing delayed-release compartment

Aliskiren hemi-fumarate and microcrystalline cellulose were sieved through a No. 35 sieve and mixed in a high-speed mixer for 5 minutes to prepare a mixture. Meanwhile, polyvinylpyrrolidone was dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. Then, a mixed solution (10% w/w) of hydroxypropylmethylcellulose and hydroxypropylmethylcellulose phthalate in a 1:1 mixture of ethanol and methylene chloride was sprayed on granules to prepare delayed-release granules.

### 2) Preparation of valsartan-containing prior-release compartment

Valsartan (Dr. Reddy, India), and microcrystalline cellulose and crosslinked polyvinylpyrrolidone (as excipients) were sieved through a No. 35 sieve and mixed in a high-speed mixer. Meanwhile, hydroxypropylcellulose was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve.

### 3) Compression and coating

The final products of Processes 1) and 2) were placed and mixed in a double cone mixer. To the mixture was added colloidal silicon dioxide, followed by mixing, and magnesium stearate was added thereto, followed by final mixing. The final mixture was compressed into tablets using a rotary tablet press (MRC-33: Sejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example I-3: Preparation of two-phase matrix tablets

According to the ingredient compositions and contents shown in Table 1 below, the preparation was carried out by the following procedure.

### 1) Preparation of aliskiren-containing delayed-release compartment

Aliskiren hemi-fumarate and microcrystalline cellulose were sieved through a No. 35 sieve and mixed in a high-speed mixer for 5 minutes to prepare a mixture. Meanwhile, polyvinylpyrrolidone was dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. The dried material was placed in a fluidized bed coater (GPCG-1: Glatt, Germany), and Kollicoat SR 30D as a coating solution was coated thereon to prepare aliskiren hemi-fumarate delayed-release granules.

### 2) Preparation of telmisartan-containing prior-release compartment

Telmisartan (Ranbaxy, India), microcrystalline cellulose, and sorbitol were sieved through a No. 35 sieve and mixed in a high-speed mixer. Meanwhile, polyvinylpyrrolidone, sodium hydroxide, and meglumine (Sigma) were dissolved in water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. After completion of the drying process, the granules were sieved again through a No. 20 sieve.

### 3) Compression and coating

The final products of Processes 1) and 2) were placed and mixed in a double cone mixer. To the mixture was added magnesium stearate, followed by final mixing. The final mixture was compressed into tablets using a rotary tablet press (MRC-33: Sejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example I-4: Preparation of two-phase matrix tablets

According to the ingredient compositions and contents shown in Table 1 below, the preparation was carried out by the following procedure.

### 1) Preparation of irbesartan-containing delayed-release compartment

Irbesartan, lactose, croscarmellose sodium, and sodium chloride were sieved through a No. 35 sieve and mixed in a high-speed mixer for 5 minutes to prepare a mixture. Meanwhile, pregelatinized starch and Poloxamer 188 were dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. The dried material was placed in a fluidized bed coater. Meanwhile, cellulose acetate (acetyl group 32%), cellulose acetate (acetyl group 39.8%), and hydroxypropylmethylcellulose were dissolved and dispersed in 220 mg of ethanol and 980 mg of methylene chloride to prepare a coating solution which was then coated on the granules in a fluidized bed coater (GPCG-1: Glatt, Germany). To the coated material were added microcrystalline cellulose and colloidal silicon dioxide, followed by mixing to prepare irbesartan-containing delayed-release granules.

### 2) Preparation of aliskiren-containing prior-release compartment

Aliskiren hemi-fumarate (as a renin inhibitor), and microcrystalline cellulose and crosslinked polyvinylpyrrolidone (as excipients) were sieved through a No. 35 sieve and mixed in a high-speed mixer. Meanwhile, polyvinylpyrrolidone was dissolved in water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. After completion of the drying process, the granules were sieved again through a No. 20 sieve.

### 3) Compression and coating

The final products of Processes 1) and 2) and colloidal silicon dioxide were placed and mixed in a double cone mixer. To the mixture was added magnesium stearate, followed by final mixing. The final mixture was compressed into tablets using a rotary tablet press (MRC-33: Sejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example I-5: Preparation of two-phase matrix tablets

According to the ingredient compositions and contents shown in Table 1 below, the preparation was carried out by the following procedure.

### 1) Preparation of valsartan-containing delayed-release compartment

Valsartan and microcrystalline cellulose were sieved through a No. 35 sieve and mixed in a high-speed mixer for 5 minutes to prepare a mixture. Meanwhile, polyvinylpyrrolidone was dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. Then, a mixed solution (10% w/w) of hydroxypropylmethylcellulose and hydroxypropylmethylcellulose phthalate in a 1:1 mixture of ethanol and methylene chloride was sprayed on the granules to prepare delayed-release granules.

### 2) Preparation of aliskiren-containing prior-release compartment

Aliskiren, and microcrystalline cellulose and crosslinked polyvinylpyrrolidone (as excipients) were sieved through a No. 35 sieve and mixed in a high-speed mixer. Meanwhile, polyvinylpyrrolidone was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve.

### 3) Compression and coating

The final products of Processes 1) and 2) were placed and mixed in a double cone mixer. To the mixture was added colloidal silicon dioxide, followed by mixing, and magnesium stearate was added thereto, followed by final mixing. The final mixture was compressed into tablets using a rotary tablet press (MRC-33: Sejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example I-6: Preparation of two-phase matrix tablets

According to the ingredient compositions and contents shown in Table 1 below, the preparation was carried out by the following procedure.

### 1) Preparation of telmisartan-containing delayed-release compartment

Telmisartan, microcrystalline cellulose, and sorbitol were sieved through a No. 35 sieve and mixed in a high-speed mixer. Meanwhile, polyvinylpyrrolidone, sodium hydroxide, and meglumine (Sigma) were dissolved in water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. The dried material was placed in a fluidized bed coater (GPCG-1: Glatt, Germany), and Kollicoat SR 30D as a coating solution was coated thereon to prepare telmisartan-containing delayed-release granules.

### 2) Preparation of aliskiren-containing prior-release compartment

Aliskiren, and microcrystalline cellulose and crosslinked polyvinylpyrrolidone (as excipients) were sieved through a No. 35 sieve and mixed in a high-speed mixer. Meanwhile, polyvinylpyrrolidone was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve.

### 3) Compression and coating

The final products of Processes 1) and 2) and colloidal silicon dioxide were placed and mixed in a double cone mixer. To the mixture was added magnesium stearate, followed by final mixing. The final mixture was compressed into tablets using a rotary tablet press (MRC-3 3 : S ejong, South Korea). Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing two-phase matrix tablets.

### Example I-7: Preparation of multi-layered tablets

According to the ingredient compositions and contents shown in Table 1 below, the preparation was carried out by the following procedure.

### 1) Preparation of irbesartan-containing delayed-release compartment

Irbesartan, lactose, croscarmellose sodium, and sodium chloride were sieved through a No. 35 sieve and mixed in a high-speed mixer for 5 minutes to prepare a mixture. Meanwhile, pregelatinized starch and Poloxamer 188 were dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. The dried material was placed in a fluidized bed coater. Meanwhile, cellulose acetate (acetyl group: 32%), cellulose acetate (acetyl group: 39.8%), and hydroxypropylmethylcellulose were dissolved in 220 mg of ethanol and 980 mg of methylene chloride to prepare a solution which was then coated on the granules in a fluidized bed coater (GPCG-1: Glatt, Germany). After completion of the coating process, microcrystalline cellulose and colloidal silicon dioxide were added and mixed with the coated granules, and magnesium stearate was added thereto, followed by final mixing in a double cone mixer for 4 minutes to prepare an irbesartan-containing delayed-release compartment.

### 2) Preparation of aliskiren-containing prior-release compartment

Aliskiren hemi-fumarate (as a renin inhibitor), and microcrystalline cellulose and crosslinked polyvinylpyrrolidone (as excipients) were sieved through a No. 35 sieve and mixed in a high-speed mixer. Meanwhile, polyvinylpyrrolidone was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve. The sieved material was mixed with colloidal silicon dioxide, and magnesium stearate was added thereto, followed by final mixing in a double cone mixer.

### 3) Compression and coating

Tablet compression was carried out using a multi-layered tablet press (MRC-37T: Sejong, South Korea). The final product of Process 2) was placed in a first powder feeder, and the final product of Process 1) was placed in a second powder feeder. The products in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing a formulation in the form of a multi-layered tablet.

### Example I-8: Preparation of multi-layered tablets

According to the ingredient compositions and contents shown in Table 1 below, the preparation was carried out by the following procedure.

### 1) Preparation of irbesartan-containing delayed-release compartment

Irbesartan, lactose, and croscarmellose sodium were sieved through a No. 35 sieve and mixed in a high-speed mixer for 5 minutes to prepare a mixture. Meanwhile, pregelatinized starch and Poloxamer 188 were dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. Then, a mixed solution (10% w/w) of hydroxypropylmethylcellulose and hydroxypropylmethylcellulose phthalate in a 1:1 mixture of ethanol and methylene chloride was sprayed on the granules to prepare delayed-release granules. Colloidal silicon dioxide was added and mixed with the granules, and magnesium stearate was added thereto, followed by final mixing in a double cone mixer.

### 2) Preparation of aliskiren-containing prior-release compartment

Aliskiren hemi-fumarate (as a renin inhibitor), and microcrystalline cellulose and crosslinked polyvinylpyrrolidone (as excipients) were sieved through a No. 35 sieve and mixed in a high-speed mixer. Meanwhile, polyvinylpyrrolidone was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve. The sieved material was mixed with colloidal silicon dioxide, and magnesium stearate was added thereto, followed by final mixing in a double cone mixer.

### 3) Compression and coating

Tablet compression was carried out using a multi-layered tablet press (MRC-37T: Sejong, South Korea). The final product of Process 2) was placed in a first powder feeder, and the final product of Process 1) was placed in a second powder feeder. The products in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing a formulation in the form of a multi-layered tablet.

### Example I-9: Preparation of multi-layered tablets

According to the ingredient compositions and contents shown in Table 1 below, the preparation was carried out by the following procedure.

### 1) Preparation of valsartan-containing delayed-release compartment

Valsartan, crospovidone, and microcrystalline cellulose were sieved through a No. 35 sieve and mixed in a high-speed mixer for 5 minutes to prepare a mixture. Meanwhile, polyvinylpyrrolidone was dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. The dried material was placed in a fluidized bed coater (GPCG-1: Glatt, Germany), and Kollicoat SR 30D as a coating solution was coated thereon to prepare aliskiren hemi-fumarate delayed-release granules.

Magnesium stearate was added to the granules, followed by final mixing in a double cone mixer for 4 minutes to prepare a valsartan-containing delayed-release compartment.

### 2) Preparation of aliskiren-containing prior-release compartment

Aliskiren hemi-fumarate (as a renin inhibitor), and microcrystalline cellulose and crosslinked polyvinylpyrrolidone (as excipients) were sieved through a No. 35 sieve and mixed in a high-speed mixer. Meanwhile, polyvinylpyrrolidone was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve. The sieved material was mixed with colloidal silicon dioxide, and magnesium stearate was added thereto, followed by final mixing in a double cone mixer.

### 3) Compression and coating

Tablet compression was carried out using a multi-layered tablet press (MRC-37T: Sejong, South Korea). The final product of Process 2) was placed in a first powder feeder, and the final product of Process 1) was placed in a second powder feeder. The products in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing a formulation in the form of a multi-layered tablet.

### Example I-10: Preparation of multi-layered tablets

According to the ingredient compositions and contents shown in Table 2 below, the preparation was carried out by the following procedure.

### 1) Preparation of aliskiren-containing delayed-release compartment

Aliskiren hemi-fumarate (as a renin inhibitor), and microcrystalline cellulose, crosslinked polyvinylpyrrolidone and sodium chloride (as excipients) were sieved through a No. 35 sieve and mixed in a high-speed mixer for 5 minutes to prepare a mixture. Meanwhile, polyvinylpyrrolidone was dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. The dried material was placed in a fluidized bed coater. Meanwhile, cellulose acetate (acetyl group 32%), cellulose acetate (acetyl group 39.8%), and hydroxypropylmethylcellulose were dissolved and dispersed in 220 mg of ethanol and 980 mg of methylene chloride to prepare a solution which was then coated on the granules in a fluidized bed coater (GPCG-1: Glatt, Germany). After completion of the coating process, magnesium stearate was added thereto, followed by mixing for 4 minutes to prepare an aliskiren hemi-fumarate-containing delayed-release compartment.

### 2) Preparation of valsartan-containing prior-release compartment

Valsartan (as an angiotensin-II-receptor blocker), and microcrystalline cellulose and crosslinked polyvinylpyrrolidone (as excipients) were sieved through a No. 35 sieve and mixed in a high-speed mixer. Meanwhile, hydroxypropylmethylcellulose was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve. The sieved material was mixed with colloidal silicon dioxide, and magnesium stearate was added thereto, followed by final mixing in a double cone mixer.

### 3) Compression and coating

Tablet compression was carried out using a multi-layered tablet press (MRC-37T: Sejong, South Korea). The final product of Process 2) was placed in a first powder feeder, and the final product of Process 1) was placed in a second powder feeder. The products in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing a formulation in the form of a multi-layered tablet.

### Example I-11: Preparation of multi-layered tablets

According to the ingredient compositions and contents shown in Table 2 below, the preparation was carried out by the following procedure.

### 1) Preparation of aliskiren-containing delayed-release compartment

Aliskiren hemi-fumarate, and microcrystalline cellulose and crosslinked polyvinylpyrrolidone (as excipients) were sieved through a No. 35 sieve and mixed in a high-speed mixer for 5 minutes to prepare a mixture. Meanwhile, polyvinylpyrrolidone was dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. Then, a mixed solution (10% w/w) of hydroxypropylcellulose and hydroxypropylmethylcellulose phthalate in a 1:1 mixture of ethanol and methylene chloride was sprayed on the granules to prepare delayed-release granules.

After completion of the coating process, magnesium stearate was added thereto, followed by mixing for 4 minutes to prepare an aliskiren hemi-fumarate-containing delayed-release compartment.

### 2) Preparation of losartan-containing prior-release compartment

Losartan potassium (Losartan K, Cipla, India), microcrystalline cellulose, and pregelatinized starch were sieved through a No. 35 sieve and mixed in a high-speed mixer. Meanwhile, hydroxypropylmethylcellulose was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve. The sieved material was mixed with croscarmellose sodium, and magnesium stearate was added thereto, followed by final mixing in a double cone mixer.

### 3) Compression and coating

Tablet compression was carried out using a multi-layered tablet press (MRC-37T: Sejong, South Korea). The final product of Process 2) was placed in a first powder feeder, and the final product of Process 1) was placed in a second powder feeder. The products in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing a formulation in the form of a multi-layered tablet.

### Example I-12: Preparation of multi-layered tablets

According to the ingredient compositions and contents shown in Table 2 below, the preparation was carried out by the following procedure.

### 1) Preparation of aliskiren-containing delayed-release compartment

Aliskiren hemi-fumarate and microcrystalline cellulose were sieved through a No. 35 sieve and mixed in a high-speed mixer for 5 minutes to prepare a mixture. Meanwhile, polyvinylpyrrolidone was dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. The granules were placed in a fluidized bed coater (GPCG-1: Glatt, Germany). Then, a solution (10% w/w) of ethylcellulose and a methacrylic acid copolymer in a 1:1 mixture of ethanol and methylene chloride was sprayed thereon to prepare delayed-release granules.

After completion of the coating process, the granules and colloidal silicon dioxide were placed and mixed in a double cone mixer, and magnesium stearate was added thereto, followed by final mixing.

### 2) Preparation of irbesartan-containing prior-release compartment

Irbesartan, microcrystalline cellulose, lactose, and pregelatinized starch were sieved through a No. 35 sieve and mixed in a high-speed mixer. Meanwhile, poloxamer was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve. The sieved material was mixed with croscarmellose sodium, and magnesium stearate was added thereto, followed by final mixing in a double cone mixer.

### 3) Compression and coating

Tablet compression was carried out using a multi-layered tablet press (MRC-37T: Sejong, South Korea). The final product of Process 2) was placed in a first powder feeder, and the final product of Process 1) was placed in a second powder feeder. The products in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing a formulation in the form of a multi-layered tablet.

### Examples I-13 to I-15: Preparation of multi-layered tablets

According to the ingredient compositions and contents shown in Table 2 below, multi-layered tablets of Examples 13 to 15 were prepared by the following procedure.

### 1) Preparation of aliskiren-containing delayed-release compartment

The preparation was carried out in the same manner as in Section 1) of Example I-12, except that ethylcellulose and a methacrylic acid copolymer, which are release-controlling materials, were used in the content given in Table 2.

### 2) Preparation of irbesartan-containing prior-release compartment

An irbesartan-containing prior-release compartment was prepared in the same manner as in Section 2) of Example I-12.

### 3) Compression and coating

Tablet compression was carried out using a multi-layered tablet press (MRC-37T: Sejong, South Korea). The final product of Process 2) was placed in a first powder feeder, and the final product of Process 1) was placed in a second powder feeder. The products in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing a formulation in the form of a multi-layered tablet.

### Example I-16: Preparation of multi-layered tablets

According to the ingredient compositions and contents shown in Table 2 below, the preparation was carried out by the following procedure.

### 1) Preparation of aliskiren-containing delayed-release compartment

Aliskiren hemi-fumarate, micro crystalline cellulose, and crosslinked polyvinylpyrrolidone were sieved through a No. 35 sieve and mixed in a high-speed mixer for 5 minutes to prepare a mixture. Meanwhile, polyvinylpyrrolidone was dissolved in purified water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. The granules were placed in a fluidized bed coater (GPCG-1: Glatt, Germany). Then, a solution (10% w/w) of ethylcellulose and a methacrylic acid copolymer in a 1:1 mixture of ethanol and methylene chloride was sprayed on the granules to prepare delayed-release granules.

After completion of the coating process, the granules and colloidal silicon dioxide were placed and mixed in a double cone mixer, and magnesium stearate was added thereto, followed by final mixing.

### 2) Preparation of irbesartan-containing prior-release compartment

An irbesartan-containing prior-release compartment was prepared in the same manner as in Section 2) of Example I-12.

### 3) Compression and coating

Tablet compression was carried out using a multi-layered tablet press (MRC-37T: Sejong, South Korea). The final product of Process 2) was placed in a first powder feeder, and the final product of Process 1) was placed in a second powder feeder. The products in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing a formulation in the form of a multi-layered tablet.

### Examples I-17 and I-18: Preparation of multi-layered tablets

According to the ingredient compositions and contents shown in Table 2 below, multi-layered tablets of Examples 17 and 18 were prepared by the following procedure.

### 1) Preparation of aliskiren-containing delayed-release compartment

Based on the composition and content of Table 2, an aliskiren-containing delayed-release compartment was prepared in the same manner as in Section 1) of Example I-16.

### 2) Preparation of irbesartan-containing prior-release compartment

An irbesartan-containing prior-release compartment was prepared in the same manner as in Section 2) of Example I-12.

### 3) Compression and coating

Tablet compression was carried out using a multi-layered tablet press (MRC-37T: Sejong, South Korea). The final product of Process 2) was placed in a first powder feeder, and the final product of Process 1) was placed in a second powder feeder. The products in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in 64.8 mg of ethanol and 16.2 mg of purified water to prepare a coating solution. The compressed tablets were coated with the coating solution in a Hi-coater (SFC-30N, Sejong Machinery Co., Ltd., South Korea) to form a film-coated layer, thereby preparing a formulation in the form of a multi-layered tablet.

### Example I-19: Preparation of press-coated tablets

According to the ingredient compositions and contents shown in Table 2 below, the preparation was carried out by the following procedure.

### 1) Preparation of valsartan-containing delayed-release compartment

Valsartan, microcrystalline cellulose, and crosslinked polyvinylpyrrolidone were sieved through a No. 35 sieve and mixed in a high-speed mixer for 5 minutes. Meanwhile, polyvinylpyrrolidone was dissolved in water to prepare a binding solution (10 w/w%) which was then sprayed thereon to form valsartan-containing granules, followed by drying. To the granule mixture was added magnesium stearate, followed by final mixing for 4 minutes. The final mixture was compressed into tablets using a rotary tablet press (MRC-33: Sejong, South Korea). The resulting tablets were used as an inner core tablet. Meanwhile, hydroxypropylmethylcellulose and hydroxypropylmethylcellulose phthalate were dissolved and dispersed in 132 mg of ethanol and 33 mg of purified water to prepare a coating solution. The inner core tablets were coated with the coating solution in a Hi-coater (SFC-30N: Sejong, South Korea) to form a delayed-release coated inner core in the form of a press-coated tablet.

### 2) Preparation of aliskiren-containing prior-release compartment

Aliskiren hemi-fumarate, micro crystalline cellulose, and crosslinked polyvinylpyrrolidone were sieved through a No. 35 sieve and mixed in a high-speed mixer. Meanwhile, polyvinylpyrrolidone was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve. Magnesium stearate was added to the sieved material, followed by final mixing in a double cone mixer.

### 3) Compression and coating

Using press-coated tablet press (RUD-1: Kilian), press-coated tablets were prepared including the coated valsartan tablet as an inner core and the aliskiren hemi-fumarate-containing composition as an outer layer. Meanwhile, hydroxypropylmethylcellulose 2910, hydroxypropylcellulose, titanium oxide, and talc were dissolved and dispersed in 132 mg of ethanol and 33 mg of purified water to prepare a coating solution. The compressed press-coated tablets were coated with the coating solution in a Hi-coater (SFC-30N: Sejong, South Korea) to prepare press-coated tablets.

### Example I-20: Preparation of press-coated tablets

According to the ingredient compositions and contents shown in Table 2 below, the preparation was carried out by the following procedure.

### 1) Preparation of aliskiren-containing delayed-release compartment

Aliskiren hemi-fumarate, micro crystalline cellulose, and crosslinked polyvinylpyrrolidone were sieved through a No. 35 sieve and mixed in a high-speed mixer for 5 minutes. Meanwhile, polyvinylpyrrolidone was dissolved in water to prepare a binding solution (10 w/w%) which was then sprayed thereon to form aliskiren-containing granules, followed by drying.

To the granule mixture was added magnesium stearate, followed by final mixing for 4 minutes. The final mixture was compressed into tablets using a rotary tablet press (MRC-33: Sejong, South Korea). The resulting tablets were used as an inner core tablet. Meanwhile, hydroxypropylmethylcellulose and hydroxypropylmethylcellulose phthalate were dissolved and dispersed in 132 mg of ethanol and 33 mg of purified water to prepare a coating solution. The inner core tablets were coated with the coating solution in a Hi-coater (SFC-30N: Sejong, South Korea) to form a delayed-release coated inner core in the form of a press-coated tablet.

### 2) Preparation of valsartan-containing prior-release compartment

Valsartan, microcrystalline cellulose, and crosslinked polyvinylpyrrolidone were sieved through a No. 35 sieve and mixed in a high-speed mixer. Meanwhile, polyvinylpyrrolidone was dissolved in water to prepare a binding solution. The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve. Magnesium stearate was added to the sieved material, followed by final mixing in a double cone mixer.

### 3) Compression and coating

Using press-coated tablet press (RUD-1: Kilian), press-coated tablets were prepared including the coated aliskiren hemi-fumarate-containing tablet as an inner core and the valsartan-containing composition as an outer layer. Meanwhile, hydroxypropylmethylcellulose 2910, hydroxypropylcellulose, titanium oxide, and talc were dissolved and dispersed in 132 mg of ethanol and 33 mg of purified water to prepare a coating solution. The compressed press-coated tablets were coated with the coating solution in a Hi-coater (SFC-30N: Sejong, South Korea) to prepare press-coated tablets.

### Example I-21: Preparation of capsules (pellets-granules)

According to the ingredient compositions and contents shown in Table 3 below, the preparation was carried out by the following procedure.

### 1) Preparation of aliskiren-containing delayed-release compartment

Sugar seeds (sugar spheres) were sieved through a No. 35 sieve and placed in a fluidized bed granulator (GPCG 1: Glatt). Meanwhile, hydroxypropylmethylcellulose and aliskiren hemi-fumarate were dissolved in a 2:8 mixture of water and ethanol to prepare a binding solution (20 w/w%) which was then sprayed thereon to form aliskiren hemi-fumarate-containing pellets, followed by drying. A solution of hydroxypropylmethylcellulose phthalate in 220 mg of ethanol and 980 mg of methylene chloride was sprayed on the pellets to prepare aliskiren hemi-fumarate delayed-release pellets.

### 2) Preparation of valsartan-containing prior-release compartment

Valsartan, and microcrystalline cellulose and crosslinked polyvinylpyrrolidone (as excipients) were sieved through a No. 35 sieve and mixed in a high-speed mixer. Meanwhile, hydroxypropylmethylcellulose was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve.

### 3) Mixing and capsule filling

The final compositions of Processes 1) and 2) were mixed in a double cone mixer. To the mixture was added magnesium stearate, followed by final mixing in a double cone mixer. The final mixture was placed in a powder feeder and then filled in capsules using a capsule filling machine (SF-40N, Sejong Pharmatech Co., Ltd., South Korea), thereby preparing a formulation in the form of a capsule.

### Example I-22: Preparation of capsules (pellets-granules)

According to the ingredient compositions and contents shown in Table 3 below, the preparation was carried out by the following procedure.

### 1) Preparation of losartan-containing delayed-release compartment

Sugar seeds (sugar spheres) were sieved through a No. 35 sieve and placed in a fluidized bed granulator (GPCG 1: Glatt). Meanwhile, hydroxypropylmethylcellulose and losartan potassium were dissolved in a 2:8 mixture of water and ethanol to prepare a binding solution (10 w/w%) which was then sprayed thereon to form losartan-containing pellets, followed by drying. A solution of hydroxypropylmethylcellulose phthalate in 220 mg of ethanol and 980 mg of methylene chloride was sprayed on the pellets to prepare losartan delayed-release pellets.

### 2) Preparation of aliskiren-containing prior-release compartment

Aliskiren hemi-fumarate, microcrystalline cellulose and crosslinked polyvinylpyrrolidone were sieved through a No. 35 sieve and mixed in a high-speed mixer. Meanwhile, polyvinylpyrrolidone was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve.

### 3) Mixing and capsule filling

The final products of Processes 1) and 2) were mixed in a double cone mixer. To the mixture was added magnesium stearate, followed by final mixing in a double cone mixer. The final mixture was placed in a powder feeder and then filled in capsules using a capsule filling machine, thereby preparing a formulation in the form of a capsule.

### Example I-23: Preparation of capsules (pellets-tablets)

According to the ingredient compositions and contents shown in Table 3 below, the preparation was carried out by the following procedure.

### 1) Preparation of aliskiren-containing delayed-release compartment

Sugar seeds (sugar spheres) were sieved through a No. 35 sieve and placed in a fluidized bed granulator (GPCG 1: Glatt). Meanwhile, hydroxypropylmethylcellulose and aliskiren hemi-fumarate were dissolved in a 2:8 mixture of water and ethanol to prepare a binding solution (10 w/w%) which was then sprayed thereon to form aliskiren hemi-fumarate-containing pellets, followed by drying. A solution of hydroxypropylmethylcellulose phthalate in 220 mg of ethanol and 980 mg of methylene chloride was sprayed on the pellets to prepare aliskiren hemi-fumarate delayed-release pellets.

### 2) Preparation of olmesartan-containing prior-release compartment

Olmesartan medoxomil (Ranbaxy, India), microcrystalline cellulose, and lactose were sieved through a No. 35 sieve and mixed in a high-speed mixer. Meanwhile, polyvinylpyrrolidone was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve. Low-substituted hydroxypropylcellulose was added to the sieved material, followed by mixing in a double cone mixer. To the mixture was added magnesium stearate, followed by final mixing. The final mixture was compressed into tablets using a rotary tablet press (MRC-33: Sejong, South Korea).

### 3) Capsule filling

The final products of Processes 1) and 2) were filled in capsules using a capsule filling machine, thereby preparing a formulation in the form of a capsule.

### Example I-24: Preparation of capsules (pellets-tablets)

According to the ingredient compositions and contents shown in Table 3 below, the preparation was by the following procedure.

### 1) Preparation of candesartan-containing delayed-release compartment

Sugar seeds (Sugar sphere) were sieved through a No. 35 sieve and placed in a fluidized bed granulator (GPCG 1: Glatt). Meanwhile, hydroxypropylmethylcellulose and candesartan cilexetil (Ranbaxy, India) were dissolved in a 2:8 mixture of water and ethanol to prepare a binding solution (10 w/w%) which was then sprayed on the granules to form candesartan-containing pellets, followed by drying. A solution of hydroxypropylmethylcellulose phthalate in 220 mg of ethanol and 980 mg of methylene chloride was sprayed thereon to prepare candesartan delayed-release pellets.

### 2) Preparation of aliskiren-containing prior-release compartment

Aliskiren hemi-fumarate, microcrystalline cellulose, and crosslinked polyvinylpyrrolidone were sieved through a No. 35 sieve and mixed in a high-speed mixer. Meanwhile, polyvinylpyrrolidone was dissolved in water to prepare a binding solution (10 w/w%). The binding solution and the mixture of main ingredients were placed in a high-speed mixer, followed by kneading. After completion of the kneading process, the kneaded material was granulated using an oscillator with a No. 20 sieve, and the granules were dried in a hot-water dryer at 60°C. After completion of the drying process, the granules were sieved again through a No. 20 sieve. The sieved material was placed in a double cone mixer, and magnesium stearate was added thereto, followed by final mixing. The final mixture was compressed into tablets using a rotary tablet press (MRC-33: Sejong, South Korea).

### 3) Capsule filling

The final products of Processes 1) and 2) were filled in capsules using a capsule filling machine, thereby preparing a formulation in the form of a capsule.

### Example I-25: Preparation of capsules (granules-granules)

According to the ingredient compositions and contents shown in Table 3 below, the preparation was carried out by the following procedure.

### 1) Preparation of aliskiren-containing delayed-release compartment

Aliskiren hemi-fumarate and microcrystalline cellulose were sieved through a No. 35 sieve and mixed in a high-speed mixer. Kollicoat SR 30D and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve to prepare aliskiren hemi-fumarate delayed-release granules.

### 2) Preparation of candesartan-containing prior-release compartment

Candesartan cilexetil, lactose, corn starch, and carboxymethylcellulose calcium were sieved through a No. 35 sieve and mixed in a high-speed mixer. Meanwhile, polyvinylpyrrolidone and polyethylene glycol were dissolved in water to prepare a binding solution. The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve.

### 3) Mixing and capsule filling

The final products of Processes 1) and 2) were mixed in a double cone mixer. To the mixture was added magnesium stearate, followed by final mixing in a double cone mixer. The final mixture was placed in a powder feeder and then filled in capsules using a capsule filling machine, thereby preparing a formulation in the form of a capsule.

### Example I-26: Preparation of capsules (granules-granules)

According to the ingredient compositions and contents shown in Table 3 below, the preparation was carried out by the following procedure.

### 1) Preparation of olmesartan-containing delayed-release compartment

Olmesartan medoxomil and microcrystalline cellulose were sieved through a No. 35 sieve and mixed in a high-speed mixer. Kollicoat SR 30D and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve to prepare olmesartan delayed-release granules.

### 2) Preparation of aliskiren-containing prior-release compartment

Aliskiren hemi-fumarate, microcrystalline cellulose, and crosslinked polyvinylpyrrolidone were sieved through a No. 35 sieve and mixed in a high-speed mixer. Meanwhile, polyvinylpyrrolidone was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve.

### 3) Mixing and capsule filling

The final products of Processes 1) and 2) were mixed in a double cone mixer. To the mixture was added magnesium stearate, followed by final mixing in a double cone mixer. The final mixture was placed in a powder feeder and then filled in capsules using a capsule filling machine, thereby preparing a formulation in the form of a capsule.

### Example I-27: Preparation of capsules (granules-tablets)

According to the ingredient compositions and contents shown in Table 3 below, the preparation was carried out by the following procedure.

### 1) Preparation of aliskiren-containing delayed-release compartment

Aliskiren hemi-fumarate and microcrystalline cellulose were sieved through a No. 35 sieve and placed and mixed in a fluidized bed granulator (GPCG 1: Glatt). Meanwhile, hydroxypropylmethylcellulose was dissolved in water to prepare a binding solution (10 w/w %) which was then sprayed thereon to form aliskiren hemi-fumarate-containing granules, followed by drying. A solution of hydroxypropylmethylcellulose phthalate in 220 mg of ethanol and 980 mg of methylene chloride was sprayed on the granules to prepare aliskiren hemi-fumarate delayed-release granules.

### 2) Preparation of irbesartan-containing prior-release compartment

Irbesartan, lactose, and croscarmellose sodium were sieved through a No. 35 sieve and mixed in a high-speed mixer. Meanwhile, pregelatinized starch and Poloxamer 188 were dissolved in water to prepare a binding solution (10% w/w). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve. To the sieved material were added microcrystalline cellulose and colloidal silicon dioxide, followed by mixing in a double cone mixer. To the mixture was added magnesium stearate, followed by final mixing. The final mixture was compressed into tablets using a rotary tablet press (MRC-33: Sejong, South Korea).

### 3) Capsule filling

The final products of Processes 1) and 2) were filled in capsules using a capsule filling machine, thereby preparing a formulation in the form of a capsule.

### Example I-28: Preparation of capsules (granules-tablets)

According to the ingredient compositions and contents shown in Table 3 below, the preparation was carried out by the following procedure.

### 1) Preparation of telmisartan-containing delayed-release compartment

Telmisartan, microcrystalline cellulose, and meglumine were sieved through a No. 35 sieve and placed in a fluidized bed granulator (GPCG 1: Glatt), followed by mixing. Meanwhile, polyvinylpyrrolidone, sorbitol, and sodium hydroxide were dissolved in water to prepare a binding solution (10 w/w%) which was then sprayed thereon to form telmisartan-containing granules, followed by drying. A solution of hydroxypropylmethylcellulose phthalate in 220 mg of ethanol and 980 mg of methylene chloride was sprayed on the granules to prepare telmisartan delayed-release granules.

### 2) Preparation of aliskiren-containing prior-release compartment

Aliskiren hemi-fumarate, micro crystalline cellulose, and crosslinked polyvinylpyrrolidone were sieved through a No. 35 sieve and mixed in a high-speed mixer. Meanwhile, polyvinylpyrrolidone was dissolved in water to prepare a binding solution (10 w/w%). The binding solution and the mixture of main ingredients were kneaded. After completion of the kneading process, the kneaded material was granulated using an oscillator with a No. 20 sieve, and the granules were dried in a hot-water dryer at 60°C. After completion of the drying process, the granules were sieved again through a No. 20 sieve. Magnesium stearate was added to the sieved material, followed by final mixing in a double cone mixer. The final mixture was compressed into tablets using a rotary tablet press (MRC-33: Sejong, South Korea).

### 3) Capsule filling

The final products of Processes 1) and 2) were filled in capsules using a capsule filling machine, thereby preparing a formulation in the form of a capsule.

### Example I-29: Preparation of aliskiren-valsartan blister package kits

Instead of 1) aliskiren delayed-release granules and 2) valsartan prior-release granules of Example I-10 being mixed and compressed, the granules of Process 1) of Example I-10 and Process 2) of Example I-10 were prepared, and the final products of individual processes were respectively compressed into tablets using a rotary tablet press (MRC-33, Sejong, South Korea), and were packed in a blister package container (silver foil, Dong-il Corporation, PVDC, Jeon Min Industry Co., Ltd., South Korea) such that they can be simultaneously administered, using a blister package machine (Minister A, Heung-A Engineering, South Korea).

### Example I-30: Preparation of aliskiren-irbesartan blister package kits

Instead of 1) irbesartan delayed-release granules and 2) aliskiren prior-release granules of Example I-7 being mixed and compressed, the granules of Process 1) of Example I-7 and Process 2) of Example I-7 were prepared, and the final products of individual processes were respectively compressed into tablets using a rotary tablet press (MRC-33, Sejong, South Korea), and were packed in a blister package container (silver foil, Dong-il Corporation, PVDC, Jeon Min Industry Co., Ltd., South Korea) such that they can be simultaneously administered, using a blister package machine (Minister A, Heung-A Engineering, South Korea).

**Table 1**

| Ingredients | | Content/unit formulation (mg) | | | | | | | | |
|---|---|---|---|---|---|---|---|---|---|---|
| | | Example No. | | | | | | | | |
| | | I-1 | I-2 | I-3 | I-4 | I-5 | I-6 | I-7 | I-8 | I-9 |
| Delayed-release compartment | Aliskiren hemi-fumarate | 167.8 | 331.5 | 167.8 | 0 | 0 | 0 | 0 | 0 | 0 |
| | Irbesartan | 0 | 0 | 0 | 150 | 0 | 0 | 150 | 150 | 0 |
| | Valsartan | 0 | 0 | 0 | 0 | 80 | 0 | 0 | 0 | 80 |
| | Telmisartan | 0 | 0 | 0 | 0 | 0 | 40 | 0 | 0 | 0 |
| | Microcrystalline cellulose | 100 | 137 | 123 | 45 | 60 | 123 | 45 | 0 | 60 |
| | Lactose | 0 | 0 | 0 | 30 | 0 | 0 | 30 | 30 | 0 |
| | Croscarmellose sodium | 0 | 0 | 0 | 15 | 0 | 0 | 15 | 15 | 0 |
| | Crospovidone | 0 | 0 | 0 | 0 | 0 | 0 | 0 | 0 | 15 |
| | Poloxamer 188¹) | 0 | 0 | 0 | 9 | 0 | 0 | 9 | 9 | 0 |
| | Pregelatinized starch | 0 | 0 | 0 | 45 | 0 | 0 | 45 | 45 | 0 |
| | Polyvinylpyrrolidone | 10 | 20 | 10 | 0 | 15 | 7 | 0 | 0 | 7 |
| | Sorbitol | 0 | 0 | 0 | 0 | 0 | 10 | 0 | 0 | 0 |
| | Sodium hydroxide | 0 | 0 | 0 | 0 | 0 | 3 | 0 | 0 | 0 |
| | Meglumine | 0 | 0 | 0 | 0 | 0 | 1 | 0 | 0 | 0 |
| | Crosslinked polyvinylpyrrolidone | 5 | 0 | 0 | 0 | 0 | 0 | 0 | 0 | 0 |
| | Kollicoat SR 30D²⁾ | 0 | 0 | 48 | 0 | 0 | 24 | 0 | 0 | 48 |
| | Hydroxypropylmethylcellulose | 4 | 8 | 0 | 4 | 8 | 0 | 4 | 8 | 0 |
| | Hydroxypropylmethylcellulose phthalate | 0 | 12 | 0 | 0 | 12 | 0 | 0 | 12 | 0 |
| | Cellulose acetate (acetyl group 32%) | 40 | 0 | 0 | 40 | 0 | 0 | 40 | 0 | 0 |
| | Cellulose acetate (acetyl group 39.8%) | 40 | 0 | 0 | 40 | 0 | 0 | 40 | 0 | 0 |
| | Sodium chloride | 50 | 0 | 0 | 50 | 0 | 0 | 50 | 0 | 0 |
| | Colloidal silicon dioxide³⁾ | 0 | 0 | 0 | 3 | 0 | 0 | 3 | 3 | 0 |
| | Magnesium stearate | 0 | 0 | 0 | 0 | 0 | 0 | 3 | 3 | 3 |
| Prior-release compartment | Aliskiren hemi-fumarate | 0 | 0 | 0 | 331.5 | 167.8 | 167.8 | 167.8 | 167.8 | 167.8 |
| | Irbesartan | 150 | 0 | 0 | 0 | 0 | 0 | 0 | 0 | 0 |
| | Valsartan | 0 | 80 | 0 | 0 | 0 | 0 | 0 | 0 | 0 |
| | Telmisartan | 0 | 0 | 40 | 0 | 0 | 0 | 0 | 0 | 0 |
| | Microcrystalline cellulose | 45 | 60 | 50 | 200 | 100 | 100 | 100 | 100 | 100 |
| | Lactose | 30 | 0 | 0 | 0 | 0 | 0 | 0 | 0 | 0 |
| | Croscarmellose sodium | 15 | 0 | 0 | 0 | 0 | 0 | 0 | 0 | 0 |
| | Crosslinked polyvinylpyrrolidone⁴⁾ | 0 | 15 | 0 | 10 | 5 | 5 | 5 | 5 | 5 |
| | Poloxamer 188¹⁾ | 9 | 0 | 0 | 0 | 0 | 0 | 0 | 0 | 0 |
| | Pregelatinized starch | 45 | 0 | 0 | 0 | 0 | 0 | 0 | 0 | 0 |
| | Hydroxypropylcellulose | 0 | 7 | 0 | 0 | 0 | 0 | 0 | 0 | 0 |
| | Polyvinylpyrrolidone | 0 | 0 | 7 | 20 | 10 | 10 | 10 | 10 | 10 |
| | Sorbitol | 0 | 0 | 10 | 0 | 0 | 0 | 0 | 0 | 0 |
| | Sodium hydroxide | 0 | 0 | 3 | 0 | 0 | 0 | 0 | 0 | 0 |
| | Meglumine | 0 | 0 | 1 | 0 | 0 | 0 | 0 | 0 | 0 |
| | Colloidal silicon dioxide | 0 | 0 | 0 | 0 | 0 | 0 | 3 | 3 | 3 |
| | Magnesium stearate | 0 | 0 | 0 | 0 | 0 | 0 | 3 | 3 | 3 |
| Post-mixing | Colloidal silicon dioxide | 3 | 3 | 0 | 3 | 3 | 3 | 0 | 0 | 0 |
| | Magnesium stearate | 3 | 3 | 3 | 3 | 3 | 3 | 0 | 0 | 0 |
| Coating layer | Hydroxypropylmethylcellulose 2910 | 7.2 | 6.8 | 4.7 | 10.1 | 4.7 | 5 | 7.3 | 6.1 | 5.1 |
| | Hydroxypropylcellulose | 7.2 | 6.8 | 4.7 | 10.1 | 4.7 | 5 | 7.3 | 6.1 | 5.1 |
| | Titanium oxide | 6.4 | 6 | 4.1 | 8.9 | 4.2 | 4.4 | 6.4 | 5.4 | 4.5 |
| | Talc | 4.3 | 4 | 2.8 | 6 | 2.8 | 3 | 4.3 | 3.6 | 3 |
| Total | | 741.9 | 700.1 | 479.1 | 1033.6 | .480.2 | 514.2 | 748.1 | 585 | 519.5 |
| 1)Poloxamer 188 (trade name: Lutrol® F68, BASF) | | | | | | | | | | |
| 2)Kollicoat SR 30D (Main ingredient: polyvinyl acetate 30% suspension, BASF) | | | | | | | | | | |
| 3) Colloidal silicon dioxide (trade name: Aerosil 200, Degussa) | | | | | | | | | | |
| 4) Crosslinked polyvinylpyrrolidone (trade name: Crospovidone, BASF) | | | | | | | | | | |
| 5) Hydroxypropylmethylcellulose 2910 (trade name: Pharmacoat 606, Shin-Etsu) | | | | | | | | | | |

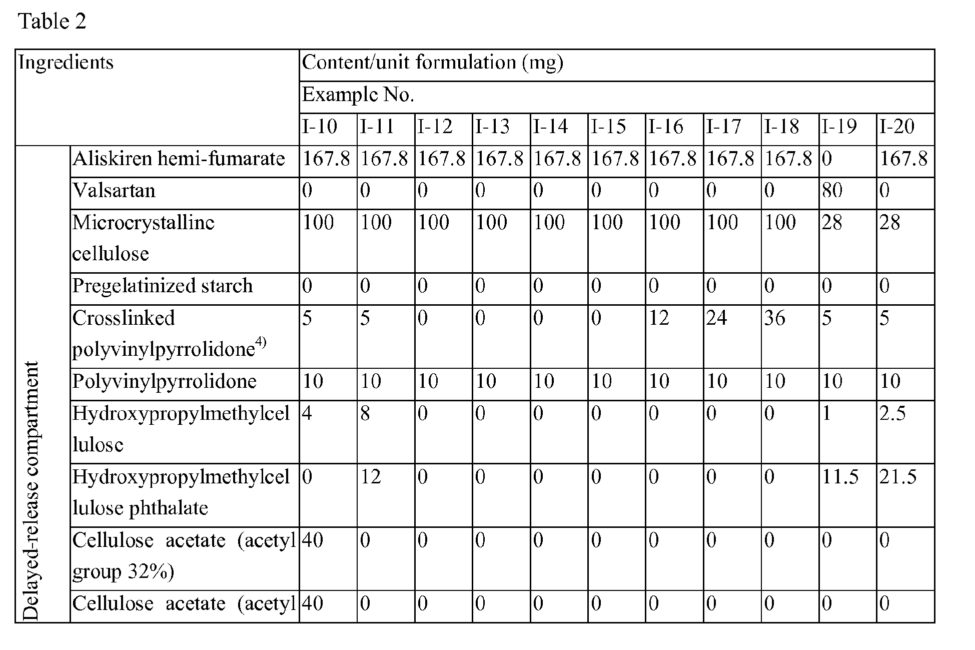

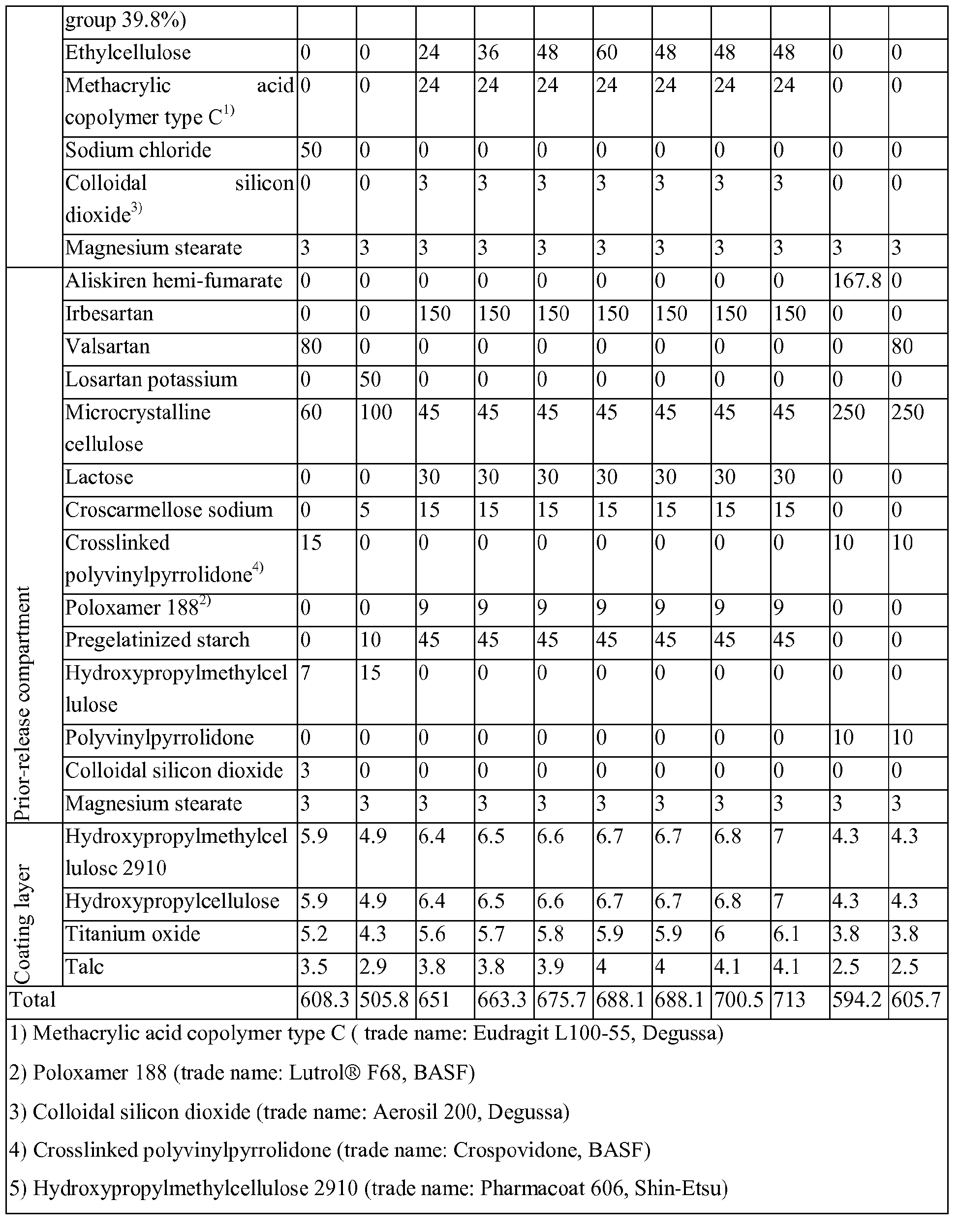

**Table 3**

| Ingredients | | Content/unit formulation (mg) | | | | | | | |
|---|---|---|---|---|---|---|---|---|---|
| | | Example No. | | | | | | | |
| | | I-21 | I-22 | I-23 | I-24 | I-25 | I-26 | I-27 | I-28 |
| Delayed-release compartment | Aliskiren hemi-fumarate | 167.8 | 0 | 167.8 | 0 | 167.8 | 0 | 167.8 | 0 |
| | Telmisartan | 0 | 0 | 0 | 0 | 0 | 0 | 0 | 40 |
| | Losartan potassium | 0 | 50 | 0 | 0 | 0 | 0 | 0 | 0 |
| | Olmesartan medoxomil | 0 | 0 | 0 | 0 | 0 | 20 | 0 | 0 |
| | Candesartan cilexetil | 0 | 0 | 0 | 16 | 0 | 0 | 0 | 0 |
| | Microcrystalline cellulose | 0 | 0 | 0 | 0 | 123 | 123 | 137 | 123 |
| | Polyvinylpyrrolidone | 0 | 0 | 0 | 0 | 0 | 0 | 0 | 7 |
| | Sorbitol | 0 | 0 | 0 | 0 | 0 | 0 | 0 | 10 |
| | Sodium hydroxide | 0 | 0 | 0 | 0 | 0 | 0 | 0 | 3 |
| | Meglumine | 0 | 0 | 0 | 0 | 0 | 0 | 0 | 1 |
| | Sugar seed | 35 | 35 | 35 | 35 | 0 | 0 | 0 | 0 |
| | Kollicoat SR 30D¹⁾ | 0 | 0 | 0 | 0 | 24 | 24 | 0 | 0 |
| | Hydroxypropylmethylcellulose | 5 | 5 | 5 | 5 | 0 | 0 | 8 | 0 |
| | Hydroxypropylmethylcellulose phthalate | 45 | 45 | 45 | 45 | 0 | 0 | 12 | 12 |
| Prior-release compartment | Aliskiren hemi-fumarate | 0 | 167.8 | 0 | 167.8 | 0 | 167.8 | 0 | 167.8 |
| | Irbesartan | 0 | 0 | 0 | 0 | 0 | 0 | 150 | 0 |
| | Valsartan | 80 | 0 | 0 | 0 | 0 | 0 | 0 | 0 |
| | Olmesartan medoxomil | 0 | 0 | 20 | 0 | 0 | 0 | 0 | 0 |
| | Candesartan cilexetil | 0 | 0 | 0 | 0 | 16 | 0 | 0 | 0 |
| | Microcrystalline cellulose | 60 | 100 | 50 | 100 | 0 | 100 | 45 | 100 |
| | Lactose | 0 | 0 | 50 | 0 | 50 | 0 | 30 | 0 |
| | Corn starch | 0 | 0 | 0 | 0 | 25 | 0 | 0 | 0 |
| | Low-substituted hydroxypropylcellulose⁵⁾ | 0 | 0 | 50 | 0 | 0 | 0 | 0 | 0 |
| | Croscarmellose sodium | 0 | 0 | 0 | 0 | 0 | 0 | 15 | 0 |
| | Crosslinked polyvinylpyrrolidone⁴⁾ | 15 | 5 | 0 | 5 | 0 | 5 | 0 | 5 |
| | Carboxymethylcellulose calcium | 0 | 0 | 0 | 0 | 20 | 0 | 0 | 0 |
| | Poloxamer 188²⁾ | 0 | 0 | 0 | 0 | 0 | 0 | 9 | 0 |
| | Pregelatinized starch | 0 | 0 | 0 | 0 | 0 | 0 | 45 | 0 |
| | Hydroxypropylmethylcellulose | 7 | 0 | 0 | 0 | 0 | 0 | 0 | 0 |
| | Polyvinylpyrrolidone | 0 | 10 | 7 | 10 | 7 | 10 | 0 | 10 |
| | Polyethylene glycol | 0 | 0 | 0 | 0 | 5 | 0 | 0 | 0 |
| | Colloidal silicon dioxide³⁾ | 0 | 0 | 0 | 0 | 0 | 0 | 3 | 0 |
| | Magnesium stearate | 0 | 0 | 3 | 3 | 0 | 0 | 3 | 3 |
| Post-mixing | Colloidal silicon dioxide | 0 | 0 | 0 | 0 | 0 | 0 | 0 | 0 |
| | Magnesium stearate | 3 | 3 | 0 | 0 | 3 | 3 | 0 | 0 |
| Total | | 417.8 | 420.8 | 432.8 | 386.8 | 440.8 | 452.8 | 624.8 | 481.8 |
| 1) Kollicoat SR 30D (Main ingredient: polyvinyl acetate 30% suspension, BASF) | | | | | | | | | |
| 2) Poloxamer 188 (trade name: Lutrol® F68, BASF) | | | | | | | | | |
| 3) Colloidal silicon dioxide (trade name: Aerosil 200, Degussa) | | | | | | | | | |
| 4) Crosslinked polyvinylpyrrolidone (trade name: Crospovidone, BASF) | | | | | | | | | |
| 5) Low-substituted hydroxypropylcellulose (trade name: LH-11, Shin-Etsu) | | | | | | | | | |

### Experimental Example I-1: Comparative dissolution profile test

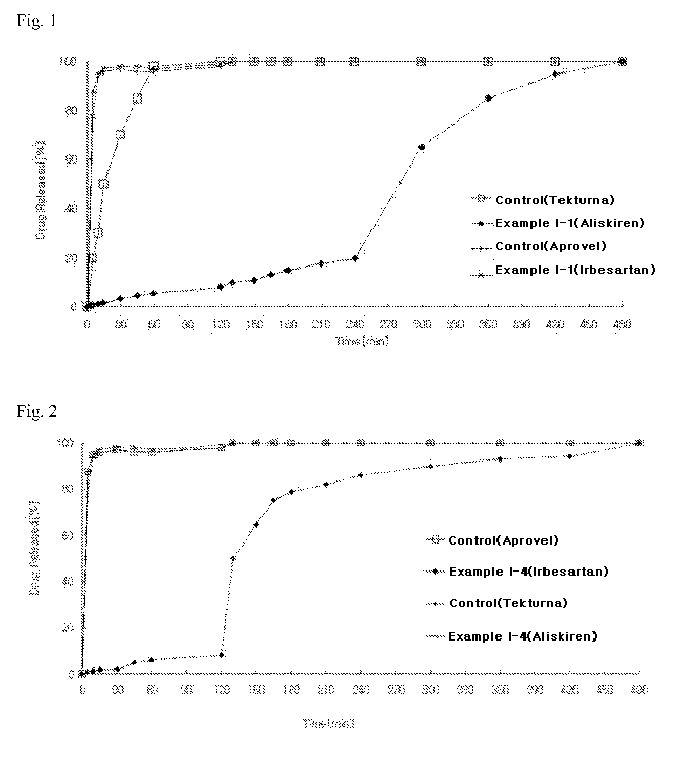

A comparative dissolution profile test was performed using aliskiren-irbesartan two-phase matrix tablets prepared in Example I-1 and control drugs (Aprovel, Sanofi-Aventis: irbesartan single drug, Tektuma, Novartis: aliskiren single drug). The dissolution profile test of the irbesartan ingredient was performed based on the United States Pharmacopoeia (USP30), and the dissolution profile test of the aliskiren ingredient was performed for a total of 480 minutes, in which the dissolution medium was changed from a simulated gastric juice to a simulated intestinal juice 120 minutes after the start of the test. Details of the dissolution profile test of each ingredient are as follows. The results obtained are shown in FIG. 1. In FIG. 1, the x-axis represents the time series (min), and the y-axis represents the dissolution rate (Drug Released, %).

As can be seen in FIG. 1, when the dissolution profile test was performed under the following conditions, the irbesartan ingredient of the two-phase matrix tablet of the present invention showed a dissolution profile substantially equal to that of the control drug Aprovel, but the aliskiren ingredient showed a very slow dissolution rate as compared to that of the control drug Tektuma. In the dissolution profile test results for the aliskiren ingredient, the dissolution rates of the aliskiren ingredient up to 120 minutes corresponding to the simulated gastric juice zone were less than 10% in the aliskiren/irbesartan two-phase matrix tablets of the present invention, but the control drugs showed about 100%. The dissolution rate of the aliskiren ingredient in the subsequent simulated intestinal juice zone was about 20% up to a total of 240 minutes in the aliskiren/irbesartan two-phase matrix tablets of the present invention, which was far lower than that of the control drugs.

As described above, the initial release of aliskiren in the aliskiren/irbesartan two-phase matrix tablets of the present invention is much slower than irbesartan, unlike dissolution profiles obtained when the aliskiren single drug and the irbesartan single drug, as the control drugs, are administered simultaneously. Thus, in the case of the inventive tablets, aliskiren is absorbed after irbesartan is absorbed first, thereby exhibiting no interaction therebetween.

### Test Method for irbesartan: Based on the "Irbesartan tablets" part in USP 30

Test method: Paddle method, 50 rpm
Dissolution medium: 0.01 N hydrochloric acid solution, 900 mL
Analysis method: High performance liquid chromatography

### Test Method for aliskiren hemi-fumarate: Based on the general dissolution test method described in the Korean Pharmacopoeia (8^{th} revision)

Test method: Paddle method, 50 rpm
Dissolution medium: 0.01 N hydrochloric acid solution 750 mL (simulated gastric juice), pH 6.8 phosphate buffer 1,000 mL (simulated intestinal juice)
Analysis method: High performance liquid chromatography

### Experimental Example I-2: Comparative dissolution profile test

A comparative dissolution profile test was performed using aliskiren-irbesartan two-phase matrix tablets prepared in Example I-4 and control drugs (Aprovel: irbesartan single drug, Tektuma: aliskiren single drug). The dissolution profile test of the aliskiren ingredient was performed based on the general dissolution test method described in the Korean Pharmacopoeia (8^{th} revision), and the dissolution profile test of the irbesartan ingredient was performed for a total of 480 minutes, in which the dissolution medium was changed from a simulated gastric juice to a simulated intestinal juice 120 minutes after the start of the test. Details of the dissolution profile test of each ingredient are as follows. The results obtained are shown in FIG. 2. The analysis method of aliskiren and irbesartan ingredients is the same as in Experimental Example I-1. In FIG. 2, the x-axis represents the time series (min), and the y-axis represents the dissolution rate (Drug Released, %).

As can be seen in FIG. 2, when the dissolution profile test was performed under the following conditions, the aliskiren ingredient of the two-phase matrix tablet of the present invention showed a dissolution profile substantially equal to that of the control drug Tektuma, but the irbesartan ingredient showed a very slow dissolution rate as compared to that of the control drug Aprovel. In the dissolution profile test results for the irbesartan ingredient, the dissolution rates of the irbesartan ingredient up to 120 minutes corresponding to the simulated gastric juice zone were all less than 10% in the aliskiren/irbesartan two-phase matrix tablets of the present invention, but the control drugs showed about 100%. The dissolution rate of the irbesartan ingredient in the subsequent simulated intestinal juice zone was about 65% up to a total of 150 minutes in the aliskiren/irbesartan two-phase matrix tablets of the present invention, which was far lower than that of the control drugs.

As described above, the initial release of irbesartan in the aliskiren/irbesartan two-phase matrix tablets of the present invention is much slower than aliskiren, unlike dissolution profiles obtained when the aliskiren single drug and the irbesartan single drug, as the control drugs, are administered simultaneously. Thus, in the case of the inventive tablets, irbesartan is absorbed after aliskiren is absorbed first, thereby exhibiting no interaction therebetween.

### Test Method for irbesartan: Based on the general dissolution test method described in the Korean Pharmacopoeia (8^{th} revision)

Test method: Paddle method, 50 rpm
Dissolution medium: 0.01 M hydrochloric acid solution 750 mL (simulated gastric juice), pH 6.8 phosphate buffer 1,000 mL (simulated intestinal juice)
Analysis method: High performance liquid chromatography

### Test Method for aliskiren hemi-fumarate: Based on the general dissolution test method described in the Korean Pharmacopoeia (8^{th} revision)

Test method: Paddle method, 50 rpm
Dissolution medium: Purified water, 900 mL
Analysis method: High performance liquid chromatography

### Experimental Example I-3: Comparative dissolution profile test

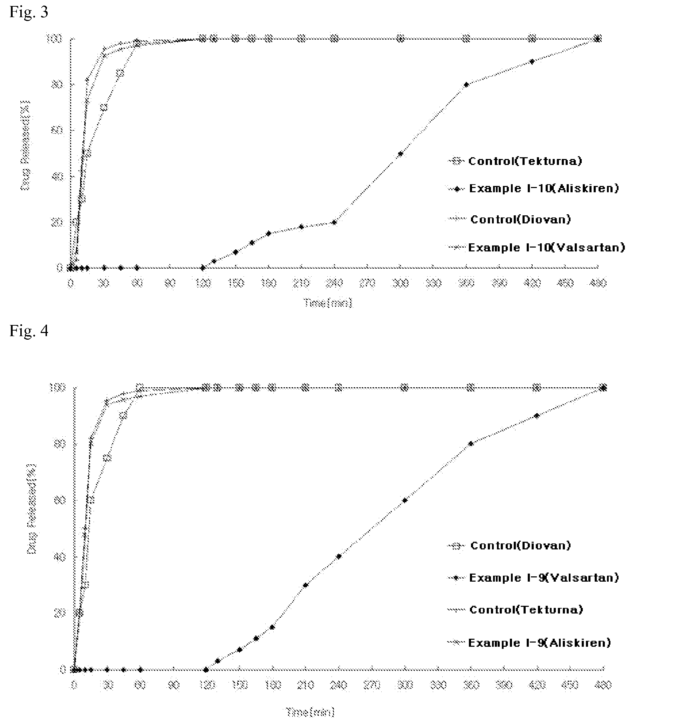

A comparative dissolution profile test was performed using aliskiren/valsartan multi-layered tablets prepared in Example I-10 and control drugs (Diovan, Novartis: valsartan single drug, Tektuma, Novartis: aliskiren single drug). The dissolution profile test of the valsartan ingredient was performed based on the United States Pharmacopoeia (USP30), and the dissolution profile test of the aliskiren ingredient was performed for a total of 480 minutes, in which the dissolution medium was changed from a simulated gastric juice to a simulated intestinal juice 120 minutes after the start of the test. Details of the dissolution profile test of the valsartan ingredient are as follows. The results obtained are shown in FIG. 3. In FIG. 3, the x-axis represents the time series (min), and the y-axis represents the dissolution rate (Drug Released, %).

### Details of the dissolution profile test of the aliskiren ingredient are the same as those of Experimental Example I-1.

As can be seen in FIG. 3, when the dissolution profile test was performed under the following conditions, the valsartan ingredient of the multi-layered tablet of the present invention showed a dissolution profile substantially equal to that of the control drug Diovan, but the aliskiren ingredient showed a very slow dissolution rate as compared to that of the control drug Tektuma. In the dissolution profile test results for the aliskiren ingredient, the dissolution rates of the aliskiren ingredient up to 120 minutes corresponding to the simulated gastric juice zone were all less than 10% in the aliskiren/irbesartan multi-layered tablets of the present invention, but the control drugs showed about 100%. The dissolution rate of the aliskiren ingredient in the subsequent simulated intestinal juice zone was about 20% up to a total of 240 minutes in the aliskiren/valsartan multi-layered tablets of the present invention, which was far lower than that of the control drugs.

As described above, the initial release of aliskiren in the aliskiren/valsartan multi-layered tablets of the present invention is much slower than valsartan, unlike dissolution profiles obtained when the aliskiren single drug and the valsartan single drug, as the control drugs, are administered simultaneously. Thus, in the case of the inventive tablets, aliskiren is absorbed after valsartan is absorbed first, thereby exhibiting no interaction therebetween.

### Test Method for valsartan: Based on the "valsartan and hydrochlorothiazide tablets" part in USP 30

Test method: Paddle method, 50 rpm
Dissolution medium: pH 6.8 phosphate buffer, 900 mL
Analysis method: UV-Vis spectrophotometry

### Experimental Example I-4: Comparative dissolution profile test

A comparative dissolution profile test was performed using aliskiren/valsartan multi-layered tablets prepared in Example I-9 and control drugs (Diovan: valsartan single drug, Tektuma: aliskiren single drug). The dissolution profile test of the aliskiren ingredient was performed based on the general dissolution test method described in the Korean Pharmacopoeia (8^{th} revision), and the dissolution profile test of the valsartan ingredient was performed for a total of 480 minutes, in which the dissolution medium was changed from a simulated gastric juice to a simulated intestinal juice 120 minutes after the start of the test. Details of the dissolution profile test of the valsartan ingredient are as follows. The results obtained are shown in FIG. 4. In FIG. 4, the x-axis represents the time series (min), and the y-axis represents the dissolution rate (Drug Released, %). Details of the dissolution profile test of the aliskiren ingredient are the same as those of Experimental Example I-2.

As can be seen in FIG. 4, when the dissolution profile test was performed under the following conditions, the aliskiren ingredient of the multi-layered tablets of the present invention showed a dissolution profile substantially equal to that of the control drug Tektuma, but the valsartan ingredient showed a very slow dissolution rate as compared to that of the control drug Diovan. In the dissolution profile test results for the valsartan ingredient, the dissolution rates of the valsartan ingredient up to 120 minutes corresponding to the simulated gastric juice zone were less than 10% in the aliskiren/valsartan multi-layered tablets of the present invention, but the control drugs showed about 100%. The dissolution rate of the valsartan ingredient in the subsequent simulated intestinal juice zone was about 40% up to a total of 240 minutes in the aliskiren/valsartan multi-layered tablets of the present invention, which was far lower than that of the control drugs.

As described above, the initial release of valsartan in the aliskiren/valsartan multi-layered tablets of the present invention is much slower than aliskiren, unlike dissolution profiles obtained when the aliskiren single drug and the valsartan single drug, as the control drugs, are administered simultaneously. Thus, in the case of the inventive tablets, valsartan is absorbed after aliskiren is absorbed first, thereby exhibiting no interaction therebetween.

### Test Method for valsartan: Based on the general dissolution test method described in the Korean Pharmacopoeia (8^{th} revision)

Test method: Paddle method, 50 rpm
Dissolution medium: 0.01 M hydrochloric acid solution 750 mL (simulated gastric juice), pH 6.8 phosphate buffer 1,000 mL (simulated intestinal juice)
Analysis method: UV-Vis spectrophotometry

### Experimental Example I-5: Comparative dissolution profile test

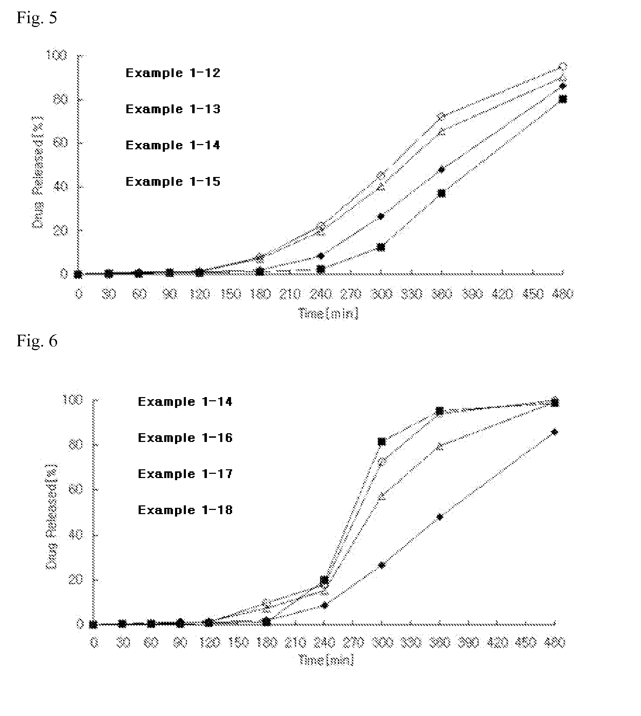

A comparative dissolution profile test was performed for the aliskiren ingredient of aliskiren-irbesartan multi-layered tablets of Examples I-12 to I-15. Details of the dissolution profile test of the aliskiren ingredient are the same as those of Experimental Example I-1. The results obtained are shown in FIG. 5. In FIG. 5, the x-axis represents the time series (min), and the y-axis represents the dissolution rate (Drug Released, %).

As can be seen in FIG. 5, when the dissolution profile test was performed under the conditions of Experimental Example I-1, the multi-layered tablets of the present invention showed a significant decrease in dissolution rate of the aliskiren ingredient in response to an increase in the amount of ethylcellulose to be used. When coated with ethylcellulose, Examples I-12 to I-15 exhibited the aliskiren dissolution rate of less than 20% up to a total of 240 minutes.

Therefore, the initial release of aliskiren in the aliskiren/irbesartan multi-layered tablet of the present invention can be delayed up to the intended time by controlling the amount of ethylcellulose coated.

As described above, the initial release of aliskiren in the aliskiren/irbesartan multi-layered tablets of the present invention is much slower than irbesartan, unlike dissolution profiles obtained when the aliskiren single drug and the irbesartan single drug, as the control drugs, are administered simultaneously. Thus, in the case of the inventive tablets, aliskiren is absorbed after irbesartan is absorbed first, thereby exhibiting no interaction therebetween.

### Experimental Example I-6: Comparative dissolution profile test

A comparative dissolution profile test was performed for the aliskiren ingredient of aliskiren-irbesartan multi-layered tablets of Examples I-14, I-16 to I-18. Details of the dissolution profile test of the aliskiren ingredient are the same as those of Experimental Example I-1. The results obtained are shown in FIG. 6. In FIG. 6, the x-axis represents the time series (min), and the y-axis represents the dissolution rate (Drug Released, %).

As can be seen in FIG. 6, when the dissolution profile test was performed under the conditions of Experimental Example I-1, the multi-layered tablets of the present invention showed relatively rapid release of the aliskiren ingredient after an intended lag time when crosslinked polyvinylpyrrolidone is incorporated in the ethylcellulose-coated delayed-release compartment. The dissolution rate of the aliskiren ingredient was less than 20% up to a total of 240 minutes and the aliskiren ingredient was rapidly released with an increase in the amount of crosslinked polyvinylpyrrolidone to be used.

Therefore, the aliskiren/irbesartan multi-layered tablet of the present invention can achieve rapid release of aliskiren after an intended lag time by controlling the content of crosslinked polyvinylpyrrolidone in the ethylcellulose-coated delayed-release layer.

As described above, the initial release of aliskiren in the aliskiren/irbesartan multi-layered tablet of the present invention is much slower than irbesartan, unlike dissolution profiles obtained when the aliskiren single drug and the irbesartan single drug, as the control drugs, are administered simultaneously. Thus, in the case of the inventive tablets, aliskiren is absorbed after irbesartan is absorbed first, thereby exhibiting no interaction therebetween.

### Experimental Example I-7: Comparative dissolution profile test

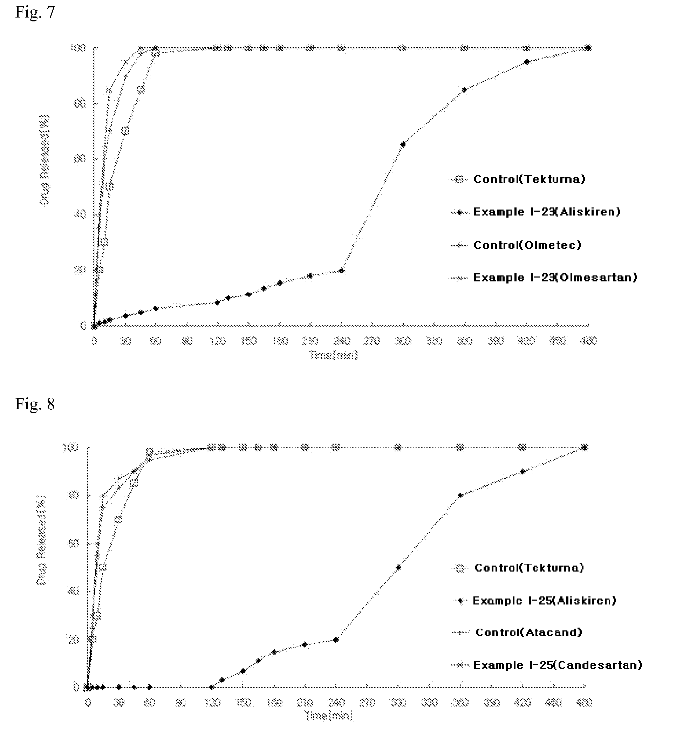

A comparative dissolution profile test was performed using aliskiren/olmesartan medoxomil capsules (pellets-tablets) prepared in Example I-23 and control drugs (Olmetec, Daiichi Sankyo Co., Ltd.: olmesartan medoxomil single drug, Tektuma: aliskiren single drug). The dissolution profile test of the olmesartan ingredient was performed based on the general dissolution test method described in the Korean Pharmacopoeia (8^{th} revision), and the dissolution profile test of the aliskiren ingredient was performed for a total of 480 minutes, in which the dissolution medium was changed from a simulated gastric juice to a simulated intestinal juice 120 minutes after the start of the test. Details of the dissolution profile test of the olmesartan ingredient are as follows. The results obtained are shown in FIG. 7. In FIG. 7, the x-axis represents the time series (min), and the y-axis represents the dissolution rate (Drug Released, %).

### Details of the dissolution profile test of the aliskiren ingredient are the same as those of Experimental Example I-1.

As can be seen in FIG. 7, when the dissolution profile test was performed under the following conditions, the olmesartan ingredient of the capsule (pellet-tablet) of the present invention showed a dissolution profile substantially equal to that of the control drug Olmetec, but the aliskiren ingredient showed a very slow dissolution rate as compared to that of the control drug Tektuma. In the dissolution profile test results for the aliskiren ingredient, the dissolution rates of the aliskiren ingredient up to 120 minutes corresponding to the simulated gastric juice zone were all less than 10% in the aliskiren/olmesartan medoxomil capsule (pellet-tablet) of the present invention, but the control drugs showed about 100%. The dissolution rate of the aliskiren ingredient in the subsequent simulated intestinal juice zone was about 20% up to a total of 240 minutes in the aliskiren/olmesartan medoxomil capsule (pellet-tablet) of the present invention, which was far lower than that of the control drugs.

As described above, the initial release of aliskiren in the aliskiren/olmesartan medoxomil capsule (pellet-tablet) of the present invention is much slower than olmesartan, unlike dissolution profiles obtained when the aliskiren single drug and the olmesartan medoxomil single drug, as the control drugs, are administered simultaneously. Thus, in the case of the inventive capsules, aliskiren is absorbed after olmesartan is absorbed first, thereby exhibiting no interaction therebetween.

### Test Method for olmesartan medoxomil: Based on the general dissolution test method described in the Korean Pharmacopoeia (8^{th} revision)

Test method: Paddle method, 50 rpm
Dissolution medium: pH 6.8 phosphate buffer, 900 mL
Analysis method: UV-Vis spectrophotometry

### Experimental Example I-8: Comparative dissolution profile test

A comparative dissolution profile test was performed using aliskiren/candesartan cilexetil capsules (granules-granules) prepared in Example I-25 and control drugs (Atacand, AstraZeneca: candesartan cilexetil single drug, Tekturna: aliskiren single drug). The dissolution profile test of the candesartan ingredient was performed based on the general dissolution test method described in the Korean Pharmacopoeia (8^{th} revision), and the dissolution profile test of the aliskiren ingredient was performed for a total of 480 minutes, in which the dissolution medium was changed from a simulated gastric juice to a simulated intestinal juice 120 minutes after the start of the test. Details of the dissolution profile test of the candesartan ingredient are as follows. The results obtained are shown in FIG. 8. In FIG. 8, the x-axis represents the time series (min), and the y-axis represents the dissolution rate (Drug Released, %).

### Details of the dissolution profile test of the aliskiren ingredient are the same as those of Experimental Example I-1.

As can be seen in FIG. 8, when the dissolution profile test was performed under the following conditions, the olmesartan ingredient of the capsule (granule-granule) of the present invention showed a dissolution profile substantially equal to that of the control drug Olmetec, but the aliskiren ingredient showed a very slow dissolution rate as compared to that of the control drug Tektuma. In the dissolution profile test results for the aliskiren ingredient, the dissolution rate of the aliskiren ingredient up to 120 minutes corresponding to the simulated gastric juice zone was less than 10% in the aliskiren/candesartan cilexetil capsule (granule-granule) of the present invention, but the control drugs showed about 100%. The dissolution rate of the aliskiren ingredient in the subsequent simulated intestinal juice zone was about 20% up to a total of 240 minutes in the aliskiren/candesartan cilexetil capsule (granule-granule) of the present invention, which was far lower than that of the control drugs.

As described above, the initial release of aliskiren in the aliskiren/candesartan cilexetil capsule (granule-granule) of the present invention is much slower than candesartan, unlike dissolution profiles obtained when the aliskiren single drug and the candesartan cilexetil single drug, as the control drugs, are administered simultaneously. Thus, in the case of the inventive capsules, aliskiren is absorbed after candesartan is absorbed first, thereby exhibiting no interaction therebetween.

### Test Method for candesartan: Based on the general dissolution test method described in the Korean Pharmacopoeia (8^{th} revision)

Test method: Paddle method, 50 rpm
Dissolution medium: pH 6.8 phosphate buffer, 900 mL
Analysis method: UV-Vis spectrophotometry

### <Example II, Experimental Example II>

### Example II-1: Preparation of aliskiren-irbesartan two-phase matrix tablets

### 1) Preparation of aliskiren-containing prior-release granules

According to the ingredient compositions and contents shown in Table 4 below, aliskiren hemi-fumarate, and microcrystalline cellulose, lactose, corn starch and sodium starch glycolate (as excipients) were sieved through a No. 35 sieve and mixed in a high-speed mixer. Meanwhile, hydroxypropylcellulose was dissolved in water to prepare a binding solution (10 w/w%). The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve to prepare prior-release granules.

### 2) Preparation of irbesartan-containing delayed-release granules

According to the ingredient compositions and contents shown in Table 4 below, irbesartan (Irbesartan USP, Ranbaxy) and microcrystalline cellulose were sieved through a No. 35 sieve and mixed in a high-speed mixer (Lab. Pharma Mixer P, Diosna, Germany) for 5 minutes to prepare a mixture. The mixture was placed in a fluidized bed coater (GPCG-1, Glatt, Germany). Meanwhile, hydroxypropylmethylcellulose was dissolved in water to prepare a binding solution (10 w/w%) which was then sprayed thereon to form granules, followed by drying. Then, a solution (5% w/w) of Eudragit RS PO in a 1:1 mixture of ethanol and methylene chloride was sprayed and coated on the granules.

### 3) Compression and coating

According the contents given in Table 4, the irbesartan-containing delayed-release granules and the aliskiren hemi-fumarate-containing prior-release granules prepared as above were placed and mixed in a double cone mixer. To the mixture was added sodium starch glycolate, followed by mixing, and magnesium stearate was added thereto, followed by final mixing. The final mixture was compressed into tablets using a rotary tablet press (MRC-33, Sejong Pharmatech Co., Ltd., South Korea). Meanwhile, hydroxypropylmethylcellulose 2910, hydroxypropylcellulose, titanium oxide, and talc were dissolved and dispersed in a 7:3 mixture of ethanol and purified water to prepare a coating solution (20% w/w). The compressed tablets were coated with the coating solution in a Hi-coater (SFC-30N, Sejong Pharmatech Co., Ltd., South Korea) to form a film-coated layer, thereby preparing two-phase matrix tablets.

### Example II-2: Preparation of aliskiren-irbesartan multi-layered tablets

### 1) Preparation of aliskiren-containing prior-release layer

According to the ingredient compositions and contents shown in Table 4 below, aliskiren hemi-fumarate, and microcrystalline cellulose and D-mannitol (as excipients) were sieved through a No. 35 sieve and mixed in a high-speed mixer. Meanwhile, hydroxypropylcellulose was dissolved in water to prepare a binding solution (10 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was mixed into the sieved material, and magnesium stearate was added thereto, followed by final mixing in a double cone mixer.

### 2) Preparation of irbesartan-containing delayed-release layer

According to the ingredient compositions and contents shown in Table 4 below, irbesartan and microcrystalline cellulose were sieved through a No. 35 sieve and mixed in a high-speed mixer for 5 minutes to prepare a mixture. The mixture was placed in a fluidized bed coater. Meanwhile, hydroxypropylmethylcellulose was dissolved in water to prepare a binding solution (10 w/w%) which was then sprayed thereon to form granules, followed by drying. Thereafter, a solution (5% w/w) of hydroxypropylmethylcellulose phthalate in a 1:1 mixture of ethanol and methylene chloride was sprayed and coated on the granules. Magnesium stearate was added thereto, followed by final mixing in a double cone mixer (Dasan Pharmatech, South Korea).

### 3) Compression and coating

Tablet compression was carried out using a multi-layered tablet press (MRC-37T, Sejong Pharmatech Co., Ltd., South Korea). The irbesartan-containing delayed-release layer composition was placed in a first powder feeder, and the aliskiren hemi-fumarate prior-release layer composition was placed in a second powder feeder. The layer compositions in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in a 7:3 mixture of ethanol and purified water to prepare a coating solution (20% w/w). The compressed tablets were coated with the coating solution in a Hi-coater (SFC-30N, Sejong Pharmatech Co., Ltd., South Korea) to form a film-coated layer, thereby preparing a formulation in the form of a multi-layered tablet.

### Example II-3: Preparation of aliskiren-irbesartan capsules (granules-pellets)

### 1) Preparation of aliskiren-containing prior-release granules

According to the contents shown in Table 4 below, aliskiren hemi-fumarate, microcrystalline cellulose and D-mannitol were sieved through a No. 35 sieve and mixed in a high-speed mixer. Meanwhile, hydroxypropylcellulose was dissolved in water to prepare a binding solution (10 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 2) Preparation of irbesartan-containing delayed-release pellets

According to the ingredient compositions and contents shown in Table 4 below, a mixture of irbesartan, microcrystalline cellulose and hydroxypropylmethylcellulose was dissolved in a 1:1 mixture of ethanol and methylene chloride to prepare a drug layer coating solution (20 w/w%). Sugar spheres were placed in a fluidized bed coater, and the drug coating solution was coated thereon. Thereafter, a solution (5% w/w) of hydroxypropylmethylcellulose phthalate in a 1:1 mixture of ethanol and methylene chloride was further coated thereon to prepare irbesartan pellets.

### 3) Mixing and capsule filling

The granules prepared in Process 1) and the pellets prepared in Process 2) were mixed in a double cone mixer. To the mixture was added sodium starch glycolate, followed by mixing in a double cone mixer. To the mixture was added magnesium stearate, followed by final mixing. The final mixture was placed in a powder feeder and then filled in capsules using a capsule filling machine (SF-40N, Sejong Pharmatech Co., Ltd., South Korea), thereby preparing a formulation in the form of a capsule.

### Example II-4: Preparation of aliskiren-irbesartan capsules (tablets-pellets)

### 1) Preparation of aliskiren-containing prior-release tablets

According to the ingredient compositions and contents shown in Table 4 below, aliskiren hemi-fumarate, microcrystalline cellulose, and D-mannitol were sieved through a No. 35 sieve and mixed in a high-speed mixer. Meanwhile, hydroxypropylcellulose was dissolved in water to prepare a binding solution (10 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was added to the sieved material, followed by mixing in a double cone mixer. To the mixture was added magnesium stearate, followed by final mixing. The final mixture was compressed into tablets using a rotary tablet press.

### 2) Preparation of irbesartan delayed-release pellets

According to the contents shown in Table 4 below, a mixture of irbesartan, microcrystalline cellulose, and hydroxypropylmethylcellulose was dissolved in a 1:1 mixture of ethanol and methylene chloride to prepare a drug coating solution (20 w/w%). Sugar spheres were placed in a fluidized bed coater, and the drug coating solution was coated thereon. Thereafter, a solution (5% w/w) of hydroxypropylmethylcellulose phthalate was further coated thereon to prepare irbesartan pellets.

### 3) Capsule filling

The tablets prepared in Process 1) and the pellets prepared in Process 2) were filled in capsules using a capsule filling machine (SF-40N, Sejong Pharmatech Co., Ltd., South Korea), thereby preparing a formulation in the form of a capsule.

### Example II-5: Preparation of aliskiren-irbesartan capsules (granules-granules)

### 1) Preparation of aliskiren-containing prior-release granules

According to the ingredient compositions and contents shown in Table 4 below, aliskiren hemi-fumarate, microcrystalline cellulose, and D-mannitol were sieved through a No. 35 sieve and mixed in a high-speed mixer. Meanwhile, hydroxypropylcellulose was dissolved in water to prepare a binding solution (10 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 2) Preparation of irbesartan-containing delayed-release granules

According to the ingredient compositions and contents shown in Table 4 below, irbesartan and microcrystalline cellulose were sieved through a No. 35 sieve and mixed in a high-speed mixer for 5 minutes to prepare a mixture. The mixture was placed in a fluidized bed coater. Meanwhile, hydroxypropylmethylcellulose was dissolved in water to prepare a binding solution (10 w/w%) which was then sprayed thereon to form granules, followed by drying. A solution (5% w/w) of hydroxypropylmethylcellulose phthalate in a 1:1 mixture of ethanol and methylene chloride was then sprayed and coated on the granules.

### 3) Mixing and capsule filling

The final compositions of Processes 1) and 2) were mixed in a double cone mixer. Sodium starch glycolate was added to the mixture, followed by mixing in a double cone mixer. To the mixture was added magnesium stearate, followed by final mixing. The final mixture was placed in a powder feeder and then filled in capsules using a capsule filling machine, thereby preparing a formulation in the form of a capsule.

### Example II-6: Preparation of aliskiren-irbesartan capsules (granules-tablets)

### 1) Preparation of aliskiren prior-release granules

According to the ingredient compositions and contents shown in Table 4 below, aliskiren hemi-fumarate, microcrystalline cellulose, and D-mannitol were sieved through a No. 35 sieve and mixed in a high-speed mixer. Meanwhile, hydroxypropylcellulose was dissolved in water to prepare a binding solution (10 w/w%). The binding solution and the mixture of-main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 2) Preparation of irbesartan delayed-release coated tablets

According to the ingredient compositions and contents shown in Table 4 below, irbesartan, microcrystalline cellulose, and sodium starch glycolate were sieved through a No. 35 sieve and mixed in a high-speed mixer for 5 minutes to prepare a mixture. The mixture was placed in a fluidized bed granulator. Meanwhile, hydroxypropylmethylcellulose was dissolved in water to prepare a binding solution which was then sprayed thereon to form granules, followed by drying. Then, powdered Carbomer 71G was added to the granules, and magnesium stearate was added thereto, followed by final mixing in a double cone mixer.

The final mixture was compressed into tablets having a hardness of 7 to 9kp, a thickness of 3.0mm, and a diameter of 5.5mm, using a rotary tablet press at 30 rpm. A solution (5% w/w) of hydroxypropylmethylcellulose phthalate in a 1:1 mixture of ethanol and methylene chloride was then coated thereon to prepare irbesartan delayed-release coated tablets.

### 3) Capsule filling

The granules prepared in Process 1) and the coated tablets prepared in Process 2) were filled in capsules using a capsule filling machine (SF-40N, Sejong Pharmatech Co., Ltd., South Korea), thereby preparing a formulation in the form of a capsule.

### Example II-7: Aliskiren-irbesartan blister package kits

### 1) Preparation of aliskiren-containing prior-release tablets

According to the ingredient compositions and contents shown in Table 4 below, aliskiren hemi-fumarate, and microcrystalline cellulose and D-mannitol (as excipients) were sieved through a No. 35 sieve and mixed in a high-speed mixer. Meanwhile, hydroxypropylcellulose was dissolved in water to prepare a binding solution (10 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was mixed into the sieved material, and magnesium stearate was added thereto, followed by final mixing in a double cone mixer.

The final mixture was compressed into tablets having a hardness of 7 to 9kp, a thickness of 3.0mm, and a diameter of 5.5mm, using a rotary tablet press at 30 rpm, thereby preparing prior-release tablets.

### 2) Preparation of irbesartan-containing delayed-release tablets

According to the ingredient compositions and contents shown in Table 4 below, irbesartan and microcrystalline cellulose were sieved through a No. 35 sieve and mixed in a high-speed mixer for 5 minutes to prepare a mixture. The mixture was placed in a fluidized bed coater. Meanwhile, hydroxypropylmethylcellulose was dissolved in water to prepare a binding solution which was then sprayed thereon to form granules. Then, a binding solution, which was prepared by heating and dissolving glyceryl behenate and glyceryl distearate at 70□, was further sprayed and coated on the granules. Thereafter, magnesium stearate was added to the granules, followed by final mixing in a double cone mixer (Dasan Pharmatech, South Korea).

The final mixture was compressed into tablets having a hardness of 7 to 9kp, a thickness of 3.0mm, and a diameter of 5.5mm, using a rotary tablet press at 30 rpm, thereby preparing delayed-release tablets.

### 3) Blister package

The tablets prepared in Processes 1) and 2) were packed in a blister package container (silver foil, Dong-il Corporation, PVDC, Jeon Min Industry Co., Ltd., South Korea) such that they can be simultaneously administered, using a blister package machine (Minister A, Heung-A Engineering, South Korea).

### Example II-8: Preparation of irbesartan-aliskiren two-phase matrix tablets

### 1) Preparation of irbesartan-containing prior-release granules

According to the ingredient contents and compositions shown in Table 5 below, irbesartan (as an active ingredient), and microcrystalline cellulose, lactose, corn starch and sodium starch glycolate (as excipients) were sieved through a No. 35 sieve and mixed in a high-speed mixer. Meanwhile, hydroxypropylcellulose was dissolved in water to prepare a binding solution (10 w/w%). The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve to prepare prior-release granules.

### 2) Preparation of aliskiren-containing delayed-release granules

According to the ingredient contents and compositions shown in Table 5 below, aliskiren (as an active ingredient) and microcrystalline cellulose were sieved through a No. 35 sieve and mixed in a high-speed mixer (Lab. Pharma Mixer P, Diosna, Germany) for 5 minutes to prepare a mixture. The mixture was placed in a fluidized bed coater (GPCG-1, Glatt, Germany). Meanwhile, hydroxypropylmethylcellulose was dissolved in water to prepare a binding solution which was then sprayed thereon to form granules, followed by drying. A solution (5% w/w) of Eudragit RS PO in a 1:1 mixture of ethanol and methylene chloride was sprayed and coated on the granules.

### 3) Compression and coating

The thus prepared delayed-release granules and prior-release granules were placed and mixed in a double cone mixer. Sodium starch glycolate was added to the mixture, followed by mixing, and magnesium stearate was added thereto, followed by final mixing. The final mixture was compressed into tablets using a rotary tablet press (MRC-33, Sejong Pharmatech Co., Ltd., South Korea). Meanwhile, hydroxypropylmethylcellulose 2910, hydroxypropylcellulose, titanium oxide, and talc were dissolved and dispersed in a 7:3 mixture of ethanol and purified water to prepare a coating solution (20% w/w). The compressed tablets were coated with the coating solution in a Hi-coater (SFC-30N, Sejong Pharmatech Co., Ltd., South Korea) to form a film-coated layer, thereby preparing two-phase matrix tablets.

### Example II-9: Preparation of irbesartan-aliskiren multi-layered tablets

### 1) Preparation of irbesartan-containing prior-release layer

According to the ingredient contents and compositions shown in Table 5 below, irbesartan (as an active ingredient), and microcrystalline cellulose and D-mannitol (as excipients) were sieved through a No. 35 sieve and mixed in a high-speed mixer. Meanwhile, hydroxypropylcellulose was dissolved in water to prepare a binding solution (10 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was mixed into the sieved material, and magnesium stearate was added thereto, followed by final mixing in a double cone mixer.

### 2) Preparation of aliskiren-containing delayed-release layer

According to the ingredient contents and compositions shown in Table 5 below, aliskiren hemi-fumarate (as an active ingredient) and microcrystalline cellulose were sieved through a No. 35 sieve and mixed in a high-speed mixer for 5 minutes to prepare a mixture. The mixture was placed in a fluidized bed coater. Meanwhile, hydroxypropylmethylcellulose was dissolved in water to prepare a binding solution which was then sprayed thereon to form granules, followed by drying. A solution (5% w/w) of hydroxypropylmethylcellulose phthalate in a 1:1 mixture of ethanol and methylene chloride was then sprayed and coated on the granules. Magnesium stearate was added thereto, followed by final mixing in a double cone mixer (Dasan Pharmatech, South Korea).

### 3) Compression and coating

The irbesartan-containing composition was placed in a first powder feeder, and the aliskiren delayed-release layer composition was placed in a second powder feeder. The layer compositions in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in a 7:3 mixture of ethanol and purified water to prepare a coating solution (20% w/w). The compressed tablets were coated with the coating solution in a Hi-coater (SFC-30N, Sejong Pharmatech Co., Ltd., South Korea) to form a film-coated layer, thereby preparing a formulation in the form of a multi-layered tablet.

### Example II-10: Preparation of irbesartan-aliskiren capsules (granules-pellets)

### 1) Preparation of irbesartan-containing prior-release granules

According to the ingredient contents and compositions shown in Table 5 below, irbesartan (as an active ingredient), microcrystalline cellulose, and D-mannitol were sieved through a No. 35 sieve and mixed in a high-speed mixer. Meanwhile, hydroxypropylcellulose was dissolved in water to prepare a binding solution (10 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 2) Preparation of aliskiren delayed-release pellets

According to the ingredient contents and compositions shown in Table 5 below, a mixture of aliskiren hemi-fumarate (as an active ingredient), microcrystalline cellulose and hydroxypropylmethylcellulose was dissolved in a 1:1 mixture of ethanol and methylene chloride to prepare a drug layer coating solution (20 w/w%). Sugar spheres were placed in a fluidized bed coater, and the drug coating solution was coated thereon. Thereafter, a solution (5% w/w) of hydroxypropylmethylcellulose phthalate in a 1:1 mixture of ethanol and methylene chloride was further coated thereon to prepare irbesartan pellets.

### 3) Mixing and capsule filling

The granules of Process 1) and the pellets of Process 2) thus prepared were mixed in a double cone mixer. To the mixture was added sodium starch glycolate, followed by mixing in a double cone mixer. To the mixture was added magnesium stearate, followed by final mixing. The final mixture was placed in a powder feeder and then filled in capsules using a capsule filling machine (SF-40N, Sejong Pharmatech Co., Ltd., South Korea), thereby preparing a formulation in the form of a capsule.

### Example II-11: Preparation of irbesartan-aliskiren capsules (tablets-pellets)

### 1) Preparation of irbesartan prior-release tablets

According to the ingredient contents and compositions shown in Table 5 below, irbesartan (as an active ingredient), microcrystalline cellulose, and D-mannitol were sieved through a No. 35 sieve and mixed in a high-speed mixer. Meanwhile, hydroxypropylcellulose was dissolved in water to prepare a binding solution (10 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was added to the sieved material, followed by mixing in a double cone mixer. To the mixture was added magnesium stearate, followed by final mixing. The final mixture was compressed into tablets using a rotary tablet press.

### 2) Preparation of aliskiren delayed-release pellets

According to the ingredient contents and compositions shown in Table 5 below, a mixture of aliskiren hemi-fumarate (as an active ingredient), microcrystalline cellulose and hydroxypropylmethylcellulose was dissolved in a 1:1 mixture of ethanol and methylene chloride to prepare a drug layer coating solution. Sugar spheres were placed in a fluidized bed coater, and the drug coating solution was coated thereon. Thereafter, a solution (5% w/w) of hydroxypropylmethylcellulose phthalate in a 1:1 mixture of ethanol and methylene chloride was further coated thereon to prepare irbesartan pellets.

### 3) Capsule filling

The tablets prepared in Process 1) and the pellets prepared in Process 2) were filled in capsules using a capsule filling machine, thereby preparing a controlled-release formulation in the form of a capsule.

### Example II-12: Preparation of irbesartan-aliskiren capsules (granules-granules)

### 1) Preparation of irbesartan prior-release granules

According to the ingredient contents and compositions shown in Table 5 below, irbesartan (as an active ingredient), microcrystalline cellulose, and D-mannitol were sieved through a No. 35 sieve and mixed in a high-speed mixer. Meanwhile, hydroxypropylcellulose was dissolved in water to prepare a binding solution (10 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 2) Preparation of aliskiren delayed-release granules

According to the ingredient contents and compositions shown in Table 5 below, aliskiren hemi-fumarate (as an active ingredient) and microcrystalline cellulose were sieved through a No. 35 sieve and mixed in a high-speed mixer for 5 minutes to prepare a mixture. The mixture was placed in a fluidized bed coater. Meanwhile, hydroxypropylmethylcellulose was dissolved in water to prepare a binding solution (10 w/w%) which was then sprayed thereon to form granules, followed by drying. Then, a solution (5% w/w) of hydroxypropylmethylcellulose phthalate in a 1:1 mixture of ethanol and methylene chloride was sprayed and coated on the granules.

### 3) Mixing and capsule filling

The granules prepared in Process 1) and the granules prepared in 2) were mixed in a double cone mixer. To the mixture was added sodium starch glycolate, followed by mixing in a double cone mixer. To the mixture was added magnesium stearate, followed by final mixing. The final mixture was placed in a powder feeder and then filled in capsules using a capsule filling machine, thereby preparing a formulation in the form of a capsule.

### Example II-13: Preparation of irbesartan-aliskiren capsules (granules-tablets)

### 1) Preparation of irbesartan prior-release granules

According to the ingredient contents and compositions shown in Table 5 below, irbesartan (as an active ingredient), microcrystalline cellulose, and D-mannitol were sieved through a No. 35 sieve and mixed in a high-speed mixer. Meanwhile, hydroxypropylcellulose was dissolved in water to prepare a binding solution (10 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 2) Preparation of aliskiren delayed-release coated tablets

According to the ingredient contents and compositions shown in Table 5 below, aliskiren hemi-fumarate (as an active ingredient), microcrystalline cellulose, and sodium starch glycolate were sieved through a No. 35 sieve and mixed in a high-speed mixer for 5 minutes to prepare a mixture. The mixture was placed in a fluidized bed granulator. Meanwhile, hydroxypropylmethylcellulose was dissolved in water to prepare a binding solution (10 w/w%) which was then sprayed thereon to form granules, followed by drying. Then, powdered Carbomer 71 G was added to the granules, and magnesium stearate was added thereto, followed by final mixing in a double cone mixer.

The final mixture was compressed into tablets having a hardness of 7 to 9kp, a thickness of 3.0mm, and a diameter of 5.5mm, using a rotary tablet press at 30 rpm. A solution (5% w/w) of hydroxypropylmethylcellulose phthalate in a 1:1 mixture of ethanol and methylene chloride was then coated thereon to prepare delayed-release coated tablets.

### 3) Capsule filling

The granules prepared in Process 1) and the tablets prepared in Process 2) were filled in capsules using a capsule filling machine, thereby preparing a controlled-release formulation in the form of a capsule.

### Example II-14: Preparation of irbesartan-aliskiren osmotic press-coated tablets

### 1) Preparation of irbesartan prior-release layer

According to the ingredient contents and compositions shown in Table 5 below, irbesartan (as an active ingredient), and microcrystalline cellulose, lactose, corn starch and sodium starch glycolate (as excipients) were sieved through a No. 35 sieve and mixed in a high-speed mixer. Meanwhile, hydroxypropylcellulose was dissolved in water to prepare a binding solution (10 w/w%). The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve, and magnesium stearate was added thereto, followed by final mixing in a double cone mixer to prepare a prior-release layer.

### 2) Preparation of aliskiren delayed-release osmotic coated tablets

According to the ingredient contents and compositions shown in Table 5 below, aliskiren hemi-fumarate (as an active ingredient), microcrystalline cellulose and sodium chloride were sieved through a No. 35 sieve, followed by mixing in a double cone mixer, and magnesium stearate was added thereto, followed by final mixing. The final mixture was compressed into tablets using a rotary tablet press (MRC-30: Sejong, South Korea). The compressed inner core tablets were coated with a dispersion (20% w/w) of Kollicoat SR 30D as a semi-permeable coating base and triethyl citrate as a film-forming aid in purified water in a Hi-coater (SFC-30N, Sejong Machinery Co., Ltd., South Korea), thereby preparing an osmotic inner core.

### 3) Compression and coating

The thus prepared irbesartan-containing prior-release layer composition was placed in a powder feeder, and the aliskiren delayed-release tablets were placed in an inner core feeder, followed by compression in a press-coated tablet press (Kilian RUD-1, Germany). Meanwhile, hydroxypropylmethylcellulose 2910, hydroxypropylcellulose, titanium oxide, and talc were dissolved and dispersed in a 7:3 mixture of ethanol and purified water to prepare a coating solution (20% w/w). The compressed tablets were coated with the coating solution in a Hi-coater (SFC-30N, Sejong Pharmatech Co., Ltd., South Korea) to form a film-coated layer, thereby preparing a formulation in the form of a press-coated tablet.

**Table 4**

| Ingredients | | Content/unit formulation (mg) | | | | | | |
|---|---|---|---|---|---|---|---|---|
| | | Example No. | | | | | | |
| | | II-1 | II-2 | II-3 | II-4 | II-5 | II-6 | II-7 |
| Prior-release compartment | Aliskiren hemi-fumarate | 167.8 | 167.8 | 167.8 | 167.8 | 167.8 | 167.8 | 167.8 |
| | Microcrystalline cellulose | 57 | 57 | 57 | 57 | 57 | 57 | 57 |
| | D-mannitol | 0 | 112.5 | 112.5 | 112.5 | 112.5 | 112.5 | 114 |
| | Lactose | 82.5 | 0 | 0 | 0 | 0 | 0 | 0 |
| | Corn starch | 25 | 0 | 0 | 0 | 0 | 0 | 0 |
| | Sodium starch glycolate | 2 | 2 | 0 | 2 | 0 | 0 | 2 |
| | Hydroxypropylcellulose | 5 | 5 | 5 | 5 | 5 | 5 | 5 |
| | Magnesium stearate | 0 | 1.5 | 0 | 1.5 | 0 | 0 | 1.5 |
| Delayed-release compartment | Irbesartan | 150 | 150 | 150 | 150 | 150 | 150 | 150 |
| | Sugar sphere | 0 | 0 | 70 | 70 | 0 | 0 | 0 |
| | Microcrystalline cellulose | 70.2 | 74.5 | 10.2 | 10.2 | 75.2 | 70.2 | 52.2 |
| | Eudragit RS PO¹⁾ | 20 | 0 | 0 | 0 | 0 | 0 | 0 |
| | Carbomer 71G²⁾ | 0 | 0 | 0 | 0 | 0 | 10 | 0 |
| | Glyceryl behenate³⁾ | 0 | 0 | 0 | 0 | 0 | 0 | 12 |
| | Glyceryl distearate⁴⁾ | 0 | 0 | 0 | 0 | 0 | 0 | 30 |
| | Hydroxypropylmethylcellulose | 2 | 7 | 2 | 2 | 7 | 2 | 7 |
| | Sodium starch glycolate | 0 | 0 | 0 | 0 | 0 | 2 | 0 |
| | Hydroxypropylmethylcellulose phthalate | 0 | 10 | 10 | 10 | 10 | 10 | 0 |
| | Magnesium stearate | 0 | 0.7 | 0 | 0 | 0 | 1.5 | 1.5 |
| Post-mixing | Sodium starch glycolate | 5 | 0 | 2 | 0 | 2 | 0 | 0 |
| | Magnesium stearate | 1.5 | 0 | 1.5 | 0 | 1.5 | 0 | 0 |
| Coating layer | Hydroxypropylmethylcellulose 2910⁵⁾ | 7 | 7 | 0 | 0 | 0 | 0 | 0 |
| | Hydroxypropylcellulose | 7 | 7 | 0 | 0 | 0 | 0 | 0 |
| | Titanium oxide | 6 | 6 | 0 | 0 | 0 | 0 | 0 |
| | Talc | 4 | 4 | 0 | 0 | 0 | 0 | 0 |
| Total | | 612 | 612 | 588 | 588 | 588 | 588 | 600 |
| 1) Eudragit RS PO: polymethacrylate copolymer, Evonik-Degussa | | | | | | | | |
| 2) Carbomer 71 G: carboxyvinyl polymer, Noveon | | | | | | | | |
| 3) Glyceryl behenate: COMPRITOL 888 ATO, GATTEFOSSE | | | | | | | | |
| 4) Glyceryl distearate: PRECIROL ATO 5, GATTEFOSSE | | | | | | | | |
| 5) Hydroxypropylmethylcellulose 2910: Pharmacoat 606, Shin-Etsu | | | | | | | | |

**Table 5**

| Ingredients | | Content/unit formulation (mg) | | | | | | |
|---|---|---|---|---|---|---|---|---|
| | | Example No. | | | | | | |
| | | II-8 | II-9 | II-10 | II-11 | II-12 | II-13 | II-14 |
| Prior-release compartment | Irbesartan | 150 | 150 | 150 | 150 | 150 | 150 | 150 |
| | Microcrystalline cellulose | 57 | 57 | 57 | 57 | 57 | 57 | 57 |
| | D-mannitol | 0 | 112.5 | 112.5 | 112.5 | 112.5 | 112.5 | 0 |
| | Lactose | 82.5 | 0 | 0 | 0 | 0 | 0 | 82.5 |
| | Corn starch | 25 | 0 | 0 | 0 | 0 | 0 | 20 |
| | Sodium starch glycolate | 2 | 2 | 0 | 2 | 0 | 0 | 2 |
| | Hydroxypropylcellulose | 5 | 5 | 5 | 5 | 5 | 5 | 5 |
| | Magnesium stearate | 0 | 1.5 | 0 | 1.5 | 0 | 0 | 1.5 |
| Delayed-release compartment | Aliskiren hemi-fumarate | 167.8 | 167.8 | 167.8 | 167.8 | 167.8 | 167.8 | 167.8 |
| | Sugar sphere | 0 | 0 | 70 | 70 | 0 | 0 | 0 |
| | Microcrystalline cellulose | 70.2 | 74.5 | 10.2 | 10.2 | 75.2 | 70.2 | 70.2 |
| | Sodium chloride | 0 | 0 | 0 | 0 | 0 | 0 | 12 |
| | Eudragit RS PO¹⁾ | 20 | 0 | 0 | 0 | 0 | 0 | 0 |
| | Carbomer 71G²⁾ | 0 | 0 | 0 | 0 | 0 | 10 | 0 |
| | Kollicoat SR 30D³⁾ | 0 | 0 | 0 | 0 | 0 | 0 | 17 |
| | Hydroxypropylmethylcellulose | 2 | 7 | 2 | 2 | 7 | 2 | 0 |
| | Triethyl citrate | 0 | 0 | 0 | 0 | 0 | 0 | 2 |
| | Sodium starch glycolate | 0 | 0 | 0 | 0 | 0 | 2 | 0 |
| | Hydroxypropylmethylcellulose phthalate | 0 | 10 | 10 | 10 | 10 | 10 | 0 |
| | Magnesium stearate | 0 | 0.7 | 0 | 0 | 0 | 1.5 | 1 |
| Post-mixing | Sodium starch glycolate | 5 | 0 | 2 | 0 | 2 | 0 | 0 |
| | Magnesium stearate | 1.5 | 0 | 1.5 | 0 | 1.5 | 0 | 0 |
| Coating layer | Hydroxypropylmethylcellulose 2910⁴⁾ | 7 | 7 | 0 | 0 | 0 | 0 | 7 |
| | Hydroxypropylcellulose | 7 | 7 | 0 | 0 | 0 | 0 | 7 |
| | Titanium oxide | 6 | 6 | 0 | 0 | 0 | 0 | 6 |
| | Talc | 4 | 4 | 0 | 0 | 0 | 0 | 4 |
| Total | | 612 | 612 | 588 | 588 | 588 | 588 | 612 |
| 1) Eudragit RS PO: polymethacrylate copolymer, Evonik-Degussa | | | | | | | | |
| 2) Carbomer 71 G: carboxyvinyl polymer, Noveon | | | | | | | | |
| 3) Kollicoat SR 30D: polyvinyl acetate 30% suspension, BASF | | | | | | | | |
| 4) Hydroxypropylmethylcellulose 2910: Pharmacoat 606, Shin-Etsu | | | | | | | | |

### Experimental Example II-1: Comparative dissolution profile test

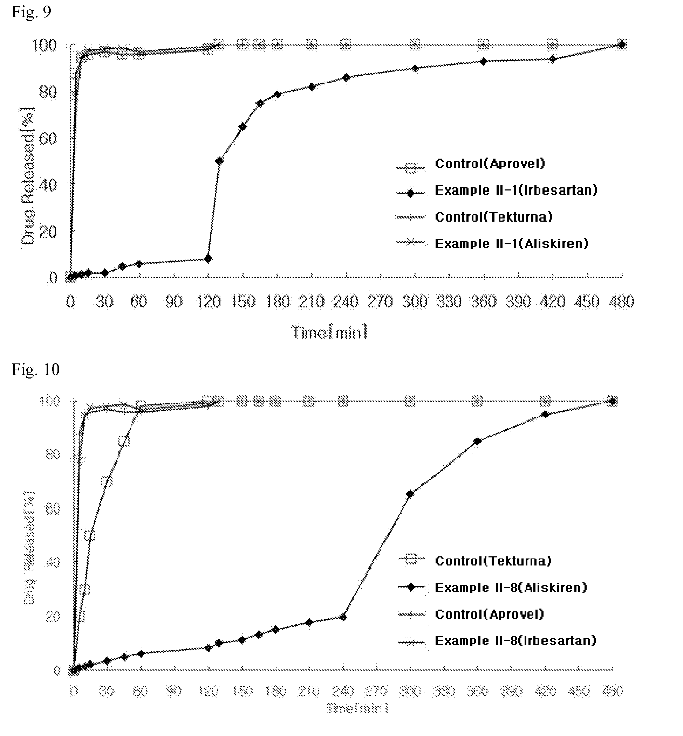

A comparative dissolution profile test was performed using the formulation prepared in Example II-1 and control drugs (Aprovel: Handok Pharmaceuticals, irbesartan single drug, Tektuma: Novartis, aliskiren single drug). The dissolution profile test of the aliskiren ingredient was performed based on the general dissolution test method described in the Korean Pharmacopoeia (8^{th} revision), and the dissolution profile test of the irbesartan ingredient was performed for a total of 480 minutes, in which the dissolution medium was changed from a simulated gastric juice to a simulated intestinal juice 120 minutes after the start of the test. Details of the dissolution profile test of each ingredient are as follows. The results obtained are shown in FIG. 9. In FIG. 9, the x-axis represents the time series (min), and the y-axis represents the dissolution rate (Drug Released, %).

As can be seen in FIG. 9, when the dissolution profile test was performed under the following conditions, the aliskiren ingredient of the formulation of the present invention showed a dissolution profile substantially equal to that of the control drug Tektuma, but the irbesartan ingredient showed a very slow dissolution rate as compared to that of the control drug Aprovel.

The dissolution rate of the irbesartan ingredient was less than 10% up to 2 hours in the formulation of the present invention, which was far lower than the dissolution rate of about 99% in the control drugs.

As described above, the initial release of irbesartan in the formulation of the present invention is much slower than aliskiren, unlike dissolution profiles obtained when the aliskiren single drug and the irbesartan single drug, as the control drugs, are administered simultaneously. Thus, in the case of the inventive formulation, the time for irbesartan to be absorbed after aliskiren is metabolized first in the liver can be sufficiently ensured.

### Test Method for irbesartan: Based on the general dissolution test method described in the Korean Pharmacopoeia (8^{th} revision)

Test method: Paddle method, 50 rpm
Dissolution medium: 0.01 M hydrochloric acid solution 750 mL (simulated gastric juice), pH 6.8 phosphate buffer 1,000 mL (simulated intestinal juice)
Analysis method: High performance liquid chromatography

### Test Method for aliskiren hemi-fumarate: Based on the general dissolution test method described in the Korean Pharmacopoeia (8^{th} revision)

Test method: Paddle method, 50 rpm
Dissolution medium: Purified water, 900 mL
Analysis method: High performance liquid chromatography

### Experimental Example II-2: Comparative dissolution profile test

A comparative dissolution profile test was performed using the formulation prepared in Example II-8 and control drugs (Aprovel: irbesartan single drug, Tekturna: aliskiren single drug). The dissolution profile test of the irbesartan ingredient was performed based on the "Irbesartan tablets" part in the United States Pharmacopoeia, and the dissolution profile test of the aliskiren ingredient was performed for a total of 480 minutes, in which the dissolution medium was changed from a simulated gastric juice to a simulated intestinal juice 120 minutes after the start of the test. Details of the dissolution profile test of each ingredient are as follows. The results obtained are shown in FIG. 10. In FIG. 10, the x-axis represents the time series (min), and the y-axis represents the dissolution rate (Drug Released, %).

As can be seen in FIG. 10, when the dissolution profile test was performed under the following conditions, the irbesartan ingredient of the formulation of the present invention showed a dissolution profile substantially equal to that of the control drug Aprovel, but the aliskiren ingredient showed a very slow dissolution rate as compared to that of the control drug Tekturna.

The dissolution rate of the aliskiren ingredient was less than 10% up to 2 hours in the aliskiren-irbesartan combination formulation of the present invention, which was far lower than the dissolution rate of about 99% in the control drugs.
Test Method for irbesartan: Based on the "Irbesartan tablets" part in USP 31
Test method: Paddle method, 50 rpm
Dissolution medium: 0.01 N hydrochloric acid solution, 900 mL
Analysis method: High performance liquid chromatography

### Test Method for aliskiren hemi-fumarate: Based on the general dissolution test method described in the Korean Pharmacopoeia (8^{th} revision)

Test method: Paddle method, 50 rpm
Dissolution medium: 0.01 N hydrochloric acid solution 750 mL (simulated gastric juice), pH 6.8 phosphate buffer 1,000 mL (simulated intestinal juice)
Analysis method: High performance liquid chromatography

### Experimental Example II-3: Comparative dissolution profile test

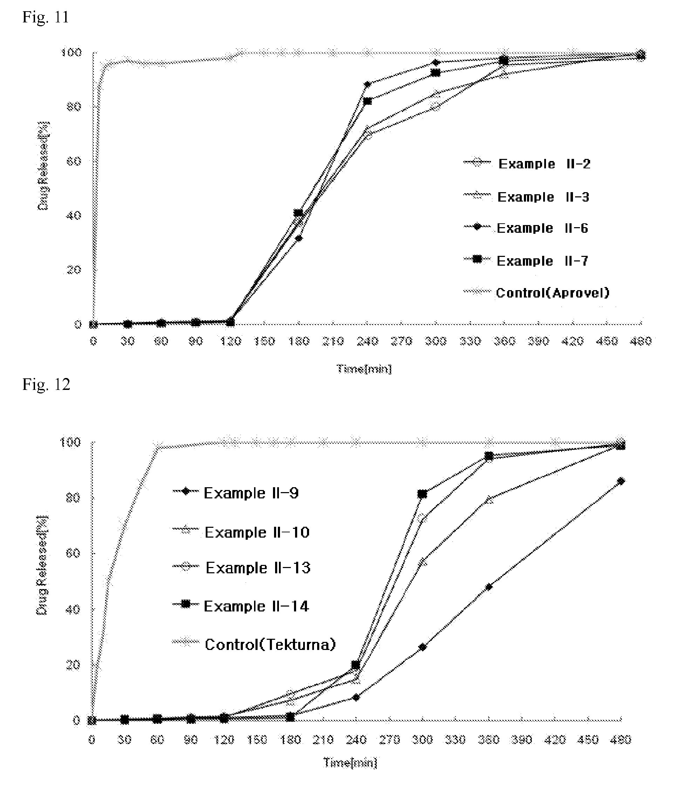

A comparative dissolution profile test was performed using the formulations prepared in Examples II-2, II-3, II-6 and II-7 and a control drug (Aprovel: irbesartan single drug). Details of the dissolution profile test are the same as those of Experimental Example II-1. The dissolution profile test results of the irbesartan ingredient are shown in FIG. 11. In FIG. 11, the x-axis represents the time series (min), and the y-axis represents the dissolution rate (Drug Released, %).

According to the test results, the dissolution rate of irbesartan which is delayed-released was less than 10% up to 2 hours in the formulation of the present invention, which was far lower than the dissolution rate of about 99% in the control drug.

### Test Method for irbesartan: Based on the general dissolution test method described in the Korean Pharmacopoeia (8^{th} revision)

Test method: Paddle method, 50 rpm
Dissolution medium: 0.01 M hydrochloric acid solution 750 mL (simulated gastric juice), pH 6.8 phosphate buffer 1,000 mL (simulated intestinal juice)
Analysis method: High performance liquid chromatography

### Experimental Example II-4: Comparative dissolution profile test

A comparative dissolution profile test was performed using the aliskiren-irbesartan formulations prepared in Examples II-9, II-10, II-13 and II-14 and a control drug (Tekturna: aliskiren single drug). Details of the dissolution profile test are as follows. The dissolution profile test results of the aliskiren ingredient are shown in FIG. 12. In FIG. 12, the x-axis represents the time series (min), and the y-axis represents the dissolution rate (Drug Released, %).

According to the dissolution profile test results, the dissolution rate of aliskiren which is delayed-released was less than 10% up to 2 hours in the formulation of the present invention, which was far lower than the dissolution rate of about 99% in the control drug.

### Test Method for aliskiren hemi-fumarate: Based on the general dissolution test method described in the Korean Pharmacopoeia (8^{th} revision)

Test method: Paddle method, 50 rpm
Dissolution medium: 0.01 N hydrochloric acid solution 750 mL (simulated gastric juice), pH 6.8 phosphate buffer 1,000 mL (simulated intestinal juice)
Analysis method: High performance liquid chromatography

### <Example III, Experimental Example III>

### Example III-1: Preparation of aliskiren-valsartan two-phase matrix tablets

### 1) Preparation of aliskiren-containing prior-release granules

According to the ingredient compositions and contents shown in Table 6 below, aliskiren hemi-fumarate, microcrystalline cellulose, lactose, corn starch, and sodium starch glycolate were sieved through a No. 35 sieve and mixed in a high-speed mixer (Lab. Pharma Mixer P, Diosna, Germany). Meanwhile, hydroxypropylcellulose was dissolved in water to prepare a binding solution (15 w/w%). The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve to prepare prior-release granule semi-finished products.

### 2) Preparation of valsartan-containing delayed-release granules

According to the ingredient compositions and contents shown in Table 6 below, valsartan (Dr. Reddy's, India) and microcrystalline cellulose were sieved through a No. 35 sieve and mixed in a high-speed mixer (Lab. Pharma Mixer P, Diosna, Germany) for 5 minutes to prepare a mixture. The mixture was placed in a fluidized bed coater (GPCG-1, Glatt, Germany). Meanwhile, hydroxypropylmethylcellulose was dissolved in water to prepare a binding solution (15 w/w%) which was then sprayed thereon to form granules, followed by drying. Thereafter, a solution (5% w/w) of Eudragit RS PO in a 1:1 mixture of ethanol and methylene chloride was sprayed and coated on the granules.

### 3) Compression and coating

The valsartan delayed-release granules and the aliskiren hemi-fumarate prior-release granules thus prepared were placed and mixed in a double cone mixer. Sodium starch glycolate was mixed into the mixture and magnesium stearate was added thereto, followed by final mixing. The final mixture was compressed into tablets using a rotary tablet press (MRC-33, Sejong Pharmatech Co., Ltd., South Korea). Meanwhile, hydroxypropylmethylcellulose 2910, hydroxypropylcellulose, titanium oxide, and talc were dissolved and dispersed in ethanol and purified water to prepare a coating solution (10 w/w%). The compressed tablets were coated with the coating solution in a Hi-coater (SFC-30N, Sejong Pharmatech Co., Ltd., South Korea) to form a film-coated layer, thereby preparing a two-phase matrix tablet.

### Example III-2: Preparation of aliskiren-valsartan multi-layered tablets

### 1) Preparation of aliskiren-containing Prior-release layer

According to the ingredient compositions and contents shown in Table 6 below, aliskiren hemi-fumarate, microcrystalline cellulose and D-mannitol were sieved through a No. 35 sieve and mixed in a high-speed mixer. Meanwhile, hydroxypropylcellulose was dissolved in water to prepare a binding solution (15 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was mixed into the sieved material, and magnesium stearate was added thereto, followed by final mixing in a double cone mixer.

### 2) Preparation of valsartan-containing delayed-release layer

According to the ingredient compositions and contents shown in Table 6 below, valsartan and microcrystalline cellulose were sieved through a No. 35 sieve and mixed in a high-speed mixer for 5 minutes to prepare a mixture. The mixture was placed in a fluidized bed coater. Meanwhile, hydroxypropylmethylcellulose was dissolved in water to prepare a binding solution (5 w/w%) which was then sprayed thereon to form granules, followed by drying. Thereafter, a solution (5% w/w) of hydroxypropylmethylcellulose phthalate in a 1:1 mixture of ethanol and methylene chloride was sprayed and coated on the granules. Magnesium stearate was added thereto, followed by final mixing in a double cone mixer (Dasan Pharmatech, South Korea).

### 3) Compression and coating

Tablet compression was carried out using a multi-layered tablet press (MRC-37T, Sejong Pharmatech Co., Ltd., South Korea). The valsartan-containing composition was placed in a first powder feeder, and the aliskiren hemi-fumarate delayed-release layer composition was placed in a second powder feeder. The layer compositions in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in ethanol and purified water to prepare a coating solution (10 w/w%). The compressed tablets were coated with the coating solution in a Hi-coater to form a film-coated layer, thereby preparing multi-layered tablets.

### Example III-3: Preparation of aliskiren-valsartan capsules (granules-pellets)

### 1) Preparation of aliskiren-containing prior-release granules

According to the ingredient contents shown in Table 6 below, aliskiren hemi-fumarate, microcrystalline cellulose, and D-mannitol were sieved through a No. 35 sieve and mixed in a high-speed mixer. Meanwhile, hydroxypropylcellulose was dissolved in water to prepare a binding solution (15 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 2) Preparation of valsartan-containing delayed-release Pellets

According to the ingredient compositions and contents shown in Table 6 below, a mixture of valsartan, microcrystalline cellulose and hydroxypropylmethylcellulose was dissolved in a 1:1 mixture of ethanol and methylene chloride to prepare a drug layer coating solution (10 w/w%). Sugar spheres were placed in a fluidized bed coater, and the drug coating solution was coated thereon. Thereafter, a solution (5% w/w) of hydroxypropylmethylcellulose phthalate was further coated thereon to prepare valsartan pellets.

### 3) Mixing and capsule filling

The granules prepared in Process 1) and the pellets prepared in Process 2) were mixed in a double cone mixer. To the mixture was added sodium starch glycolate, followed by mixing in a double cone mixer. To the mixture was added magnesium stearate, followed by final mixing. The final mixture was placed in a powder feeder and then filled in capsules using a capsule filling machine (SF-40N, Sejong Pharmatech Co., Ltd., South Korea), thereby preparing a formulation in the form of a capsule.

### Example III-4: Preparation of aliskiren-valsartan capsules (tablets-pellets)

### 1) Preparation of aliskiren-containing prior-release tablets

According to the ingredient compositions and contents shown in Table 6 below, aliskiren hemi-fumarate, microcrystalline cellulose, and D-mannitol were sieved through a No. 35 sieve and mixed in a high-speed mixer. Meanwhile, hydroxypropylcellulose was dissolved in water to prepare a binding solution (15 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was added to the sieved material, followed by mixing in a double cone mixer. To the mixture was added magnesium stearate, followed by final mixing. The final mixture was compressed into tablets using a rotary tablet press.

### 2) Preparation of valsartan-containing delayed-release pellets

According to the ingredient compositions and contents shown in Table 6 below, the preparation was carried out in the same manner as in Process 2) of Example III-3.

### 3) Capsule filling

The tablets prepared in Process 1) and the pellets prepared in Process 2) were filled in capsules using a capsule filling machine, thereby preparing a formulation in the form of a capsule.

### Example III-5: Preparation of aliskiren-valsartan capsules (granules-granules)

### 1) Preparation of aliskiren-containing prior-release granules

According to the ingredient compositions and contents shown in Table 6 below, the preparation was carried out in the same manner as in Process 1) of Example III-3.

### 2) Preparation of valsartan-containing delayed-release granules

According to the ingredient compositions and contents shown in Table 6 below, valsartan and microcrystalline cellulose were sieved through a No. 35 sieve and mixed in a high-speed mixer for 5 minutes to prepare a mixture. The mixture was placed in a fluidized bed coater. Meanwhile, hydroxypropylmethylcellulose was dissolved in water to prepare a binding solution (5% w/w) which was then sprayed thereon to form granules, followed by drying. A solution (5% w/w) of hydroxypropylmethylcellulose phthalate in a 1:1 mixture of ethanol and methylene chloride was then sprayed and coated on the granules.

### 3) Mixing and capsule filling

The granules prepared in Process 1) and the granules prepared in Process 2) were mixed in a double cone mixer. To the mixture was added sodium starch glycolate, followed by mixing in a double cone mixer. To the mixture was added magnesium stearate, followed by final mixing. The final mixture was placed in a powder feeder and then filled in capsules using a capsule filling machine, thereby preparing a formulation in the form of a capsule.

### Example III-6: Preparation of aliskiren-valsartan capsules (granules-tablets)

### 1) Preparation of aliskiren-containing prior-release granules

According to the ingredient compositions and contents shown in Table 6 below, the preparation was carried out in the same manner as in Process 1) of Example III-3.

### 2) Preparation of valsartan-containing delayed-release coated tablets

According to the ingredient compositions and contents shown in Table 6 below, valsartan, microcrystalline cellulose, and sodium starch glycolate were sieved through a No. 35 sieve and mixed in a high-speed mixer for 5 minutes to prepare a mixture. The mixture was placed in a fluidized bed granulator. Meanwhile, hydroxypropylmethylcellulose was dissolved in water to prepare a binding solution (15% w/w) which was then sprayed thereon to form granules, followed by drying. Then, powdered Carbomer 71G was added to the granules, and magnesium stearate was added thereto, followed by final mixing in a double cone mixer.

The final mixture was compressed into tablets having a hardness of 7 to 9kp, a thickness of 3.0mm, and a diameter of 5.5mm, using a rotary tablet press at 30 rpm. A solution (5% w/w) of hydroxypropylmethylcellulose phthalate in a 1:1 mixture of ethanol and methylene chloride was then coated thereon to prepare valsartan delayed-release coated tablets.

### 3) Capsule filling

The granules prepared in Process 1) and the tablets prepared in Process 2) were filled in capsules using a capsule filling machine, thereby preparing a formulation in the form of a capsule.

### Example III-7: Aliskiren-valsartan blister package kits

### 1) Preparation of aliskiren-containing prior-release tablets

According to the ingredient compositions and contents shown in Table 6 below, aliskiren hemi-fumarate, and microcrystalline cellulose and D-mannitol (as excipients) were sieved through a No. 35 sieve and mixed in a high-speed mixer. Meanwhile, hydroxypropylcellulose was dissolved in water to prepare a binding solution (15% w/w). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was mixed into the sieved material, and magnesium stearate was added thereto, followed by final mixing in a double cone mixer.

The final mixture was compressed into tablets having a hardness of 7 to 9kp, a thickness of 3.0mm, and a diameter of 5.5mm, using a rotary tablet press at 30 rpm, thereby preparing prior-release tablets.

### 2) Preparation of valsartan-containing delayed-release tablets

According to the ingredient compositions and contents shown in Table 6 below, valsartan and microcrystalline cellulose were sieved through a No. 35 sieve and mixed in a high-speed mixer for 5 minutes to prepare a mixture. The mixture was placed in a fluidized bed coater. Meanwhile, hydroxypropylmethylcellulose was dissolved in water to prepare a binding solution (15 w/w%) which was then sprayed thereon to form granules. Then, a solution, which was prepared by heating and dissolving glyceryl behenate and glyceryl distearate at 70□, was further sprayed and coated on the granules. Thereafter, magnesium stearate was added to the granules, followed by final mixing in a double cone mixer (Dasan Pharmatech, South Korea).

The final mixture was compressed into tablets having a hardness of 7 to 9kp, a thickness of 3.0mm, and a diameter of 5.5mm, using a rotary tablet press at 30 rpm, thereby preparing delayed-release tablets.

### 3) Blister package

The tablets prepared in Processes 1) and 2) were packed in a blister package container (silver foil, Dong-il Corporation, PVDC, Jeon Min Industry Co., Ltd., South Korea) such that they can be simultaneously administered, using a blister package machine (Minister A, Heung-A Engineering, South Korea).

### Example III-8: Preparation of valsartan-aliskiren two-phase matrix tablets

### 1) Preparation of valsartan-containing prior-release granules

According to the ingredient contents and compositions shown in Table 7 below, valsartan (as an active ingredient), and microcrystalline cellulose, lactose, corn starch and sodium starch glycolate were sieved through a No. 35 sieve and mixed in a high-speed mixer. Meanwhile, hydroxypropylcellulose was dissolved in water to prepare a binding solution (15 w/w%). The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 2) Preparation of aliskiren-containing delayed-release granules

According to the ingredient contents and compositions shown in Table 7 below, aliskiren hemi-fumarate (as an active ingredient) and microcrystalline cellulose were sieved through a No. 35 sieve and mixed in a high-speed mixer (Lab. Pharma Mixer P, Diosna, Germany) for 5 minutes to prepare a mixture. The mixture was placed in a fluidized bed coater (GPCG-1, Glatt, Germany). Meanwhile, hydroxypropylmethylcellulose was dissolved in water to prepare a binding solution (15 w/w%) which was then sprayed thereon to form granules, followed by drying. A solution (5% w/w) of Eudragit RS PO in a 1:1 mixture of ethanol and methylene chloride was sprayed and coated on the granules.

### 3) Compression and coating

The delayed-release granules and the prior-release granules thus prepared were placed and mixed in a double cone mixer. To the mixture was added sodium starch glycolate, followed by mixing, and magnesium stearate was added thereto, followed by final mixing. The final mixture was compressed into tablets using a rotary tablet press (MRC-33, Sejong Pharmatech Co., Ltd., South Korea). Meanwhile, hydroxypropylmethylcellulose 2910, hydroxypropylcellulose, titanium oxide, and talc were dissolved and dispersed in a 1:1 mixture of ethanol and purified water to prepare a coating solution (10 w/w%). The compressed tablets were coated with the coating solution in a Hi-coater (SFC-30N, Sejong Pharmatech Co., Ltd., South Korea) to form a film-coated layer, thereby preparing two-phase matrix tablets.

### Example III-9: Preparation of valsartan-aliskiren multi-layered tablets

### 1) Preparation of valsartan-containing prior-release layer

According to the ingredient contents and compositions shown in Table 7 below, valsartan (as an active ingredient), microcrystalline cellulose, and D-mannitol were sieved through a No. 35 sieve and mixed in a high-speed mixer. Meanwhile, hydroxypropylcellulose was dissolved in water to prepare a binding solution (15% w/w). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was mixed into the sieved material, and magnesium stearate was added thereto, followed by final mixing in a double cone mixer.

### 2) Preparation of aliskiren-containing delayed-release layer

According to the ingredient contents and compositions shown in Table 7 below, aliskiren hemi-fumarate (as an active ingredient) and microcrystalline cellulose were sieved through a No. 35 sieve and mixed in a high-speed mixer for 5 minutes to prepare a mixture. The mixture was placed in a fluidized bed coater. Meanwhile, hydroxypropylmethylcellulose was dissolved in water to prepare a binding solution (15 w/w%) which was then sprayed thereon to form granules, followed by drying. A solution (5% w/w) of hydroxypropylmethylcellulose phthalate in a 1:1 mixture of ethanol and methylene chloride was then sprayed and coated on the granules. Magnesium stearate was added thereto, followed by final mixing in a double cone mixer (Dasan Pharmatech, South Korea).

### 3) Compression and coating

The thus prepared valsartan-containing composition was placed in a first powder feeder, and the aliskiren delayed-release layer composition was placed in a second powder feeder. The layer compositions in the feeders were compressed into tablets under such conditions that the interlayer incorporation can be minimized. Meanwhile, hydroxypropylmethylcellulose 2910, hydroxypropylcellulose, titanium oxide, and talc were dissolved and dispersed in ethanol and purified water to prepare a coating solution (10 w/w%). The compressed tablets were coated with the coating solution in a Hi-coater to form a film-coated layer, thereby preparing a formulation in the form of a multi-layered tablet.

### Example III-10: Preparation of valsartan-aliskiren capsules (granules-pellets)

### 1) Preparation of valsartan-containing prior-release granules

According to the ingredient contents and compositions shown in Table 7 below, valsartan (as an active ingredient), microcrystalline cellulose, and D-mannitol were sieved through a No. 35 sieve and mixed in a high-speed mixer. Meanwhile, hydroxypropylcellulose was dissolved in water to prepare a binding solution (15 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve.

### 2) Preparation of aliskiren-containing delayed-release pellets

According to the ingredient contents and compositions shown in Table 7 below, a mixture of aliskiren hemi-fumarate (as an active ingredient), microcrystalline cellulose, and hydroxypropylmethylcellulose was dissolved in a 1:1 mixture (10 w/w%) of ethanol and methylene chloride to prepare a drug coating solution. Sugar spheres were placed in a fluidized bed coater, and the drug coating solution was coated thereon. Thereafter, a solution (5% w/w) of hydroxypropylmethylcellulose phthalate in a 1:1 mixture of ethanol and methylene chloride was further coated thereon to prepare pellets.

### 3) Mixing and capsule filling

The granules prepared in Process 1) and the pellets prepared in Process 2) were mixed in a double cone mixer. To the mixture was added sodium starch glycolate, followed by mixing in a double cone mixer. To the mixture was added magnesium stearate, followed by final mixing. The final mixture was placed in a powder feeder and then filled in capsules using a capsule filling machine (SF-40N, Sejong Pharmatech Co., Ltd., South Korea), thereby preparing a formulation in the form of a capsule.

### Example III-11: Preparation of valsartan-aliskiren capsules (tablets-pellets)

### 1) Preparation of valsartan-containing prior-release tablets

According to the ingredient contents and compositions shown in Table 7 below, valsartan (as an active ingredient), microcrystalline cellulose and D-mannitol were sieved through a No. 35 sieve and mixed in a high-speed mixer. Meanwhile, hydroxypropylcellulose was dissolved in water to prepare a binding solution (15 w/w%). The binding solution and the mixture of main ingredients were kneaded. After completion of the kneading process, the kneaded material was granulated using an oscillator with a No. 18 sieve, and the granules were dried in a hot-water dryer at 60°C. After completion of the drying process, the granules were sieved again through a No. 20 sieve. Sodium starch glycolate was added to the sieved material, followed by mixing in a double cone mixer. To the mixture was added magnesium stearate, followed by final mixing. The final mixture was compressed into tablets using a rotary tablet press.

### 2) Preparation of aliskiren-containing delayed-release pellets

According to the ingredient compositions and contents shown in Table 7 below, the preparation was carried out in the same manner as in Process 2) of Example III-10.

### 3) Capsule filling

The tablets prepared in Process 1) and the pellets prepared in Process 2) were filled in capsules using a capsule filling machine, thereby preparing a formulation in the form of a capsule.

### Example III-12: Preparation of valsartan-aliskiren capsules (granules-granules)

### 1) Preparation of valsartan-containing prior-release granules

According to the ingredient compositions and contents shown in Table 7 below, the preparation was carried out in the same manner as in Process 1) of Example III-10.

### 2) Preparation of aliskiren-containing delayed-release granules

According to the ingredient contents and compositions shown in Table 7 below, aliskiren hemi-fumarate (as an active ingredient) and microcrystalline cellulose were sieved through a No. 35 sieve and mixed in a high-speed mixer for 5 minutes to prepare a mixture. The mixture was placed in a fluidized bed coater. Meanwhile, hydroxypropylmethylcellulose was dissolved in water to prepare a binding solution (15 w/w%) which was then sprayed thereon to form granules, followed by drying. A solution (5% w/w) of hydroxypropylmethylcellulose phthalate in a 1:1 mixture of ethanol and methylene chloride was then sprayed and coated on the granules.

### 3) Mixing and capsule filling

The granules prepared in Process 1) and the granules prepared in Process 2) were mixed in a double cone mixer. To the mixture was added sodium starch glycolate, followed by mixing in a double cone mixer. To the mixture was added magnesium stearate, followed by final mixing. The final mixture was placed in a powder feeder and then filled in capsules using a capsule filling machine, thereby preparing a formulation in the form of a capsule.

### Example III-13: Preparation of valsartan-aliskiren capsules (granules-tablets)

### 1) Preparation of valsartan-containing prior-release granules

According to the ingredient compositions and contents shown in Table 7 below, the preparation was carried out in the same manner as in Process 1) of Example 10.

### 2) Preparation of aliskiren-containing delayed-release coated tablets

According to the ingredient contents and compositions shown in Table 7 below, aliskiren hemi-fumarate (as an active ingredient), microcrystalline cellulose, and sodium starch glycolate were sieved through a No. 35 sieve and mixed in a high-speed mixer for 5 minutes to prepare a mixture. The mixture was placed in a fluidized bed granulator. Meanwhile, hydroxypropylmethylcellulose was dissolved in water to prepare a binding solution (15 w/w%) which was then sprayed thereon to form granules, followed by drying. Then, powdered Carbomer 71 G was added to the granules, and magnesium stearate was added thereto, followed by final mixing in a double cone mixer.

The final mixture was compressed into tablets having a hardness of 7 to 9kp, a thickness of 3.0mm, and a diameter of 5.5mm, using a rotary tablet press at 30 rpm. A solution (5% w/w) of hydroxypropylmethylcellulose phthalate in a 1:1 mixture of ethanol and methylene chloride was then coated thereon to prepare delayed-release coated tablets.

### 3) Capsule filling

The granules prepared in Process 1) and the tablets prepared in Process 2) were filled in capsules using a capsule filling machine, thereby preparing a controlled-release formulation in the form of a capsule.

### Example III-14: Preparation of valsartan-aliskiren osmotic press-coated tablets

### 1) Preparation of valsartan-containing prior-release layer

According to the ingredient contents and compositions shown in Table 7 below, valsartan (as an active ingredient), microcrystalline cellulose, lactose, corn starch, and sodium starch glycolate were sieved through a No. 35 sieve and mixed in a high-speed mixer. Meanwhile, hydroxypropylcellulose was dissolved in water to prepare a binding solution (15% w/w). The binding solution and the mixture of main ingredients were placed in a high-speed mixer, followed by kneading. After completion of the kneading process, the kneaded material was granulated using an oscillator with a No. 18 sieve, and the granules were dried in a hot-water dryer at 60°C. After completion of the drying process, the granules were sieved again through a No. 20 sieve, and magnesium stearate was added thereto, followed by final mixing in a double cone mixer to prepare a prior-release layer.

### 2) Preparation of aliskiren-containing delayed-release osmotic coated tablets

According to the ingredient contents and compositions shown in Table 7 below, aliskiren hemi-fumarate (as an active ingredient), microcrystalline cellulose, and sodium chloride were sieved through a No. 35 sieve and mixed in a double cone mixer. Magnesium stearate was added thereto, followed by final mixing. The final mixture was compressed into tablets using a rotary tablet press (MRC-30: Sejong, South Korea). The compressed inner core tablets were coated with a dispersion (20% w/w) of Kollicoat SR 30D as a semi-permeable coating base and triethyl citrate as a film-forming aid in purified water in a Hi-coater (SFC-30N, Sejong Machinery Co., Ltd., South Korea), thereby preparing osmotic inner core tablets.

### 3) Compression and coating

The thus prepared valsartan-containing prior-release layer composition was placed in a powder feeder, and the aliskiren-containing osmotic inner core tablets were placed in an inner core feeder, followed by compression in a press-coated tablet press (Kilian RUD-1, Germany). Meanwhile, hydroxypropylmethylcellulose 2910, hydroxypropylcellulose, titanium oxide, and talc were dissolved and dispersed in a 7:3 mixture of ethanol and purified water to prepare a coating solution (20% w/w). The compressed tablets were coated with the coating solution in a Hi-coater (SFC-30N, Sejong Pharmatech Co., Ltd., South Korea) to form a film-coated layer, thereby preparing a formulation in the form of a press-coated tablet.

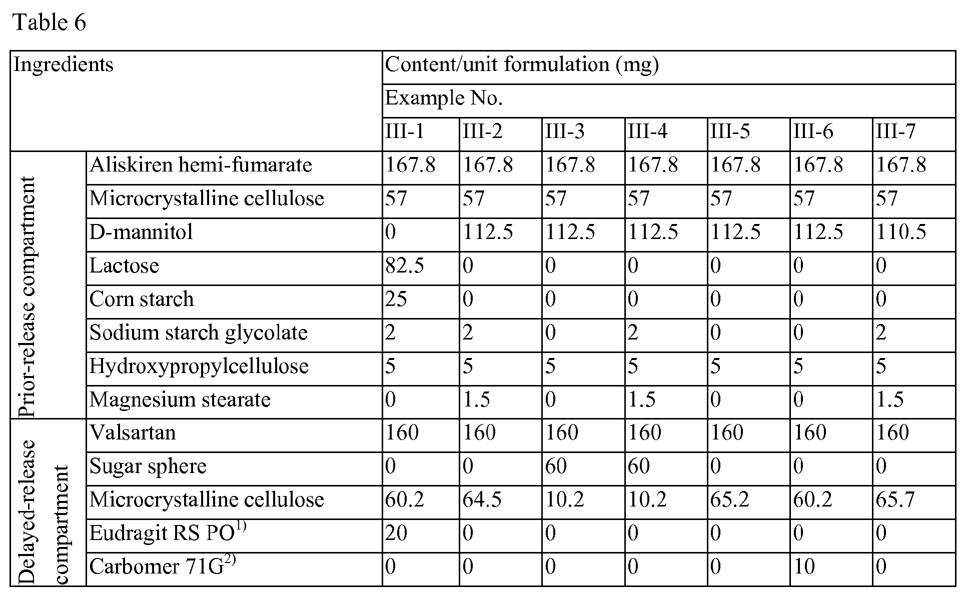

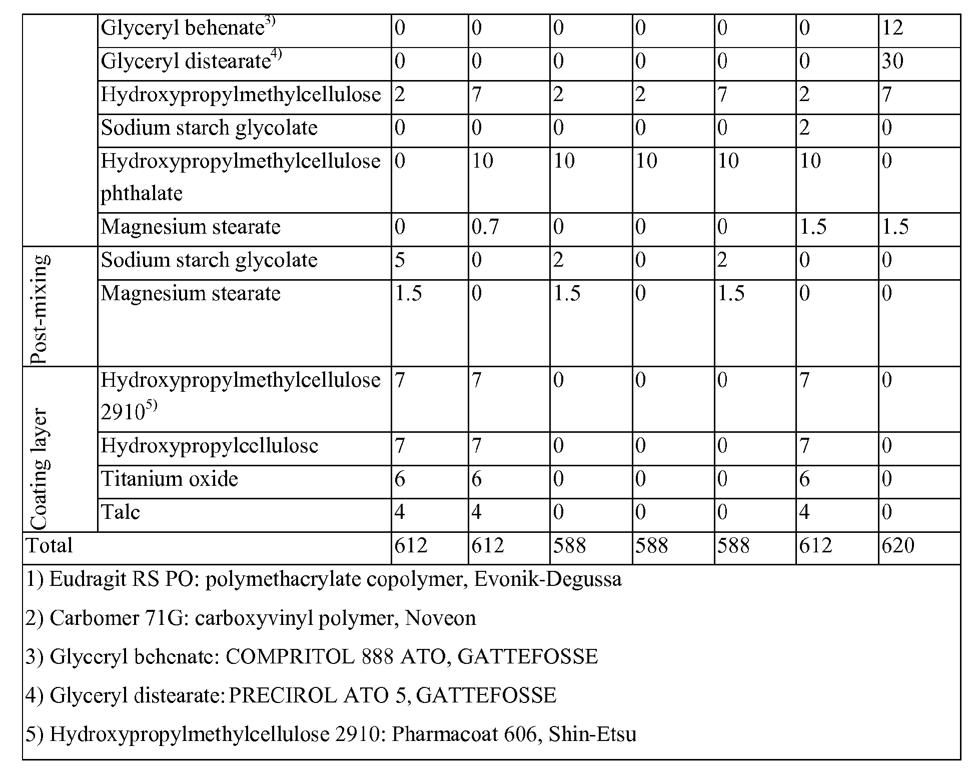

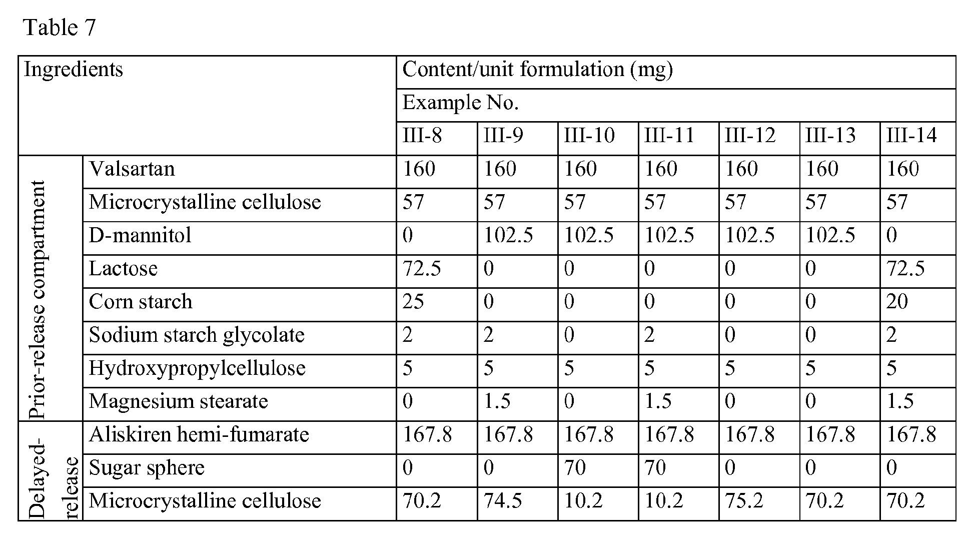

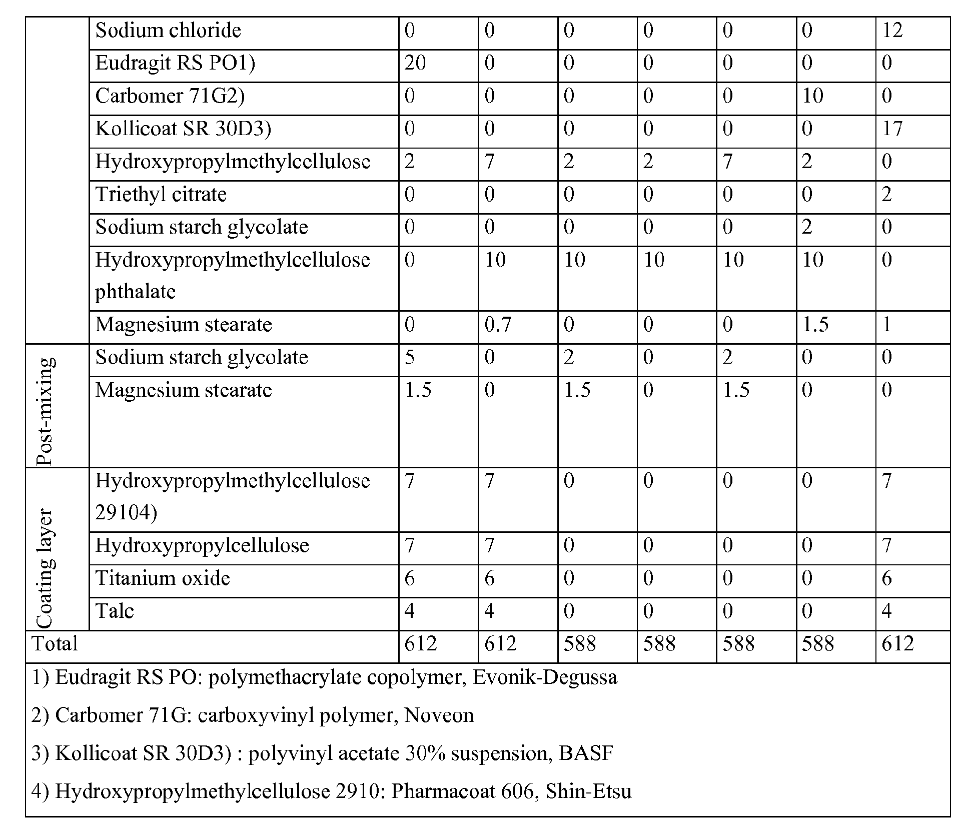

### Experimental Example III-1: Comparative dissolution profile test

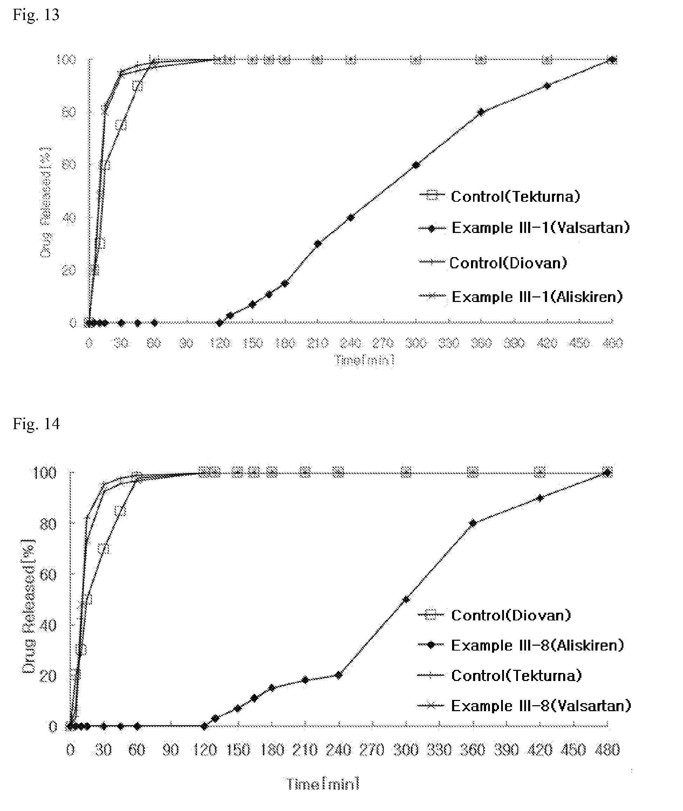

A comparative dissolution profile test was performed using the formulation prepared in Example III-1 and control drugs (Diovan, Novartis: valsartan single drug, Tektuma, Novartis: aliskiren single drug). The dissolution profile test of the aliskiren ingredient was performed based on the general dissolution test method described in the Korean Pharmacopoeia (8^{th} revision), and the dissolution profile test of the valsartan ingredient was performed for a total of 480 minutes, in which the dissolution medium was changed from a simulated gastric juice to a simulated intestinal juice 120 minutes after the start of the test. Details of the dissolution profile test of each ingredient are as follows. The results obtained are shown in FIG. 13. In FIG. 13, the x-axis represents the time series (min), and the y-axis represents the dissolution rate (Drug Released, %).

As can be seen in FIG. 13, when the dissolution profile test was performed under the following conditions, the aliskiren ingredient of the formulation of the present invention showed a dissolution profile substantially equal to that of the control drug Tektuma, but the valsartan ingredient showed a very slow dissolution rate as compared to that of the control drug Diovan.

The dissolution rate of the valsartan ingredient was less than 20% up to 2 hours in the formulation of the present invention, which was far lower than the dissolution rate of about 99% in the control drugs.

As described above, the initial release of valsartan in the formulation of the present invention is much slower than aliskiren, unlike dissolution profiles obtained when the aliskiren single drug and the valsartan single drug, as the control drugs, are administered simultaneously. Thus, in the case of the inventive formulation, the time for valsartan to be absorbed after aliskiren is metabolized first in the liver can be sufficiently ensured.

### Test Method for valsartan: Based on the general dissolution test method described in the Korean Pharmacopoeia (8^{th} revision)

Test method: Paddle method, 50 rpm
Dissolution medium: 0.01 M hydrochloric acid solution 750 mL (simulated gastric juice), pH 6.8 phosphate buffer 1,000 mL (simulated intestinal juice)
Analysis method: UV-Vis spectrophotometry, measurement wavelength: 250 nm

### Test Method for aliskiren hemi-fumarate: Based on the general dissolution test method described in the Korean Pharmacopoeia (8^{th} revision)

Test method: Paddle method, 50 rpm
Dissolution medium: Purified water 900 mL
Analysis method: High performance liquid chromatography

### Experimental Example III-2: Comparative dissolution profile test

A comparative dissolution profile test was performed using the formulation prepared in Example III-8 and control drugs (Diovan: valsartan single drug, Tekturna: aliskiren single drug). The dissolution profile test of the valsartan ingredient was performed based on the "Valsartan and Hydrochlorothiazide Tablet" part in the United States Pharmacopoeia, and the dissolution profile test of the aliskiren ingredient was performed for a total of 480 minutes, in which the dissolution medium was changed from a simulated gastric juice to a simulated intestinal juice 120 minutes after the start of the test. Details of the dissolution profile test of each ingredient are as follows. The results obtained are shown in FIG. 14. In FIG. 14, the x-axis represents the time series (min), and the y-axis represents the dissolution rate (Drug Released, %).

As can be seen in FIG. 14, when the dissolution profile test was performed under the following conditions, the valsartan ingredient of the formulation of the present invention showed a dissolution profile substantially equal to that of the control drug Diovan, but the aliskiren ingredient showed a very slow dissolution rate as compared to that of the control drug Tekturna,.

The dissolution rate of the aliskiren ingredient was less than 20% up to 2 hours in the aliskiren-valsartan formulation of the present invention, which was far lower than the dissolution rate of about 99% in the control drugs.

### Test Method for valsartan: Based on the "Valsartan and Hydrochlorothiazide Tablet" part in USP 31

Test method: Paddle method, 50 rpm
Dissolution medium: 0.01 N hydrochloric acid solution, 900 mL
Analysis method: UV-Vis spectrophotometry, measurement wavelength: 250 nm

### Test Method for aliskiren hemi-fumarate: Based on the general dissolution test method described in the Korean Pharmacopoeia (8^{th} revision)

Test method: Paddle method, 50 rpm
Dissolution medium: 0.01 N hydrochloric acid solution 750 mL (simulated gastric juice), pH 6.8 phosphate buffer 1,000 mL (simulated intestinal juice)
Analysis method: High performance liquid chromatography

### Experimental Example III-3: Comparative dissolution profile test

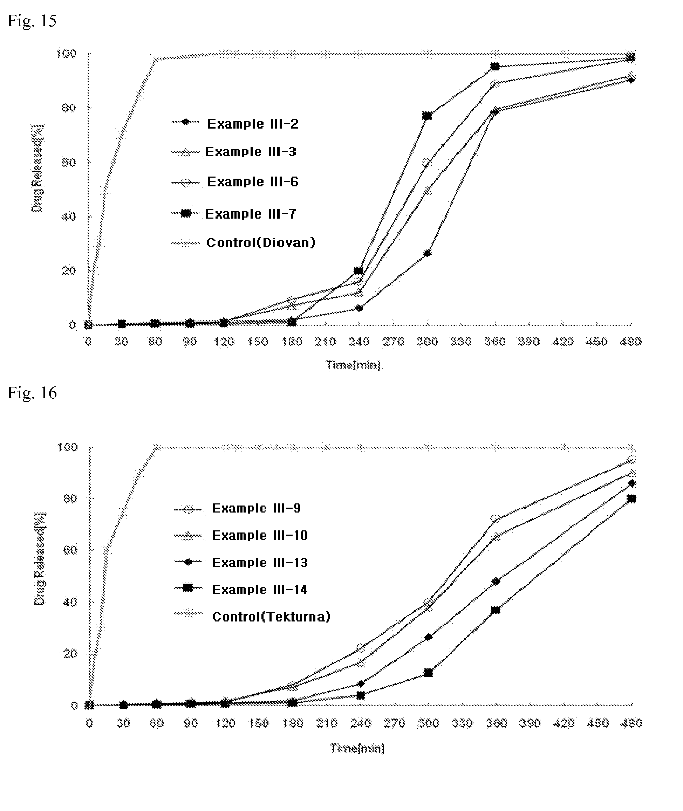

A comparative dissolution profile test was performed using the formulations prepared in Examples III-2, III-3, III-6 and III-7 and a control drug (Diovan: valsartan single drug). Details of the dissolution profile test are the same as those of Experimental Example III-1. The dissolution profile test results of the valsartan ingredient are shown in FIG. 15. In FIG. 15, the x-axis represents the time series (min), and the y-axis represents the dissolution rate (Drug Released, %).

According to the dissolution profile test results, the dissolution rate of the valsartan ingredient was less than 10% up to 1 hour in the aliskiren-valsartan formulation of the present invention, thus showing remarkable delay of the valsartan ingredient.

### Test Method for valsartan: Based on the general dissolution test method described in the Korean Pharmacopoeia (8^{th} revision)

Test method: Paddle method, 50 rpm
Dissolution medium: 0.01 M hydrochloric acid solution 750 mL (simulated gastric juice), pH 6.8 phosphate buffer 1,000 mL (simulated intestinal juice)
Analysis method: UV-Vis spectrophotometry, measurement wavelength: 250 nm

### Experimental Example III-4: Comparative dissolution profile test

A comparative dissolution profile test was performed using the formulations prepared in Examples III-9, III-10, III-13 and III-14 and a control drug (Tekturna: aliskiren single drug). Details of the dissolution profile test are as follows. The dissolution profile test results of the aliskiren ingredient are shown in FIG. 16. In FIG. 16, the x-axis represents the time series (min), and the y-axis represents the dissolution rate (Drug Released, %).

According to the test results, the dissolution rate of the aliskiren ingredient was less than 20% up to 2 hours in the formulation of the present invention, thus showing remarkable delay of the aliskiren ingredient.

### Test Method for aliskiren hemi-fumarate: Based on the general dissolution test method described in the Korean Pharmacopoeia (8^{th} revision)

Test method: Paddle method, 50 rpm
Dissolution medium: 0.01 N hydrochloric acid solution 750 mL (simulated gastric juice), pH 6.8 phosphate buffer 1,000 mL (simulated intestinal juice)
Analysis method: High performance liquid chromatography

### INDUSTRIAL APPLICABILITY

The formulation of the present invention can deliver a renin inhibitor and an angiotensin-II-receptor blocker with a time interval at a specific speed, thus reducing undesirable side-effects, improving the drug efficacy and promoting the patient compliance. Further, the pharmaceutical formulation of the present invention has pharmacological, clinical, scientific and economical advantages in the prevention or treatment of metabolic syndromes, cardiovascular diseases, renal diseases and the like, as compared with the complex drug regimens in which medicament ingredients are taken individually or simultaneously.

## Claims

1. A pharmaceutical formulation comprising a compartment containing a renin inhibitor as a pharmacologically active ingredient, and a compartment containing an angiotensin-II-receptor blocker as a pharmacologically active ingredient, wherein one compartment is a prior-release compartment and the other compartment is a delayed-release compartment.

2. The pharmaceutical formulation according to claim 1, wherein the renin inhibitor is at least one selected from aliskiren, remikiren, enalkiren, zankiren, detikiren, terlakiren, isomers thereof and pharmaceutically acceptable salts thereof.

3. The pharmaceutical formulation according to claim 1, wherein the angiotensin-II-receptor blocker is at least one selected from irbesartan, valsartan, losartan, telmisartan, candesartan, olmesartan, isomers thereof, pharmaceutically acceptable salts thereof, and prodrugs thereof.

4. The pharmaceutical formulation according to claim 1, wherein the renin inhibitor is aliskiren, an isomer thereof or a pharmaceutically acceptable salt thereof and the angiotensin-II-receptor blocker is irbesartan, an isomer thereof or a pharmaceutically acceptable salt thereof.

5. The pharmaceutical formulation according to claim 1, wherein the renin inhibitor is aliskiren, an isomer thereof or a pharmaceutically acceptable salt thereof and the angiotensin-II-receptor blocker is valsartan, an isomer thereof or a pharmaceutically acceptable salt thereof.

6. The pharmaceutical formulation according to claim 1, wherein the renin inhibitor is aliskiren, an isomer thereof or a pharmaceutically acceptable salt thereof and the angiotensin-II-receptor blocker is losartan, an isomer thereof or a pharmaceutically acceptable salt thereof.

7. The pharmaceutical formulation according to claim 1, wherein the renin inhibitor is aliskiren, an isomer thereof or a pharmaceutically acceptable salt thereof and the angiotensin-II-receptor blocker is telmisartan, an isomer thereof or a pharmaceutically acceptable salt thereof.

8. The pharmaceutical formulation according to claim 1, wherein the renin inhibitor is aliskiren, an isomer thereof or a pharmaceutically acceptable salt thereof and the angiotensin-II-receptor blocker is candesartan, an isomer thereof, a pharmaceutically acceptable salt thereof, or a prodrug thereof.

9. The pharmaceutical formulation according to claim 8, wherein the candesartan prodrug is candesartan cilexetil.

10. The pharmaceutical formulation according to claim 1, wherein the renin inhibitor is aliskiren, an isomer thereof or a pharmaceutically acceptable salt thereof and the angiotensin-II-receptor blocker is olmesartan, an isomer thereof, a pharmaceutically acceptable salt thereof, or a prodrug thereof.

11. The pharmaceutical formulation according to claim 10, wherein the olmesartan prodrug is olmesartan medoxomil.

12. The pharmaceutical formulation according to any one of claims 1 to 11, wherein a pharmacologically active ingredient of the prior-release compartment is released at a level of more than 80% by weight of a total amount of the pharmacologically active ingredient in the formulation within one hour after the release of the pharmacologically active ingredient is initiated.

13. The pharmaceutical formulation according to any one of claims 1 to 11, wherein a pharmacologically active ingredient of the delayed-release compartment is released at a level of less than 40% by weight of a total amount of the pharmacologically active ingredient of the delayed-release compartment by 2 hours after the release of the pharmacologically active ingredient of the prior-release compartment is initiated.

14. The pharmaceutical formulation according to any one of claims 1 to 11, wherein a content of the renin inhibitor in the pharmaceutical formulation is in the range of 10 to 1000 mg.

15. The pharmaceutical formulation according to any one of claims 1 to 11, wherein a content of the angiotensin-II-receptor blocker in the pharmaceutical formulation is in the range of 10 to 1000 mg.

16. The pharmaceutical formulation according to any one of claims 1 to 11, wherein the delayed-release compartment further includes at least one release-controlling material selected from an enteric polymer, a water-insoluble polymer, a hydrophobic compound, a hydrophilic polymer and a mixture thereof, in addition to pharmacologically active ingredients.

17. The pharmaceutical formulation according to claim 16, wherein a content of the release-controlling material is in the range of 0.01 to 100 parts by weight, relative to 1 part by weight of the pharmacologically active ingredient.

18. The pharmaceutical formulation according to claim 16, wherein the enteric polymer is at least one selected from the group consisting of an enteric cellulose derivative, an enteric acrylic acid copolymer, an enteric maleic acid copolymer, an enteric polyvinyl derivative, and a mixture thereof.

19. The pharmaceutical formulation according to claim 18, wherein the enteric cellulose derivative is at least one selected from hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose, ethylhydroxyethylcellulose phthalate, methylhydroxyethylcellulose and a mixture thereof; the enteric acrylic acid copolymer is at least one selected from a styrene/acrylic acid copolymer, a methyl acrylate/acrylic acid copolymer, a methyl acrylate/methacrylic acid copolymer, a butyl acrylate/styrene/acrylic acid copolymer, a methacrylic acid/methyl methacrylate copolymer, a methacrylic acid/ethyl acrylate copolymer, a methyl acrylate/methacrylic acid/octyl acrylate copolymer and a mixture thereof; the enteric maleic acid copolymer is at least one selected from a vinyl acetate/maleic anhydride copolymer, a styrene/maleic anhydride copolymer, a styrene/maleic monoester copolymer, a vinyl methyl ether/maleic anhydride copolymer, an ethylene/maleic anhydride copolymer, a vinyl butyl ether/maleic anhydride copolymer, an acrylonitrile/methyl acrylate/maleic anhydride copolymer, a butyl acrylate/styrene/maleic anhydride copolymer and a mixture thereof; and the enteric polyvinyl derivative is at least one selected from polyvinylalcohol phthalate, polyvinylacetal phthalate, polyvinylbutyrate phthalate, polyvinylacetacetal phthalate and a mixture thereof.

20. The pharmaceutical formulation according to claim 18, wherein a content of the enteric polymer is in the range of 0.1 parts by weight to 20 parts by weight, relative to 1 part by weight of the pharmacologically active ingredient.

21. The pharmaceutical formulation according to claim 16, wherein the water-insoluble polymer is at least one selected from the group consisting of polyvinyl acetate, a water-insoluble polymethacrylate copolymer, ethylcellulose, cellulose ester, cellulose ether, cellulose acylate, cellulose diacylate, cellulose triacylate, cellulose acetate, cellulose diacetate, cellulose triacetate and a mixture thereof.

22. The pharmaceutical formulation according to claim 21, wherein a content of the water-insoluble polymer is in the range of 0.1 parts by weight to 30 parts by weight, relative to 1 part by weight of the pharmacologically active ingredient.

23. The pharmaceutical formulation according to claim 21, wherein the water-insoluble polymer is at least one selected from the group consisting of polyvinylacetate, a poly(ethyl acrylate, methyl methacrylate, trimethylaminoethyl methacrylate) copolymer and a mixture thereof.

24. The pharmaceutical formulation according to claim 16, wherein the release-controlling material is at least one selected from an enteric polymer, a hydrophilic polymer and a water-insoluble polymer.

25. The pharmaceutical formulation according to claim 24, wherein the release-controlling material is at least one selected from a water-insoluble polymer, hydroxypropylcellulose, hydroxypropylmethylcellulose, hydroxypropylmethylcellulose phthalate, a methacrylic acid/ethyl acrylate copolymer, and a carboxyvinyl polymer.

26. The pharmaceutical formulation according to claim 16, wherein the hydrophobic compound is at least one selected from a fatty acid or fatty acid ester, a fatty acid alcohol, a wax, an inorganic material, and a mixture thereof.

27. The pharmaceutical formulation according to claim 26, wherein the fatty acid or fatty acid ester is at least one selected from glyceryl palmitostearate, glyceryl stearate, glyceryl distearate, glyceryl behenate, cetyl palmitate, glyceryl monooleate, stearic acid and a mixture thereof; the fatty acid alcohol is at least one selected from cetostearyl alcohol, cetyl alcohol, stearyl alcohol and a mixture thereof; the wax is at least one selected from carnauba wax, beeswax, microcrystalline wax and a mixture thereof; and the inorganic material is at least one selected from talc, precipitated calcium carbonate, calcium hydrogen phosphate, zinc oxide, titanium oxide, kaolin, bentonite, montmorillonite, veegum and a mixture thereof.

28. The pharmaceutical formulation according to claim 26, wherein a content of the hydrophobic compound is in the range of 0.1 parts by weight to 20 parts by weight, relative to 1 part by weight of the pharmacologically active ingredient.

29. The pharmaceutical formulation according to claim 16, wherein the hydrophilic polymer is at least one selected from a saccharide, a cellulose derivative, a gum, a protein, a polyvinyl derivative, a hydrophilic polymethacrylate copolymer, a polyethylene derivative, a carboxyvinyl copolymer and a mixture thereof.

30. The pharmaceutical formulation according to claim 29, wherein the saccharide is at least one selected from dextrin, polydextrin, dextran, pectin and a pectin derivative, alginate, polygalacturonic acid, xylan, arabinoxylan, arabinogalactan, starch, hydroxypropyl starch, amylose, amylopectin and a mixture thereof; the cellulose derivative is at least one selected from hydroxypropylmethylcellulose, hydroxypropylcellulose, hydroxymethylcellulose, hydroxyethylcellulose, methylcellulose, sodium carboxymethylcellulose, hydroxypropylmethylcellulose acetate succinate, hydroxyethylmethylcellulose and a mixture thereof; the gum is at least one selected from guar gum, locust bean gum, tragacanth, carrageenan, gum acacia, gum arabic, gellan gum, xanthan gum and a mixture thereof; the protein is at least one selected from gelatin, casein, zein and a mixture thereof; the polyvinyl derivative is at least one selected from polyvinyl alcohol, polyvinyl pyrrolidone, polyvinylacetal diethylaminoacetate and a mixture thereof; the hydrophilic polymethacrylate copolymer is at least one selected from a poly(butyl methacrylate, (2-dimethylaminoethyl) methacrylate, methyl methacrylate) copolymer, a poly(methacrylic acid, methyl methacrylate) copolymer, a poly(methacrylic acid, ethyl acrylate) copolymer and a mixture thereof; the polyethylene derivative is at least one selected from polyethylene glycol, polyethylene oxide and a mixture thereof; and the carboxyvinyl polymer is carbomer.

31. The pharmaceutical formulation according to claim 29, wherein a content of the hydrophilic polymer is in the range of 0.05 parts by weight to 30 parts by weight, relative to 1 part by weight of the pharmacologically active ingredient.

32. The pharmaceutical formulation according to any one of claims 1 to 11, wherein the pharmaceutical formulation is in the form of a two-phase matrix tablet which is obtained by uniformly mixing a delayed-release compartment and a prior-release compartment, followed by compression.

33. The pharmaceutical formulation according to any one of claims 1 to 11, wherein the pharmaceutical formulation is in the form of a film-coated tablet including a tablet of a delayed-release compartment and a film-coated layer of a prior-release compartment enclosing the exterior of the tablet.

34. The pharmaceutical formulation according to any one of claims 1 to 11, wherein the pharmaceutical formulation is in the form of a multi-layered tablet having a multi-layered structure of the delayed-release compartment and the prior-release compartment.

35. The pharmaceutical formulation according to any one of claims 1 to 11, wherein the pharmaceutical formulation is in the form of a press-coated tablet including an inner core tablet of a delayed-release compartment and an outer layer of a prior-release compartment enclosing the outer surface of the inner core tablet.

36. The pharmaceutical formulation according to claim 35, wherein the press-coated tablet is an osmotic press-coated tablet.

37. The pharmaceutical formulation according to any one of claims 1 to 11, wherein the pharmaceutical formulation is in the form of a capsule including a particle, granule, pellet, or tablet of a delayed-release compartment and a particle, granule, pellet, or tablet of a prior-release compartment.

38. The pharmaceutical formulation according to any one of claims 1 to 11, further comprising a coating layer on the outside of the delayed-release compartment and/or the prior-release compartment.

39. The pharmaceutical formulation according to any one of claims 1 to 11, wherein the delayed-release compartment is a compartment which contains an osmo-regulator and is coated by a semi-permeable membrane coating base.

40. The pharmaceutical formulation according to claim 39, wherein the osmo-regulator is at least one selected from the group consisting of magnesium sulfate, magnesium chloride, sodium chloride, lithium chloride, potassium sulfate, sodium sulfate, lithium sulfate and a mixture thereof.

41. The pharmaceutical formulation according to claim 39, wherein the semi-permeable membrane coating base is at least one selected from the group consisting of polyvinyl acetate, a polymethacrylate copolymer, a poly(ethyl acrylate, methyl methacrylate) copolymer, a poly(ethyl acrylate, methyl methacrylate, trimethylaminoethyl methacrylate) copolymer, ethylcellulose, cellulose ester, cellulose ether, cellulose acylate, cellulose diacylate, cellulose triacylate, cellulose acetate, cellulose diacetate, cellulose triacetate and a mixture thereof.

42. The pharmaceutical formulation according to any one of claims 1 to 11, wherein the pharmaceutical formulation is in the form of a coated tablet further including a coating layer on the outside thereof.

43. The pharmaceutical formulation according to any one of claims 1 to 11, wherein the pharmaceutical formulation is in the form of a kit including a delayed-release compartment and a prior-release compartment.

44. A method for preventing and treating at least one disease selected from a metabolic syndrome, a cardiovascular disease and a renal disease, comprising administering a pharmaceutical formulation of any one of claims 1 to 11 to a mammal including a human.
